# EUROPEAN PATENT SPECIFICATION

(11) **EP 1 414 802 B1**
(45) Date of publication and mention of the grant of the patent: **26.04.2006**
(21) Application number: 02749497.0
(22) Date of filing: 26.07.2002
(51) Int. Cl.: C07D 213/85, C07D 409/12, C07D 413/12, C07D 417/12, A61K 31/44, A61K 31/4427, A61P 29/00, A61P 25/00

(54) **HETEROARYLHETEROALKYLAMINE DERIVATIVES AND THEIR USE AS INHIBITORS OF NITRIC OXIDE SYNTHASE**
HETEROARYLHETEROALKYLAMIN DERIVATE UND DEREN VERWENDUNG ALS INHIBITOREN DER STICKOXIDSYNTHASE
DERIVES D'HETEROARYLHETEROALKYLAMINE, ET LEUR UTILISATION COMME INHIBITEURS DE LA SYNTHASE DU MONOXYDE D'AZOTE

(30) Priority: 31.07.2001 SE 0102641
(43) Date of publication of application: 06.05.2004
(73) Proprietor: AstraZeneca AB, 151 85 Södertälje (SE)
(72) Inventor: BIRKINSHAW, Timothy, Loughborough, Leicestershire LE11 5RH (GB); CHESHIRE, David, Loughborough, Leicestershire LE11 5RH (GB); CONNOLLY, Stephen, Loughborough, Leicestershire LE11 5RH (GB); LUKER, Timothy, Loughborough, Leicestershire LE11 5RH (GB); METE, Antonio, Loughborough, Leicestershire LE11 5RH (GB)
(86) International application number: PCT/SE2002/001413
(87) International publication number: WO 2003/011830

(56) References cited:
- EP-A1- 0 571 685
- EP-A1- 0 707 007
- EP-A2- 0 399 504
- GB-A- 2 060 622
- US-A- 4 666 910
- DATABASE CAPLUS [Online] DOC. NO. 86:189458 'Aromatic amino ether quaternary ammonium salts', XP002958414 Retrieved from STN Database accession no. 1977:189458 & JP 51 044 934 B 01 December 1976

## Description

### Field of the Invention

The present invention relates to novel heteroarylheteroalkylamine derivatives, processes for their preparation, compositions containing them and their use in therapy.

### Background of the Invention

Nitric oxide is produced in mammalian cells from L-arginine by the action of specific nitric oxide synthases (NOSs). These enzymes fall into two distinct classes - constitutive NOS (cNOS) and inducible NOS (iNOS). At the present time, two constitutive NOSs and one inducible NOS have been identified. Of the constitutive NOSs, an endothelial enzyme (eNOS) is involved with smooth muscle relaxation and the regulation of blood pressure and blood flow, whereas the neuronal enzyme (nNOS) appears to be involved in the regulation of various biological functions. Inducible NOS has been particularly implicated in the pathogenesis of inflammatory diseases. Regulation of these enzymes should therefore offer considerable potential in the treatment of a wide variety of disease states (J. E. Macdonald, *Ann. Rep. Med. Chem*., 1996, **31**, 221 - 230).

Considerable effort has been expended to identify compounds that act as specific inhibitors of one or more isoforms of the enzyme nitric oxide synthase. The use of such compounds in therapy has also been widely claimed.

GB 2060622 discloses certain 3-aryl-3-aryloxyalkylamines useful, for example, as antidepressant agents.

EP 0 399 504 and EP 0 571 685 disclose aryloxyheteroarylpropylamines that have activity against calcium overload in brain cells.

US 4,666,910 discloses (2-phenyl-2-(pyridyl-oxy or -thio)-ethyl)amines having antidepressant properties.

JP 51044934 discloses quaternary ammonium salts that possess anticholinergic activity.

EP 0 707 007 discloses (R)-(-)-2-[5-(4-fluorophenyl)-3-pyridyl-methylaminomethyl]-chromane which has utility as a serotonin agonist and antagonist.

### Disclosure of the invention

According to the present invention, there is provided a compound of formula (I) 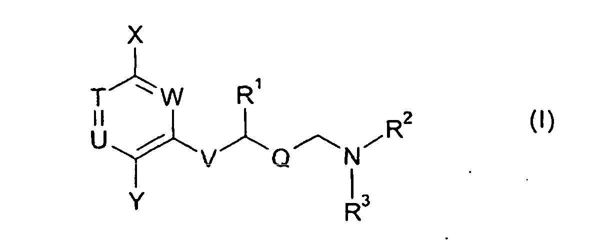 wherein:
X represents H, C1 to 4 alkyl, C1 to 4 alkoxy, halogen, OH, NHR⁹, CN, C=CH, NO₂, CHO, COCH₃ or NHCHO; said alkyl or alkoxy group being optionally further substituted by one or more fluorine atoms or by an OH group;
Y represents C1 to 4 alkyl, C1 to 4 alkoxy, halogen, OH, CN, C≡CH, NO₂, CHO, COCH₃ or NHCHO; said alkyl or alkoxy group being optionally further substituted by one or more fluorine atoms;
One of T, U and W represents N and the other two independently represent CR⁴; and each R⁴ group independently represents H, F or CH₃;
V represents O or S(O)ₙ;
n represents an integer 0, 1 or 2;
Q represents CH₂ or (CH₂)₂;
R¹ represents phenyl or a five or six membered aromatic heterocyclic ring containing 1 to 3 heteroatoms independently selected from O, S and N; said phenyl or aromatic heterocyclic ring being optionally substituted by one or more substituents selected independently from halogen, C1 to 4 alkyl, C1 to 4 alkoxy, OH, CN, NO₂ or NR⁵R⁶; said alkyl or alkoxy group being optionally further substituted by one or more fluorine atoms;
R² and R³ independently represent H, C1 to 4 alkyl or C3 to 6 cycloalkyl; said alkyl group being optionally substituted by C1 to 4 alkoxy, halogen, hydroxy, -Z-NR⁷R⁸, phenyl or a five or six membered aromatic or saturated heterocyclic ring containing 1 to 3 heteroatoms independently selected from O, S and N; said phenyl or aromatic heterocyclic ring being optionally further substituted by halogen, C1 to 4 alkyl, C1 to 4 alkoxy, CF₃, OCF₃, OH, CN or NO₂;
Z represents -CO- or a bond;
R⁵, R⁶, R⁷ and R⁸ independently represent H or C1 to 4 alkyl;
R⁹ represents H or C1 to 4 alkyl; said alkyl group being optionally further substituted by one or more fluorine atoms;
or a pharmaceutically acceptable salt thereof.

It will be recognised that compounds of formula (I) wherein W represents N and X represents OH may exist in the alternative tautomeric form (Ia): 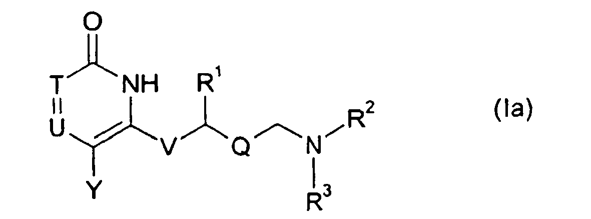 Analogous tautomeric structures will also exist for compounds of formula (1) wherein T represents N and X represents OH; or wherein U represents N and Y represents OH. All possible tautomers of compounds of formula (I) and mixtures thereof are included within the scope of the present invention.

The compounds of formula (I) may exist in enantiomeric forms. All enantiomers, diastereomers, racemates and mixtures thereof are included within the scope of the invention.

In one particular embodiment the invention provides compounds of formula (I) wherein:
X represents H, C1 to 4 alkyl, C1 to 4 alkoxy, halogen, OH, CN, C≡CH, NO₂, CHO, COCH₃ or NHCHO; said alkyl or alkoxy group being optionally further substituted by one or more fluorine atoms or by an OH group;
Q represents CH₂;
R² and R³ independently represent H, C1 to 4 alkyl or C3 to 6 cycloalkyl; said alkyl group being optionally substituted by C1 to 4 alkoxy, halogen, hydroxy, -Z-NR⁷R⁸, phenyl or a five or six membered aromatic or saturated heterocyclic ring containing 1 to 3 heteroatoms independently selected from O, S and N; said phenyl or aromatic heterocyclic ring being optionally further substituted by halogen, C1 to 4 alkyl, C1 to 4 alkoxy, CF₃, OCF₃, CN or NO₂;
and R¹, R⁴, R⁵, R⁶, R⁷, R⁸, n, T, U, Y, V and W are as defined above.

The compounds of formula (I) and their pharmaceutically acceptable salts have the advantage that they are inhibitors of the enzyme nitric oxide synthase (NOS). In general, the compounds of formula (I) and their pharmaceutically acceptable salts have the advantage that they are inhibitors of the inducible isoform of the enzyme nitric oxide synthase (iNOS). Certain compounds of formula (I) and their pharmaceutically acceptable salts have the advantage that they are additionally or alternatively inhibitors of the neuronal isoform of the enzyme nitric oxide synthase (nNOS). In general, compounds of formula (I) and their pharmaceutically acceptable salts have the advantage that they show selectivity for the inhibition of iNOS and/or nNOS in comparison to the inhibition of the endothelial isoform, eNOS.

The invention further provides a process for the preparation of compounds of formula (I) or a pharmaceutically acceptable salt, enantiomer or racemate thereof.

According to the invention there is also provided a compound of formula (I), or a pharmaceutically acceptable salt thereof, for use as a medicament.

An aspect of the invention provides the use of a compound of formula (I) or a pharmaceutically acceptable salt thereof, in the manufacture of a medicament, for the treatment or prophylaxis of inflammatory disease.

The compounds of the present invention may also be used advantageously in combination with a second pharmaceutically active substance; particularly in combination with a cyclooxygenase inhibitor; more particularly in combination with a selective inhibitor of the inducible isoform of cyclooxygenase (COX-2). Thus, in a further aspect of the invention there is provided the use of a compound of formula (I) or a pharmaceutically acceptable salt thereof, in combination with a COX-2 inhibitor for the treatment of inflammation, inflammatory disease and inflammatory related disorders.

In one embodiment, V represents S(O)ₙ and n represents 0.

In another embodiment, V represents O.

In another embodiment, X and Y independently represent Br, Cl, CH₃, CH₂F, CHF₂, CF₃, CH₃CH₂, NH₂, OCH₃, COCH₃ or CN. In yet another embodiment Y represents CN.

In further embodiments, R¹ represents phenyl, pyridyl, thienyl, isoxazolyl, isothiazolyl, oxazolyl or thiazolyl, optionally substituted by one or more substituents selected independently from halogen, C1 to 4 alkyl, C1 to 4 alkoxy, OH, CN, NO₂ or NR⁵R⁶; said alkyl or alkoxy group being optionally further substituted by one or more fluorine atoms.

In one embodiment, R² represents H or C1 to 4 alkyl; said alkyl being optionally substituted by hydroxy. In another embodiment, R² represents H or CH₃.

In one embodiment, R³ represents H or CH₃.

In one embodiment, each R⁴ represents H or F.

In another embodiment, one of the groups T, U and W represents N, and the other two groups independently represent CH or CF. In a particular embodiment, W represents N and T and U each represent CH.

In a particular embodiment, the compounds of formula (I) have the absolute stereochemistry as shown in formula (Ib): 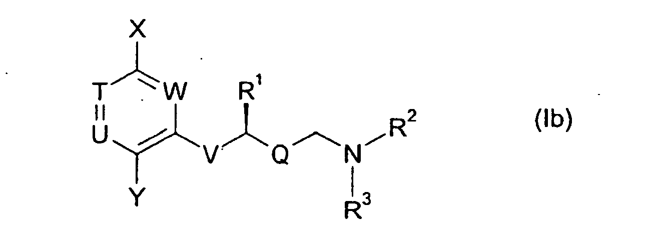 In one particular aspect the invention relates to compounds of formula (I) wherein V represents O or S; X and Y independently represent Br, Cl, CH₃, CH₂F, CHF₂, CF₃, CH₃CH₂, NH₂, OCH₃, COCH₃ or CN; R¹ represents optionally substituted phenyl, pyridyl, thienyl, isoxazolyl, isothiazolyl, oxazolyl or thiazolyl; R² represents H or C1 to 4 alkyl; said alkyl being optionally substituted by hydroxy; R³ represents H or CH₃; R⁴ represents H or F; one of the groups T, U and W represents N, and the other two groups independently represent CH or CF; and pharmaceutically acceptable salts thereof.

Particular compounds of the invention include:
2-[[(1*R*)-3-(methylamino)-1-phenylpropyl]oxy]-6-(trifluoromethyl)-3-pyridinecarbonitrile;
2-[[(1*R*)-3-amino-1-phenylpropyl]thio]-6-methyl-3-pyridinecarbonitrile;
2-[[(1*R*)-3-amino-1-phenylpropyl]thio]-6-(fluoromethyl)-3-pyridinecarbonitrile;
2-[[(1*R*)-3-amino-1-phenylpropyl]thio]-6-(difluoromethyl)-3-pyridinecarbonitrile;
2-[[(1*R*)-3-amino-1-(5-isoxazolyl)propyl]oxy]-6-(trifluoromethyl)- 3-pyridinecarbonitrile;
2-[[(1*R*)-3-amino-1-(5-isoxazolyl)propyl]thio]-6-methyl-3-pyridinecarbonitrile;
2-[[(1*R*)-3-amino-1-(3-thienyl)propyl]oxy]-6-(trifluoromethyl)-3-pyridinecarbonitrile;
2-[[(1*R*)-3-[(3-hydroxypropyl)amino]-1-(3-thienyl)propyl]oxy]-6-(trifluoromethyl)-3-pyridinecarbonitrile;
3-[[(3R)-3-[[3-cyano-6-(trifluoromethyl)-2-pyridinyl]oxy]-3-(3-thienyl)propyl]amino]-*N-*methyl propanamide;
2-[[3-amino-1-(5-isothiazolyl)propyl]thio]-6-(trifluoromethyl)-3-pyridinecarbonitrile;
2-[[(1*R*)-3-amino-1-(3-isoxazolyl)propyl]oxy]-6-(trifluoromethyl)-3-pyridinecarbonitrile;
2-[[(1*R*)-3-amino-1-(2-thiazolyl)propyl]oxy]-6-(trifluoromethyl)-3-pyridinecarbonitrile;
(R)-2-(3-dimethylamino-1-isoxazol-5-yl-propoxy)-6-trifluoromethyl-nicotinonitrile;
6-amino-4-[[(1*R*)-3-amino-1-(5-isoxazolyl)propyl]thio]-3-pyridinecarbonitrile;
γ-[(5-chloro-2-methoxy-4-pyridinyl)thio]-(γ⁵*R*)-5-isoxazolepropanamine, 4-[[(1*R*)-3-amino-1-(5-isoxazolyl)propyl]thio]-6-methoxy-3-pyridinecarbonitrile;
4-[[(1*R*)-3-[(2-hydroxyethyl)amino]-1-(5-isoxazolyl)propyl]thio]-6-methoxy-3-pyridinecarbonitrile;
2-[[(1*R*)-3-amino-1-(3-chloro-5-isoxazolyl)propyl]oxy]-6-(trifluoromethyl)- 3-pyridinecarbonitrile;
2-[[(1 *R*)-3-amino-1-(3-chloro-5-isoxazolyl)propyl]thio]-6-methyl-3-pyridinecarbonitrile;
2-[3-amino-1-(3-methyl-4-isoxazolyl)propoxy]-6-(trifluoromethyl)- 3-pyridinecarbonitrile;
2-[[3-amino-1-(5-isothiazolyl)propyl]thio]-6-methyl-3-pyridinecarbonitrile;
2-[3-amino-1-(5-isothiazolyl)propoxy]-6-methyl-3-pyridinecarbonitrile;
2-[3-amino-1-(3-isothiazolyl)propoxy]-6-methyl-3-pyridinecarbonitrile;
2-[[(1R)-3-amino-1-(5-methyl-3-isoxazolyl)propyl]oxy]-6-(trifluoromethyl)-3-pyridinecarbonitrile;
4-[[(1*R*)-3-amino-1-(3-isoxazolyl)propyl]oxy]-6-methoxy-3-pyridinecarbonitrile;
2-[[3-amino-1-(4-methyl-3-isoxazolyl)propyl]thio]-6-methyl-3-pyridinecarbonitrile;
2-[3-amino-1-(2-oxazolyl)propoxy]-6-(trifluoromethyl)-3-pyridinecarbonitrile;
4 -[[3-amino-1-(4-chloro-5-thiazolyl)propyl]thio]-6-methyl-3-pyridinecarbonitrile;
2-[1-(4-chloro-1,3-thiazol-5-yl)-3-(methylamino)propoxy]-6-methylnicotinonitrile;
2-[[1-(3-fluorophenyl)-3-(methylamino)propyl]oxy]pyridine-3-carbonitrile;
6-acetyl-2-[[3-(methylamino)-1-phenylpropyl]thio]-3-pyridinecarbonitrile;
5-fluoro-6-methyl-2-[[(1*R*)-3-(methylamino)-1-phenylpropyl]thio]-3-pyridinecarbonitrile;
6-ethyl-5-fluoro-2-[[(1*R*)-3-(methylamino)-1-phenylpropyl]thio]-3-pyridinecarbonitrile;
2-[[(1*R*)-3-[(2-hydroxyethyl)methylamino]-1-phenylpropyl]thio]-6-(trifluoromethyl)- 3-pyridinecarbonitrile;
2-[[(1*R*)-3-[(2-hydroxyethyl)propylamino]-1-phenylpropyl]oxy]-6-(trifluoromethyl) 3-pyridinecarbonitrile;
2-[[(1*R*)-3-[(3-hydroxypropyl)amino]-1-phenylpropyl]oxy]-6-(trifluoromethyl)- 3-pyridinecarbonitrile;
2-[[(1*R*)-3-(methylpropylamino)-1-phenylpropyl]oxy]-6-(trifluoromethyl)- 3-pyridinecarbonitrile;
2-[[(1*R*)-3-[[(2*S*)-2-hydroxypropyl]amino]-1-phenylpropyl]oxy]-6-(trifluoromethyl)-3-pyridinecarbonitrile;
2-[[(1*R*)-1-phenyl-3-[(phenylmethyl)amino]propyl]oxy]-6-(trifluoromethyl)- 3-pyridinecarbonitrile;
2-[[(1*R*)-3-[(1,1-dimethylethyl)amino]-1-phenylpropyl]oxy]-6-(trifluoromethyl)-3-pyridinecarbonitrile;
2-[[(1*R*)-3-[[2-(4-hydroxyphenyl)ethyl]amino]-1-phenylpropyl]oxy]-6-(trifluoromethyl)-3-pyridinecarbonitrile;
2-[4-amino-1-(5-isoxazolyl)butoxy]-6-(trifluoromethyl)- 3-pyridinecarbonitrile;
2-[[4-amino-1-(5-isoxazolyl)butyl]thio]-6-methyl-3-pyridinecarbonitrile;
4-[[4-amino-1-(5-isoxazolyl)butyl]thio]-6-methoxy-3-pyridinecarbonitrile; and pharmaceutically acceptable salts thereof.

Unless otherwise indicated, the term "C 1 to 4 alkyl" referred to herein denotes a straight or branched chain alkyl group having from 1 to 4 carbon atoms. Examples of such groups include methyl, ethyl, n-propyl, i-propyl, n-butyl, i-butyl and t-butyl.

Unless otherwise indicated, the term "C3 to 6 cycloalkyl" referred to herein denotes a cycloalkyl group having from 3 to 6 carbon atoms. Examples of such groups include cyclopropyl, cyclopentyl and cyclohexyl.

Unless otherwise indicated, the term "C1 to 4 alkoxy" referred to herein denotes a straight or branched chain alkoxy group having from 1 to 4 carbon atoms. Examples of such groups include methoxy, ethoxy, n-propoxy, i-propoxy and t-butoxy.

The term "C1 to 4 alkylthio" is to be interpreted analogously.

Unless otherwise indicated, the term "halogen" referred to herein denotes fluoro, chloro, bromo and iodo.

Examples of a five or six membered aromatic heterocyclic ring containing 1 to 3 heteroatoms independently selected from O, S and N include furan, thiophene, pyridine, thiazole, imidazole, oxazole, triazole, oxadiazole, thiadiazole and pyrimidine.

Examples of a five or six membered saturated heterocyclic ring containing 1 to 3 heteroatoms independently selected from O, S and N include morpholine, pyrrolidine, tetrahydrofuran, piperidine and piperazine.

Examples of a "C1 to 4 alkyl or C1 to 4 alkoxy optionally further substituted by one or more fluorine atoms" include CH₂F, CHF₂, CF₃, CF₃CF₂, CF₃CH₂, CH₂FCH₂, CH₃CF₂, CF₃CH₂CH₂, OCF₃ and OCH₂CF₃.

According to the invention, we further provide a process for the preparation of compounds of formula (I), or a pharmaceutically acceptable salt, enantiomer or racemate thereof which comprises:
(a) reaction of a compound of formula (II) 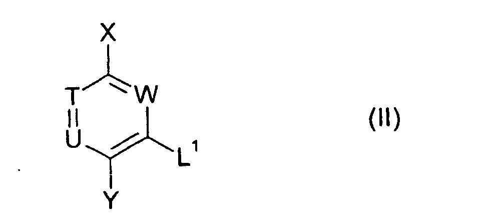 wherein T, U, X, Y and W are as defined in formula (I) and L¹ represents a leaving group, with a compound of formula (III) 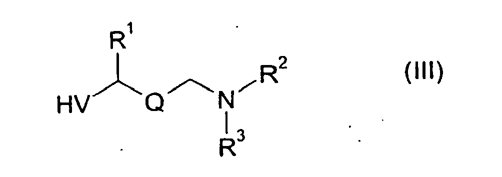 wherein R¹, R², R³, Q and V are as defined in formula (I); or
(b) reaction of a compound of formula (IV) 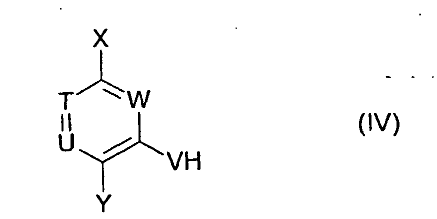 wherein T, U, W, X, Y and V are as defined in formula (I), with a compound of formula (V) 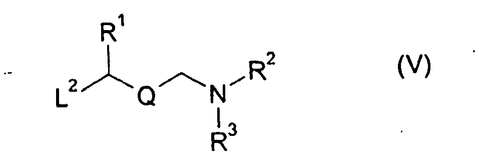 wherein R¹, R², R³ and Q are as defined in formula (I) and L² is a leaving group; or
(c) reaction of a compound of formula (VI) 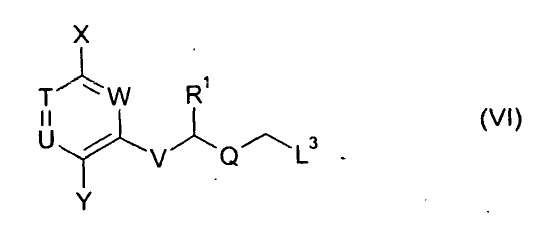 wherein R¹, Q, T, U, W, X, Y and V are as defined in formula (I) and L³ is a leaving group,
   with a compound of formula (VII)

   R²R³NH (VII)

   wherein R² and R³ are as defined in formula (I); or
(d) reduction of a compound of formula (VIII) 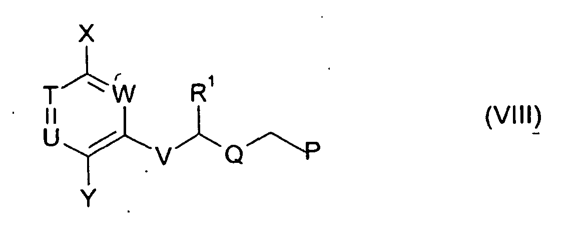 wherein R¹, Q, T, U, W, X, Y and V are as defined in formula (I) and P represents azide (N₃); or
(e) hydrolysis of a compound of formula (VIII) 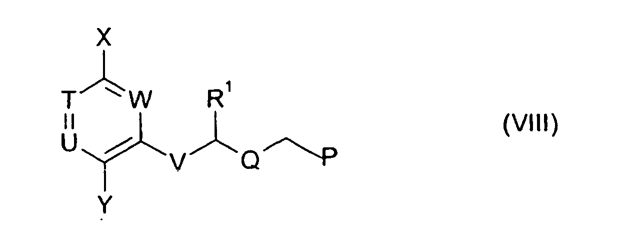 wherein R¹, T, U, W, X, Y and V are as defined in formula (I) and P represents an imide group;

and where desired or necessary converting the resultant compound of formula (I), or another salt thereof, into a pharmaceutically acceptable salt thereof; or converting one compound of formula (I) into another compound of formula (I); and where desired converting the resultant compound of formula (I) into an optical isomer thereof.

In process (a), the reaction is performed by treating a nucleophile of formula (III) with an electrophile of formula (II) in an inert solvent. Suitable leaving groups L¹ include sulphonates and halides, particularly fluoride or chloride. The reaction is generally performed in the presence of a non-nucleophilic base such as sodium hydride or caesium carbonate. Suitable organic solvents are those such as N,N-dimethylformamide, N-methyl-2-pyrrolidinone, tetrahydrofuran and dimethylsulfoxide. The reaction is generally conducted at a temperature between 0 °C and the boiling point of the solvent.

In process (b), the reactants (IV) and (V) are coupled together in a suitable inert solvent such as tetrahydrofuran using, for example, Mitsunobu conditions. Thus, for example, the reactants are treated with a phosphine derivative and an azo derivative at a suitable temperature, generally between 0 °C and the boiling point of the solvent. Suitable phosphine derivatives include triphenylphosphine and tributylphosphine. Suitable azo derivatives include diethyl azodicarboxylate, diisopropyl azodicarboxylate and 1,1'-(azodicarbonyl)dipiperidine. Suitable leaving groups L² include hydroxy.

Alternatively in process (b), the reaction is performed by treating a nucleophile of formula (IV) with an electrophile of formula (V) in an inert solvent. Suitable leaving groups L² include sulphonates and halides, particularly chloride or bromide. The reaction is generally performed in the presence of a non-nucleophilic base such as sodium hydride or caesium carbonate. Suitable organic solvents are those such as N,N-dimethylformamide, N-methyl-2-pyrrolidinone, tetrahydrofuran and dimethylsulfoxide. The reaction is generally conducted at a temperature between 0 °C and the boiling point of the solvent.

In process (c), the compounds of formulae (VI) and (VII) are reacted together in a suitable inert solvent such as dimethylsulphoxide or tetrahydrofuran. The reaction is generally carried out in the presence of a base. The base may be either an added component or an excess of the amine (VII). Suitable leaving groups L³ include iodide and p-toluenesulphonate.

In processes (d) and (e), the reactions are carried out using standard conditions that will be well known to the man skilled in the art.

It will be apparent to a person skilled in the art that in the above processes it may be desirable or necessary to protect an amine or hydroxyl or other potentially reactive group. Suitable protecting groups and details of processes for adding and removing such groups may be found by reference to the standard text "Protective Groups in Organic Synthesis", 3rd Edition (1999) by Greene and Wuts.

In one preferred embodiment, amine groups are protected as carbamate derivatives, for example, as t-butyloxycarbamates.

Specific examples of the use of protecting groups are given in the Examples section.

The present invention includes compounds of formula (I) in the form of salts, in particular acid addition salts. Suitable salts include those formed with both organic and inorganic acids. Such acid addition salts will normally be pharmaceutically acceptable although salts of non-pharmaceutically acceptable acids may be of utility in the preparation and purification of the compound in question. Thus, preferred salts include those formed from hydrochloric, hydrobromic, sulphuric, phosphoric, citric, tartaric, lactic, pyruvic, acetic, succinic, fumaric, maleic, methanesulphonic and benzenesulphonic acids.

Salts of compounds of formula (I) may be formed by reacting the free base, or a salt, enantiomer or racemate thereof, with one or more equivalents of the appropriate acid. The reaction may be carried out in a solvent or medium in which the salt is insoluble or in a solvent in which the salt is soluble, for example, water, dioxane, ethanol, tetrahydrofuran or diethyl ether, or a mixture of solvents, which may be removed *in vacuo* or by freeze drying. The reaction may also be a metathetical process or it may be carried out on an ion exchange resin.

Compounds of formula (III) may be prepared by reaction of a compound of formula (IX) 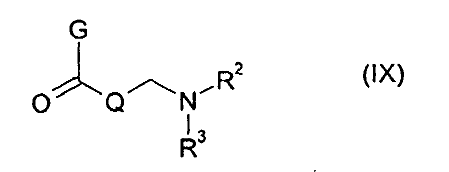 wherein R², R³ and Q are as defined in formula (I), and G represents H, Cl or NCH₃(OCH₃), with an organometallic derivative, R¹―M, wherein R¹ is as defined in formula (I) and M represents a metallic residue such as lithium or magnesium-halide, followed if necessary by reduction. The resulting compound of formula (III) wherein V represents oxygen may then be subsequently converted into compounds of formula (III) wherein V represents sulphur.

Compounds of formulae (II), (IV), (V), (VI), (VIII) and (IX) are either known or may be prepared by conventional methods that will be readily apparent to the man skilled in the art.

Intermediate compounds may be used as such or in protected form. Protecting groups and details of processes for their removal may be found by reference to the standard text "Protective Groups in Organic Synthesis", 3rd Edition (1999) by Greene and Wuts.

The compounds of the invention and intermediates thereto may be isolated from their reaction mixtures and, if necessary further purified, by using standard techniques.

The compounds of formula I may exist in enantiomeric forms. Therefore, all enantiomers, diastereomers, racemates and mixtures thereof are included within the scope of the invention. The various optical isomers may be isolated by separation of a racemic mixture of the compounds using conventional techniques, for example, fractional crystallisation, or HPLC.

Intermediate compounds may also exist in enantiomeric forms and may be used as purified enantiomers, diastereomers, racemates or mixtures.

The compounds of formula (I), and their pharmaceutically acceptable salts are useful because they possess pharmacological activity in animals. In particular, the compounds are active as inhibitors of the enzyme nitric oxide synthase. More particularly, they are inhibitors of the inducible isoform of the enzyme nitric oxide synthase and as such are predicted to be useful in therapy, for example, as anti-inflammatory agents.

The compounds and their pharmaceutically acceptable salts are indicated for use in the treatment or prophylaxis of diseases or conditions in which synthesis or oversynthesis of nitric oxide synthase forms a contributory part. In one aspect, the compounds are indicated for use in the treatment of inflammatory conditions in mammals including man.

Conditions that may be specifically mentioned are:
osteoarthritis, rheumatoid arthritis, rheumatoid spondylitis, gouty arthritis and other arthritic conditions, inflamed joints;
eczema, psoriasis, dermatitis or other inflammatory skin conditions such as sunburn; inflammatory eye conditions including uveitis, glaucoma and conjunctivitis;
lung disorders in which inflammation is involved, for example, asthma, bronchitis, chronic obstructive pulmonary disease, pigeon fancier's disease, farmer's lung, acute respiratory distress syndrome;
bacteraemia, endotoxaemia (septic shock), aphthous ulcers, gingivitis, pyresis, pain, meningitis and pancreatitis;
conditions of the gastrointestinal tract including inflammatory bowel disease, Crohn's disease, atrophic gastritis, gastritis varialoforme, ulcerative colitis, coeliac disease, regional ileitis, peptic ulceration, irritable bowel syndrome, reflux oesophagitis, damage to the gastrointestinal tract resulting from infections by, for example, *Helicobacter pylori*, or from treatments with non-steroidal anti-inflammatory drugs;
and other conditions associated with inflammation.

The compounds will also be useful in the treatment and alleviation of acute pain or persistent inflammatory pain or neuropathic pain or pain of a central origin.

The compounds may also be useful in the treatment of cancer.

We are particularly interested in the conditions inflammatory bowel disease, rheumatoid arthritis, osteoarthritis, chronic obstructive pulmonary disease, cancer and pain.

The compounds of formula (I) and their pharmaceutically acceptable salts, enantiomers and racemates may also be useful in the treatment or prophylaxis of diseases or conditions in addition to those mentioned above. For example, the compounds may be useful in the treatment of atherosclerosis, cystic fibrosis, hypotension associated with septic and/or toxic shock, in the treatment of dysfunction of the immune system, as an adjuvant to short-term immunosuppression in organ transplant therapy, in the control of onset of diabetes, in the maintenance of pancreatic function in diabetes, in the treatment of vascular complications associated with diabetes and in co-therapy with cytokines, for example TNF or interleukins.

The compounds of formula (I) may alternatively be useful in the treatment of hypoxia, for example in cases of cardiac arrest and stroke, neurodegenerative disorders including nerve degeneration and/or nerve necrosis in disorders such as ischaemia, hypoxia, hypoglycaemia, epilepsy, and in external wounds (such as spinal cord and head injury), hyperbaric oxygen convulsions and toxicity, dementia, for example pre-senile dementia, Alzheimer's disease and AIDS-related dementia, Sydenham's chorea; Parkinson's disease, Tourette's Syndrome, Huntington's disease, Amyotrophic Lateral Sclerosis, Multiple Sclerosis, muscular dystrophy, Korsakoff's disease, imbecility relating to a cerebral vessel disorder, sleeping disorders, schizophrenia, depression, pain, autism, seasonal affective disorder, jet-lag, depression or other symptoms associated with Premenstrual Syndrome (PMS), anxiety and septic shock. Compounds of formula (I) may also be expected to show activity in the prevention and reversal of drug addiction or tolerance such as tolerance to opiates and diazepines, treatment of drug addiction, treatment of migraine and other vascular headaches, neurogenic inflammation, in the treatment of gastrointestinal motility disorders, and in the induction of labour.

Prophylaxis is expected to be particularly relevant to the treatment of persons who have suffered a previous episode of, or are otherwise considered to be at increased risk of, the disease or condition in question. Persons at risk of developing a particular disease or condition generally include those having a family history of the disease or condition, or those who have been identified by genetic testing or screening to be particularly susceptible to developing the disease or condition.

For the above mentioned therapeutic indications, the dosage administered will, of course, vary with the compound employed, the mode of administration and the treatment desired. However, in general, satisfactory results are obtained when the compounds are administered at a dosage of the solid form of between 1 mg and 2000 mg per day.

The compounds of formula (I), and pharmaceutically acceptable derivatives thereof, may be used on their own, or in the form of appropriate pharmaceutical compositions in which the compound or derivative is in admixture with a pharmaceutically acceptable adjuvant, diluent or carrier. A further aspect of the invention provides a pharmaceutical composition comprising a compound of formula (I) or a pharmaceutically acceptable salt thereof, in admixture with a pharmaceutically acceptable adjuvant, diluent or carrier. Administration may be by, but is not limited to, enteral (including oral, sublingual or rectal), intranasal, inhalation, intravenous, topical or other parenteral routes. Conventional procedures for the selection and preparation of suitable pharmaceutical formulations are described in, for example, "Pharmaceuticals - The Science of Dosage Form Designs", M. E. Aulton, Churchill Livingstone, 1988. The pharmaceutical composition preferably comprises less than 80% and more preferably less than 50% of a compound of formula (I), or a pharmaceutically acceptable salt thereof.

There is also provided a process for the preparation of such a pharmaceutical composition which comprises mixing the ingredients.

The compounds of formula (I), and pharmaceutically acceptable derivatives thereof, may also be advantageously used in combination with a COX inhibitor, more particularly in combination with a COX-2 inhibitor. Particularly preferred COX-2 inhibitors are Celecoxib and MK-966. The NOS inhibitor and the COX-2 inhibitor may either be formulated together within the same pharmaceutical composition for administration in a single dosage unit, or each component may be individually formulated such that separate dosages may be administered either simultaneously or sequentially.

The invention is illustrated, by the following examples:

The following abbreviations are used:- DMSO (dimethylsulphoxide); DMF (N,N-dimethylformamide); THF (tetrahydrofuran); NMP (N-methylpyrrolidinone).

### Example 1

### 2-[[(1R)-3-(Methylamino)-1-phenylpropyl]oxy]-6-(trifluoromethyl)-3-pyridinecarbonitrile hydrochloride

### a) 1,1-Dimethylethyl [(3R)-3-[[3-cyano-6-(trifluoromethyl)-2-pyridinyl]oxy]-3-phenylpropyl]methylcarbamate

1,1-Dimethylethyl [(3R)-3-hydroxy-3-phenylpropyl]methylcarbamate (265 mg) and 2-chloro-6-(trifluoromethyl)-3-pyridinecarbonitrile (207 mg) were dissolved in DMF (10 ml). Sodium hydride (60% suspension in oil; 80 mg) was added and the mixture was stirred for 2 days. The reaction mixture was poured into water and extracted with diethyl ether three times. The combined organic extracts were washed (brine), dried (magnesium sulphate) and concentrated. Purification (silica, 20% ethyl acetate/hexane as eluent) gave the subtitle compound (160 mg).
MS APCI +ve ^{m}/z 336 ([M-100+H]⁺).
¹H NMR 300MHz (CDCl₃) 8.00 (1H, d), 7.47 (1H, d), 7.35 (4H, m), 6.13 (1H, m), 3.20-3.40 (2H, m), 2.85 (3H, s), 2.40 (1H, m), 2.16 (1H, m), 1.37 (9H, s).

### b) 2-[[(1R)-3-(Methylamino)-1-phenylpropyl]oxy]-6-(trifluoromethyl)-3-pyridinecarbonitrile hydrochloride

The product from step (a) (155 mg) was stirred in 4M hydrogen chloride in dioxane (10 ml) for 1 h. The solvent was evaporated and the residue recrystallised from diethyl ether containing a little ethanol to give the title compound (80 mg).
MS APCI +ve ^{m}/z 336 ([M+H]⁺).
¹H NMR 300MHz (d₆-DMSO) 8.76 (2H, bs), 8.59 (1 H, d), 7.67 (1H, d), 7.30-7.48 (5H, m) 6.20 (1 H, m), 3.02 (2H, m), 2.56 (3H, s), 2.45 (1H, m), 2.30 (1H, m).

### Example 2

### 2-[[(1R)-3-Amino-1-phenylpropyl]thio]-6-methyl-3-pyridinecarbonitrile (E)-butenedioate

### a) (α'S)-α-(2-Azidoethyl)benzenemethanol

(α¹*S*)-α-(2-Chloroethyl)benzenemethanol (1.68 g) and sodium azide (960 mg) in DMSO (15 ml) and water (0.5 ml) were stirred and heated at 50°C for 17 h. The reaction mixture was diluted with water (300 ml) and the products extracted into diethyl ether (2 x 200 ml). The combined extracts were dried (magnesium sulphate) and concentrated to an oil. Purification (silica, 10% acetone/isohexane as eluent) afforded the subtitle compound as a colourless oil (1.6 g).
¹H NMR 300MHz (CDCl₃) 7.41-7.27 (5H, m), 4.88-4.82 (1H, m), 3.55-3.35 (2H, m), 2.11-1.89 (3H, m).

### b) S-[(1R)-3-Azido-1-phenylpropyl]benzenecarbothioate

A solution of diisopropylazodicarboxylate (1.33 ml) in dry THF (25 ml) at 0 °C was treated with triphenylphosphine (1.48 g) and the mixture stirred for 20 minutes. The resulting suspension was then treated with (α¹*S*)-α-(2-azidoethyl)benzenemethanol (1 g) followed by benzenecarbothioic acid (0.66 ml). The suspension rapidly turned into a yellow solution on addition of the benzenecarbothioic acid and after 2.5 h the reaction was complete. The reaction mixture was concentrated to dryness and the residue purified by chromatography (silica, isohexane/diethylether (98:2) as eluent) to give the subtitle compound (800 mg) (as an amber oil).
¹H NMR 300MHz (CDCl₃) 7.94 (2H, m), 7.6-7.2 (8H, m), 4.91 (1H, dd), 3.4-3.2 (2H, m), 2.4-2.2 (2H, m).

### c) 2-[[(1R)-3-Azido-1-phenylpropyl]thio]-6-methyl-3-pyridinecarbonitrile

A mixture of 2-chloro-6-methyl-3-pyridinecarbonitrile (152 mg), the product from step (b) (300 mg) and potassium carbonate (150 mg) in methanol (2 ml) was stirred and refluxed for 1.5 h. The cooled reaction mixture was quenched with water (50 ml) and the products extracted into diethyl ether (2 x 100 ml). The organic extracts were dried (magnesium sulphate) and concentrated to an oil. The crude product was purified by chromatography (silica, isohexane/diethyl ether 1:1 as eluent) to afford the subtitle compound (280 mg) contaminated with ~30% of the starting chloropyridine.
MS APCI +ve ^{m}/z 282 ([M+H-N₂]⁺).

### d) 2-[[(1R)-3-Amino-1-phenylpropyl]thio]-6-methyl-3-pyridinecarbonitrile (E)-butenedioate

The crude product from step (c) (280 mg) was dissolved in THF (10 ml) and the solution treated with water (0.1 ml) and triphenylphosphine (360 mg). The mixture was heated under reflux for 1 h, then concentrated to dryness. The residue was purified by chromatography (silica, 100% ethyl acetate followed by 10% 7M ammonia in methanol/dichloromethane as eluent) followed by preparation of the salt (170 mg) by treatment of the amine in ethanol with (*E*)-butenedioic acid (1 equivalent).
MS APCI +ve ^{m}/z 284 ((M+H)⁺).
¹H NMR 300MHz (d₆-DMSO) 8.05 (1H, d), 7.49-7.15 (6H, m), 6.34 (2H, s), 5.19 (1H, dd), 2.73-2.5 (2H, m), 2.56 (3H, s), 2.3-2.15 (2H, m).

### Example 3

### 2-[[(1R)-3-Amino-1-phenylpropyl]thio]-6-(fluoromethyl)-3-pyridinecarbonitrile ethanedioate

### a) 6-(Fluoromethyl)-2-(methlythio)-3-pyridinecarbonitrile

To a solution of 6-formyl-2-(methylthio)-3-pyridinecarbonitrile (1 g) in ethanol (12 ml) was added sodium borohydride (212 mg). After 2 h, the volatiles were removed and ethyl acetate and water added. The organic layer was separated and the aqueous layer extracted with further ethyl acetate. The combined organic layers were dried (sodium sulphate) and the solvent removed to afford 6-(hydromethyl)-2-(methylthio)-3-pyridinecarbonitrile (1 g) as a yellow solid. This material was taken up in dichloromethane (10 ml) under nitrogen and [bis(methoxyethyl)amino]sulfur trifluoride (1 ml) in dichloromethane (3 ml) was added. After 16 h the reaction mixture was cautiously poured into saturated aqueous sodium bicarbonate solution. The organic layer was separated, dried (sodium sulphate) and the solvent removed. The residue was taken up in methanol and passed through a SCX ion exchange resin eluting with methanol. The solvents were removed to afford the sub-title product (0.88 g) as a yellow solid.
¹H NMR 400MHz (CDCl₃) 7.85 (1H, d), 7.23 (1H, d), 5.48 (2H, d), 2.60 (3H, s).

### b) 6-(Fluoromethyl)-2-(methylsulfonyl)-3-pyridinecarbonitrile

The title compound was prepared by the method of Example 4 step (b) using the product of step (a) above and 3-chloroperoxybenzoic acid. The product was obtained as a pale green oil which solidified upon standing.
¹H NMR 400MHz (CDCl₃) 8.33 (1H, d), 7.87 (1H, d), 5.60 (2H, d), 3.37 (3H, s).

### c) 2-[[(1R)-3-Amino-1-phenylpropyl]thio]-6-(fluoromethyl)-3-pyridinecarbonitrile ethanedioate

*S*-[(1*R*)-3-Azido-1-phenylpropyl]benzenecarbothioate (0.22 g) was dissolved in a solution of ammonia in methanol (10 ml, 7M) and stirred for 3 h under a nitrogen atmosphere. The volatiles were removed in vacuo, and the residue taken up in DMF (6 ml). Solid caesium carbonate (0.48 g) and 6-(fluoromethyl)-2-(methylsulfonyl)-3-pyridinecarbonitrile (0.16 g) were added, and the reaction stirred for 16 h at room temperature. Ethyl acetate and water were added, and the organic layer was separated. It was washed with water, 1M aqueous sodium hydroxide / brine (1:1), dried (sodium sulphate), evaporated and purified by chromatography (silica, 5-10% ethyl acetate in isohexane as eluent) to give the coupled product as an oil. This was taken up in THF (3 ml), triphenylphosphine (0.13 g) added and the solution stirred for 1 h. Water (1 ml) was added and stirring was continued for 16 h. The solvent was removed *in vacuo*, the residue was purified by passage through SCX ion exchange resin. The free base of the title product was taken up in diethyl ether and ethanedioic acid (1 equivalent) in diethyl ether added. The resulting crystals were filtered off and dried in vacuo to yield the title product as a white solid (0.11 g).
¹H NMR 400MHz (CD₃OD) 8.06 (1H, d), 7.47 (2H, d), 7.39-7.27 (4H, m), 5.64-5.42 (2H, m), 5.17 (1H, dd), 3.03 (1H, ddd), 2.87 (1H, ddd), 2.52-2.35 (2H, m).

### Example 4

### 2-[[(1R)-3-Amino-1-phenylpropyl]thio]-6-(difluoromethyl)-3-pyridinecarbonitrile ethanedioate

### a) 6-(Difluoromethyl)-2-(methylthio)-3-pyridinecarbonitrile

To a solution of 6-formyl-2-(methylthio)-3-pyridinecarbonitrile (1 g) in dichloromethane under nitrogen was added [bis(methoxyethyl)amino]sulfur trifluoride (2 ml) followed by ethanol (0.05 ml). After 16 h, the reaction mixture was cautiously poured into saturated aqueous sodium bicarbonate solution. The organic layer was separated and the aqueous layer extracted with further dichloromethane. The combined organic layers were dried (sodium sulphate) and the solvent removed. The residue was taken up in methanol and passed through a SCX ion exchange resin eluting with methanol. The solvents were removed to afford the title product (1.2 g) as a yellow solid.
¹H NMR. 400MHz (CDCl₃) 7.93 (1H, d), 7.38 (1H, d), 6.59 (1H, t), 2.65 (3H, s).

### b) 6-(Difluoromethyl)-2-(methylsulfonyl)-3-pyridinecarbonitrile

To a solution of the product from step (a) above (1.2 g) in dichloromethane (12 ml) at 0 °C was added 3-chloroperoxybenzoic acid (6.8 g of minimum 57% purity). The reaction was warmed to room temperature and stirred for 2 h. The reaction was washed with aqueous sodium bicarbonate solution and dried (sodium sulphate). The solvent was evaporated and the residue taken up in diethyl ether. The organic solution was washed with aqueous sodium metabisulfite solution, ice cold aqueous 0.5M sodium hydroxide solution, brine, and then dried (sodium sulphate). The solvent was removed to give the sub-title compound (0.58 g) as a pale yellow oil.
¹H NMR 400MHz (CDCl₃) 8.44 (1H, d), 8.03 (1H, d), 6.72 (1H, t), 3.42 (3H, s).

### c) 2-[[(1R)-3-Amino-1-phenylpropyl]thio]-6-(difluoromethyl)-3-pyridinecarbonitrile ethanedioate

The title compound was prepared by the method of Example 3 step (c) using benzenecarbothioic acid, *S*-[(1*R*)-3-azido-1-phenylpropyl] ester and 6-(difluoromethyl)-2-(methylsulphonyl)-3-pyridinecarbonitrile to give, after ethanedioate salt formation, the title compound (0.13 g) as a white solid.
¹H NMR 400MHz (CD₃OD) 8.18(1H,d), 7.50 (3H, m), 7.36 (2H, m), 7.30 (1H, m), 6.81 (1H, t), 5.21 (1H, dd), 3.05 (1H, ddd), 2.88 (1H, ddd), 2.55-2.37 (2H, m).

### Example 5

### 2-[[(1R)-3-Amino-1-(5-isoxazolyl)propyl]oxy]-6-(trifluoromethyl)- 3-pyridinecarbonitrile ethanedioate

### a) N-Methoxy-N-methyl-5-isoxazolecarboxamide

A solution of isoxazole-5-carboxylic acid (2.81 g), *N*-methoxy-*N*-methylamine hydrochloride (2.49 g), EDCI (4.96 g), DMAP (3.15 g) and 4-methylmorpholine (2.8 ml) in dichloromethane (20 ml) was stirred for 18 h. 2M Hydrochloric acid was added and the mixture was extracted with dichloromethane (three times). The organic layers were washed with aqueous sodium hydrogen carbonate and then brine, combined, dried (magnesium sulphate), evaporated and purified by chromatography (silica, petrol-ether as eluent) to give the sub-title compound as a colourless oil (2.94 g).
¹H NMR 300MHz (CDCl₃) 8.35 (1H, d), 6.89 (1H, d), 3.83 (3H, s), 3.39 (3H, s).

### b) 3-Chloro-1-(3-isoxazolyl)-1-propanone

Vinyl magnesium bromide (20 ml, 1 M in THF) was added to a solution of the product from step (a) (2.59 g) in THF (35 ml) at -78 °C and warmed to 0 °C over 2.5 h. After cooling to -50 °C the mixture was slowly poured into excess iced 2M hydrochloric acid. The mixture was extracted with ether (five times). The organic extracts were dried (magnesium sulphate) and evaporated to give a brown oil. Hydrogen chloride in diethyl ether (20 ml, 1M) was added and the mixture was stirred for 40 minutes. The solvent was removed *in vacuo* to give the sub-title compound (2.0 g).
¹H NMR 300MHz (CDCl₃) 8.39 (1H, s), 6.96 (1H, s), 3.91 (2H, t), 3.49 (2H, t):

### c) (α⁵-R) α-(2-Chloroethyl)-5-isoxazolemethanol

Borane (4.2 ml, 1M in THF) was added to a solution of (3a*S*)-tetrahydro-1-methyl-3,3-diphenyl-3*H*-pyrrolo[1,2-*c*][1,3,2]oxazaborole (0.06 ml, 1M in toluene) in THF (5 ml) at 0 °C. A solution of the product from step (b) (998 mg) in THF (5 ml) was added slowly and then the mixture was stirred at 20 °C for 4 h. Methanol was added and the solution was evaporated and the residue azeotroped with methanol. Purification by chromatography (silica, petrol-ether as eluent) gave the sub-title compound as a colourless oil (562 mg).
MS APCI +ve ^{m}/z 162 ([M+H]⁺).

### d) (α⁵-R) α-(2-Azidoethyl)-5-isoxazolemethanol

A solution of the product from step (c) (1.62 g) and sodium azide (726 mg) in dry DMSO (11 ml) was stirred at 40 °C for 24 h and then at 60 °C for 1 h. Water was added and the mixture was extracted with ethyl acetate. The organic layers were washed with water, dried (magnesium sulphate), evaporated and purified by chromatography (silica, petrol/diethyl ether as eluent) to give the sub-titte compound as a colourless oil (1.16 g).
¹H NMR 300MHz (CDCl₃) 8.22 (1H, d), 6.25 (1H, d), 5.13 - 5.01 (1H, m), 3.66 - 3.45 (2H, m), 2.54 (1H, d), 2.22 - 2.02 (2H, m).

### e) 1,1-Dimethylethyl [(3R)-3-hydroxy-3-(5-isoxazolyl)propyl]carbamate

Triphenylphosphine (1.80 g) and water (3 ml) were added to a solution of the product from step (d) (1.15 g) in THF (16 ml) and stirred for 18 h. Di-*tert*-butyldicarbonate (1.64 g) was added and the mixture was stirred for two hours. The solvent was removed *in vacuo*, the residue was extracted with dichloromethane and the organic layers were evaporated and purified by chromatography (silica, petrol/diethyl ether (1:3) as eluent) to give the sub-title compound as a colourless oil (1.43 g).
MS APCI +ve ^{m}/z 243 ([M+H]⁺).

### f) 1,1-Dimethylethyl [(3R)-3-[[3-cyano-6-(trifluoromethyl)-2-pyridinyl]oxy]-3-(5-isoxazolyl)propyl]carbamate

Caesium carbonate (5.67 g) was added to a solution of the product from step (e) (1.40 g) and 2-chloro-6-(trifluoromethyl)-3-pyridinecarbonitrile (1.47 g) in DMF (15 ml) at 0 °C and the mixture was stirred at 20 °C for 1 h. 2M Hydrochloric acid and aqueous ammonium chloride were added and the mixture was extracted with ether (three times). The organic layers were washed with water, dried (sodium sulphate), evaporated and purified by chromatography (silica, petrol/diethyl ether as eluent) to give the sub-title compound as a white solid (1.70 g).

Further purification using chiral HPLC (Chiralpak® AD, isohexane/ethanol as eluent) gave the sub-title compound (1.10 g) as a colourless oil.
MS APCl +ve ^{m}/z 413 ([M+H]⁺).
¹H NMR 300MHz (CDCl₃) 8.22 (1H, s), 8.11 (1H, d), 7.41 (1H, dd), 6.46 (1H, dt), 6.42 (1H, s), 4.76 (1H, s), 3.49-3.27 (2H, m), 2.56-2.27 (2H, m), 1.40 (9H, s).

Further elution gave the (3*S*)-isomer as a colourless oil (405 mg).
MS APCI +ve ^{m}/z 413 ([M+H]⁺).

### g) 2-[[(1R)-3-Amino-1-(5-isoxazolyl)propyl]oxy]-6-(trifluoromethyl)-3-pyridinecarbonitrile ethanedioate

4M Hydrogen chloride in dioxan (9 ml) was added to a solution of the product from step (f) (779 mg) in dioxan and stirred for 1.25 h. Aqueous potassium carbonate was added and the mixture was extracted with ethyl acetate (three times) and dichloromethane. The organic extracts were dried (sodium sulphate), evaporated and dissolved in acetonitrile (10 ml) and the ethanedioate salt was prepared by addition of a solution of ethanedioic acid in methanol to give the title compound as a white solid (552 mg). M.p. 179-180 °C.
MS APCI +ve ^{m}/z 313 ([M+H]⁺).
¹H NMR 400MHz (d₆-DMSO) 8.65 (2H, d), 8.60 (1H, d), 7.76 (1H, d), 6.60 (1H, d), 6.43 (1H, dd), 3.01 (2H, t), 2.49 - 2.32 (6H, m).

### Example 6

### 2-[[(1R)-3-Amino-1-(5-isoxazolyl)propyl]thio]-6-methyl-3-pyridinecarbonitrile ethanedioate

### a) (α⁵⁻-S) α-(2-Chloroethyl)-5-isoxazolemethanol

The sub-title compound was prepared by the method of Example 5 step (c) using the product of Example 5 step (b) and (3a*R*)-tetrahydro-1-methyl-3,3-diphenyl- 3*H-*pyrrolo[1,2-*c*][1,3,2]oxazaborole as catalyst.
MS APCI +ve ^{m}/z 162 ([M+H]⁺).

### b) (α⁵-S) α-(2-Azidoethyl)-5-isoxazolemethanol

The sub-title compound was prepared by the method of Example 5 step (d) using the product of step (a) above.
¹H NMR 300MHz (CDCl₃) 8.22 (1H, d), 6.25 (1H, d), 5.13 - 5.01 (1H, m), 3.66 - 3.45 (2H, m), 2.54 (1H, d), 2.22 - 2.02 (2H, m).

### c) 1,1-Dimethylethyl [(3S)-3-hydroxy-3-(5-isoxazolyl)propyl]carbamate

The sub-title compound was prepared by the method of Example 5 step (e) using the product of step (b) above.
MS APCI +ve ^{m}/z 243 ([M+H]⁺).

### d) 1,1-Dimethylethyl [(3R)-3-(benzoylthio)-3-(5-isoxazolyl)propyl]-carbamate

Diisopropylazodicarboxylate (0.52 ml) was added dropwise to triphenylphosphine (695 mg) in dry THF (10 ml) at 0 °C. After 0.5 h, a solution of the product from step (c) (317 mg) and benzenecarbothioic acid (359 mg) in THF (7 ml) was added slowly, the cooling bath was removed and stirring continued overnight. The solvent was removed in *vacuo* and the residue purified by chromatography (silica, petrol/diethyl ether (1:3) as eluent) to give the sub-title compound as a solid (864 mg).
MS APCI +ve ^{m}/z 363 ([M+H]⁺).

### e) 1,1-Dimethylethyl[(3R)-3-[(3-cyano-6-methyl-2-pyridinyl)thio]-3-(5-isoxazolyl)propyl]-carbamate

The product from step (d) (431 mg) was dissolved in 7M ammonia in methanol (5 ml), stirred at room temperature under nitrogen for 3 h, and then the solvent was evaporated. The residue was dissolved in DMF (3 ml) and a mixture of caesium carbonate (410 mg) and 2-chloro-6-methyl-3-pyridinecarbonitrile (144 mg) added. After stirring for 1.75 h, 2M hydrochloric acid and water were added, and the mixture was extracted with ether (three times). The organic layers were washed with water, dried magnesium sulphate), evaporated and purified by chromatography (silica, petrol/diethyl ether as eluent) to give the sub-title compound as a white solid (186 mg).
MS APCI +ve ^{m}/z 375 ([M+H]⁺).

### f) 2-[[(1R)-3-Amino-1-(5-isoxazolyl)propyl]thio]-6-methyl-3-pyridinecarbonitrile

The title compound was prepared from the product of step (e) using the method of Example 5 step (g). M.p. 165-167 °C.
MS APCI +ve ^{m}/z 275 ([M+H]⁺).
¹H NMR 400MHz (d₆-DMSO) 8.55 (1H, d), 8.15 (1H, d), 7.27 (1H, d), 6.57 (1H, d), 5.55 (1H, t), 3.00 - 2.83 (2H, m), 2.57 (3H, s), 2.47 - 2.32 (2H, m).

### Example 7

### 2-[[(1R)-3-Amino-1-(3-thienyl)propyl]oxy]-6-(trifluoromethyl)-3-pyridinecarbonitrile ethanedioate

### a) N-Methoxy-N-methyl-3-thiophene carboxamide

3-Thiophene carboxylic acid (10.78 g) was dissolved in dichloromethane (250 ml) and 4-dimethylaminopyridine (10.28 g), *N*,*O*-dimethylhydroxylamine hydrochloride (8.21 g), *N*-methylmorpholine (8.51 g) and 1-(3-dimethylaminopropyl)-3-ethylcarbodiimide hydrochloride (EDCI) (16.12 g) added and the resultant solution stirred for 24 h at room temperature. The reaction mixture was diluted with dichloromethane (100 ml) and washed with aqueous 2M hydrochloric acid (3 x 30 ml), aqueous sodium bicarbonate solution (2 x 30 ml) and water (3 x 30 ml). The organic phase was dried (magnesium sulphate), filtered and evaporated to give the sub-title compound as a colourless oil (13.35 g).
¹H NMR 300MHz (CDCl₃) 8.07 (1H, dd), 7.58 (1H, dd), 7.29 (1H, dd), 3.66 (3H, s), 3.37 (3H, s).

### b) 1-(3-Thienyl)-2-propen-1-one

The product from step (a) (2.07 g) was dissolved in anhydrous THF (30 ml) and the solution cooled to -10 °C. Vinyl magnesium bromide (1M, 14.5 ml) was added dropwise, keeping the temperature below 0 °C. The resultant solution was stirred at 0 °C for 2.5 h, then allowed to warm to room temperature. The reaction mixture was slowly poured into aqueous 2M hydrochloric acid (200 ml) and ice. The mixture was extracted with ethyl acetate (3 x 70 ml) and the combined extracts were washed with water (2 x 30 ml) and brine (20 ml), dried (magnesium sulphate) and evaporated *in vacuo* to give the sub-title compound (1.29 g).
¹H NMR 300MHz (CDCl₃) 8.11 (1H, dq), 7.61 (1H, td), 7.41 - 7.30 (1H, m), 7.06 (1H, dd), 6.46 (1H, dd), 5.89 (1H, dd).

### c) 3-Chloro-1-(3-thienyl)-1-propanone

The product from step (b) (1.29 g) was dissolved in a mixture of diethyl ether (30 ml) and dichloromethane (20 ml), then 1M hydrogen chloride in diethyl ether (25 ml) was added. The reaction mixture was stirred for 18 h at room temperature. The solvent was removed in *vacuo* to give the sub-title compound (1.48 g).
¹H NMR 300MHz (CDCl₃) 8.09 (1H, dd), 7.56 (1H, dd), 7.35 (1H, dd), 3.90 (2H, t), 3.37 (2H, t).

### d) (R)-α-(2-Chloroethyl)-3-thiophenemethanol

The sub-title compound was prepared by the method of Example 5 step (c) using the product of step (c) above and (3a*S*)-tetrahydro-1-methyl-3,3-diphenyl- 3*H*-pyrrolo[1,2-*c*][1,3,2]oxazaborole as catalyst.
¹H NMR 300MHz (CDCl₃) 7.33 (1H, dd), 7.24 (1H, dt), 7.09 (1H, dd), 5.06 (1H, quintet), 3.75 (1H, ddd), 3.62 - 3.54 (1H, m), 2.31 - 2.11 (2H, m).

### e) 2-[[(1R)-3-Chloro-1-(3-thienyl)-propyl]oxy]-6-(trifluoromethyl)-3-pyridinecarbonitrile

(R)-α-(2-Chloroethyl)-3-thiophenemethanol (1.05 g) and 2-chloro-6-(trifluoromethyl)-3-pyridinecarbonitrile (1.23 g) were dissolved in DMF (20 ml) and sodium hydride added (286 mg, 60% dispersion in oil). The reaction was stirred at room temperature for 2 h before being quenched with ammonium chloride solution and extracted with ethyl acetate (3 x 50 ml). The combined organic extracts were washed with water (3 x 30 ml), dried (magnesium sulphate) and evaporated *in vacuo*. The residue was purified by chromatography (silica, hexane/ethyl acetate as eluent) to give the sub-title compound (1.55 g, 75%).
¹H NMR 400MHz (CDCl₃) 8.03 (1H, d), 7.45 (1H, t), 7.35 - 7.28 (2H, m), 7.20 (1H, d), 6.57 - 6.51 (1H, m), 3.77 - 3.68 (1H, m), 3.62 - 3.54 (1H, m), 2.76 - 2.65 (1H, m), 2.45 - 2.35 (1H, m).

### f) 2-[[(1R)-3-Iodo-1-(3-thienyl)propyl]oxy]-6-(trifluoromethyl)-3-pyridinecarbonitrile

The product from step (e) (1.13 g) was dissolved in a saturated solution of sodium iodide in acetone (100 ml) and refluxed for 20 h, then cooled, and the solvent removed *in vacuo*. The residue was taken up in water (50 ml) and extracted with ethyl acetate (3 x 60 ml). The combined extracts were washed with water (30 ml), dried (magnesium sulphate) and evaporated *in vacuo*. The residue was purified by chromatography (silica, hexane/ethyl acetate as eluent) to afford the sub-title compound (1.35 g, 95%) as an oil that crystallised on standing.
¹H NMR 400MHz (CDCl₃) 8.03 (1H, dd), 7.45 (1H, dd), 7.34 - 7.29 (2H, m), 7.20 (1H, dd), 6.44 (1H, dd), 3.28 (1H, dt), 3.17 (1H, dddd), 2.78 - 2.68 (1H, m), 2.52 - 2.42 (1H, m).

### g) 2-[[(1R)-3-Azido-1-(3-thienyl)propyl]oxy]-6-(trifluoromethyl)-3-pyridinecarbonitrile

The product from step (f) (300 mg) was dissolved in DMSO (10 ml), sodium azide (71 mg) added and the reaction mixture was heated to 65 °C and stirred for 24 h. After cooling, water (30 ml) was added and the mixture extracted with ethyl acetate (3 x 50 ml). The combined extracts were washed with water (3 x 20 ml), dried (magnesium sulphate) and evaporated *in vacuo* to give the sub-title compound (180 mg).
MS APCI +ve ^{m}/z 325 ([M-N₂]⁺).

### h) 2-[[(1R)-3-Amino-1-(3-thienyl)propyl]oxy]-6-(trifluoromethyl)-3-pyridinecarbonitrile ethanedioate

The product from step (g) (180 mg) was dissolved in THF (20 ml), the solution treated with triphenylphosphine (213 mg) and stirred for 1 h at room temperature. Water (4 ml) was added and the reaction stirred for 48 h. Water (30ml) was added and the reaction extracted with ethyl acetate (3 x 50 ml). The combined extracts were washed with water (3 x 20 ml), dried (magnesium sulphate) and evaporated *in vacuo.* The residue was purified by chromatography (silica, 7M ammonia in methanol/dichloromethane as eluent) to give the free base which was converted into the ethandioate salt to give the title compound (120 mg, 56%).
¹H NMR 300MHz (d₆-DMSO) 8.59 (1H, d), 7.69 (1H, d), 7.60 - 7.54 (2H, m), 7.20 (1H, dd), 6.36 - 6.29 (1H, m), 2.91 (2H, t), 2.42 (1H, quintet), 2.35 - 2.22 (1H, m).

### Example 8

### 2-[[(1R)-3-[(3-Hydroxypropyl)amino]-1-(3-thienyl)propyl]oxy]-6-(trifluoromethyl)-3-pyridinecarbonitrile ethanedioate

The product of Example 7 step (f) (250 mg) and 3-amino-1-propanol (257 mg) were dissolved in THF (25 ml) and stirred at room temperature for 48 h. Water (30 ml) was added and the mixture extracted with ethyl acetate (3 x 50 ml). The combined extracts were washed with water (4 x 20 ml), dried (magnesium sulphate) and evaporated *in vacuo.* The residue was purified by chromatography (silica, 7M ammonia in methanol/dichloromethane as eluent) to give the free base which was converted into the ethanedioate salt to give the title compound (101 mg, 37%).
¹H NMR 300MHz (d₆-DMSO) 8.59 (1H, d), 7.69 (1H, d), 7.61 - 7.55 (2H, m), 7.21 (1H, dd), 6.33 (1H, dd), 3.46 (2H, t), 3.07 - 2.92 (4H, m), 2.40 - 2.26 (2H, m), 1.77 - 1.65 (2H, m).

### Example 9

### 3-[[(3R)-3-[[3-Cyano-6-(trifluoromethyl)-2-pyridinyl]oxy]-3-(3-thienyl)propyl]amino]-N-methyl propanamide ethanedioate

The product of Example 7 step (f) (250 mg), 3-amino-N-methyl-propanamide hydrochloride (475 mg) and triethylamine (0.15 ml) were dissolved in THF (25 ml) and stirred at room temperature for 48 h. Water (30 ml) was added and the mixture extracted with ethyl acetate (3 x 50 ml). The combined extracts were washed with water (4 x 20 ml), dried (magnesium sulphate) and evaporated *in vacuo*. The residue was purified by chromatography (silica, 7M ammonia in methanol/dichloromethane as eluent) to give the free base which was converted into the ethanedioate salt to give the title compound (49 mg, 17%).
¹H NMR 300MHz (d₆-DMSO) 8.60 (1H, d), 8.04 (1H, d), 7.69 (1 H, d), 7.60 - 7.55 (2H, m), 7.21 (1H, dd), 6.33 (1H, dd), 3.18 - 2.97 (4H, m), 2.58 (3H, d), 2.49 - 2.42 (3H, m), 2.40 - 2.30 (2H, m).

### Example 10

### 2-[[3-Amino-1-(5-isothiazolyl)propyl]thio]-6-(trifluoromethyl)-3-pyridinecarbonitrile ethanedioate

### a) 1,1-Dimethylethyl [3-(5-isothiazolyl)-3-oxopropyl]-carbamate

Butyl lithium (3.3 ml, 2.5M in hexanes) was added to a solution of isothiazole (860 mg) in THF (15 ml) at -78 °C, and the dark red solution was stirred for 1.5 h at -78 °C. A solution of 1,1-dimethylethyl [3-(methoxymethylamino)-3-oxopropyl]carbamate (1.09 g) in THF (5 ml) was added slowly. After addition of three-quarters of the amide, further butyl lithium (0.7 ml) was added and the mixture stirred for 10 minutes before the amide addition was completed. The mixture was allowed to warm to 20 °C over 20 h, then quenched with aqueous ammonium chloride. The mixture was extracted with diethyl ether and the organic layers were dried (magnesium sulphate), evaporated and purified by chromatography (silica, petrol/diethyl ether as eluent) to give the sub-title compound as a colourless oil (725 mg).
¹H NMR 300MHz (CDCl₃) 8.58 (1H, d), 7.68 (1H, d), 5.07 (1H, s), 3.61 - 3.48 (2H, m), 3.24 - 3.13 (2H, m), 1.48 (7H, s).

### b) 1,1-Dimethylethyl [3-hydroxy-3-(5-isothiazolyl)propyl]-carbamate

The sub-title compound was prepared by the method of Example 5 step (c) using the product of step (a) above.
MS APCI +ve ^{m}/z 259 ([M+H]⁺).

### c) 1,1-Dimethylethyl [3-[[3-cyano-6-(trifluoromethyl)-2-pyridinyl]oxy]-3-(5-isothiazolyl)propyl] carbamate

The sub-title compound was prepared by the method of Example 1 step (a) using the product of step (b) above, but with a reaction time of 1 h.
MS APCI +ve ^{m}/z 429 ([M+H]⁺).

### d) 2-[[3-Amino-1-(5-isothiazolyl)propyl]thio]-6-(trifluoromethyl)-3-pyridinecarbonitrile ethanedioate

The title compound was prepared from the product of step (c) above using the method of Example 5 step (g), with purification of the crude amine by chromatography (silica, dichloromethane/7M ammonia in methanol as eluent) followed by preparation of the salt in isopropanol and methanol. M.p. 223-225 °C.
MS APCI +ve ^{m}/z 329 ([M+H]⁺).
¹H NMR 300MHz (d6-DMSO) 8.64 (1H, d), 8.53 (1H, d), 7.75 (1H, d), 7.51 (1H, d), 6.66 (1H, dd), 3.96 (3H, s), 2.86 (2H, t), 2.45 - 2.21 (2H, m).

### Example 11

### 2-[[(1R)-3-Amino-1-(3-isoxazolyl)propyl]oxy]-6-(trifluoromethyl)-3-pyridinecarbonitrile (E)-butenedioate

### a) N-Methoxy-N-methyl-3-isoxazolecarboxamide

A mixture of 3-isoxazolecarboxylic acid (9.6 g) (Micetich, R.G. *Can. J. Chem*., **1970**, *48*, 467-476), 4-dimethylaminopyridine (11.7 g), *N,O*-dimethylhydroxylamine hydrochloride (9.1 g), *N*-methylmorpholine (9.6 g) and 1-(3-diethylaminopropyl)-3-ethylcarbodiimide hydrochloride (17.1 g) in dichloromethane (350 ml) was stirred at 20 °C overnight. The reaction mixture was then washed with 2M hydrochloric acid (200 ml), saturated sodium bicarbonate (200 ml), brine, dried (magnesium sulphate) and evaporated to give the sub-title compound (8.5 g) as a dark orange oil.
¹H NMR 300MHz (CDCl₃) 8.03 (1H, t), 7.42 (1H, t), 6.87 (1H, dd), 3.72 (3H, s), 3.34 (3H, s).

### b) 3-Chloro-(3-isoxazolyl)-1-propanone

The sub-title compound was prepared by the method of Example 5 step (b) using the product of step (a) above.
¹H NMR 300MHz (CDCl₃) 8.51 (1H, d), 6.79 (1H, d), 3.92 (2H, t), 3.57 (2H, t).

### c) (α³-R)-α-(2-Chloroethyl)-3-isoxazolemethanol

The sub-title compound was prepared by the method of Example 5 step (c) using the product of step (b) above.
¹H NMR 300MHz (CDCl₃) 8.39 (1H, s), 6.41 (1H, s), 5.21-5.16 (1H, m), 3.84-3.78 (1H, m), 3.72-3.66 (1H, m), 2.31-2.25 (2H, m).

### d) (α³-R)-(2-Azidoethyl)-3-isoxazolemethanol

The sub-title compound was prepared by the method of Example 5 step (d) using the product of step (c) above.
¹H NMR 300MHz (CDCl₃) 8.39 (1H, s), 6.41 (1H, s), 5.07 (1H, bd s), 3.62-3.48 (2H, m), 2.12-2.07 (2H, m).

### e) 1,1-Dimethylethyl [(3S)-3-hydroxy-3-(3-isoxazolyl)propyl]carbamate and 1,1-Dimethylethyl [(3R)-3-hydroxy-3-(3-isoxazolyl)propyl]carbamate

The sub-title compound was prepared by the method of Example 5 step (e) using the product of step (d) above.
Purification using chiral HPLC (Chiralpak® AD, *iso*hexane/ethanol as eluent) gave the (3S) sub-title compound (175 mg) as a colourless oil.
¹H NMR 300MHz (CDCl₃) 8.35 (1H, s), 6.46 (1H, s), 4.99-4.91 (1H, m), 4.88 (1H, bd s), 4.20 (1H, s), 3.68-3.56 (1H, m), 3.23-3.16 (1H, m), 2.00-1.90 (2H, m), 1.45 (9H, s).

Further elution gave the (3R) sub-title compound as a colourless oil (1.25 g).
¹H NMR 300MHz (CDCl₃) 8.35 (1H, s), 6.46 (1H, s), 4.99-4.91 (1H, m), 4.88 (1H, bd s), 4.20 (1H, s), 3.68-3.56 (1H, m), 3.23-3.16 (1H, m), 2.00-1.90 (2H, m), 1.45 (9H, s).

### f) 1,1-Dimethylethyl [(3R)-[[3-cyano-6-(trifluoromethyl)-2-pyridinyl]oxy]-3-(3-isoxazolyl)propyl]-carbamate

The sub-title compound was prepared by the method of Example 5 step (f) using the (3R) product of step (e) above.
¹H NMR 300MHz (CDCl₃) 8.40 (1 H, s), 8.11 (1H, d), 7.40 (1H, d), 6.51-6.47 (2H, m), 4.86 (1H, bd s), 3.39-3.31 (2H, m), 2.48-2.26 (2H, m), 1.40 (9H, s).
MS APCI +ve ^{m}/z 313 ([M+H][-Boc]⁺).

### g) 2-[[(1R)-3-Amino-1-(3-isoxazolyl)propyl]oxy]-6-(trifluoromethyl)-3-pyridinecarbonitrile (E)-butenedioate

4M Hydrogen chloride in dioxan (9 ml) was added to a solution of the product from step (f) (1.0 g) in dioxan and stirred for 6 h. Aqueous potassium carbonate was added and the mixture was extracted with ethyl acetate (three times) and dichloromethane. The organic extracts were dried (sodium sulphate) and then evaporated. The residue was dissolved in acetonitrile (10 ml) and the (*E*)-butenedioate salt was prepared by addition of a solution of (*E*)-butenedioic acid in methanol to give the title compound as a white solid (850 mg).
M.p. 160-161 °C.
¹H NMR 300MHz (d₆-DMSO) 8.93 (1H, d), 8.63 (1H, d), 7.72 (1H, d), 6.66 (1H, d), 6.40 (2H, s), 6.39-6.34 (1H, m), 3.07-2.92 (2H, m), 2.46-2.32 (2H, m).
MS APCI +ve ^{m}/z 313 ([M+H]⁺).

### Example 12

### 2-[[(1R)-3-Amino-1-(2-thiazolyl)propyl]oxy]-6-(trifluoromethyl)-3-pyridinecarbonitrile hydrochloride

### a) 1,1-Dimethylethyl [3-oxo-3-(2-thiazolyl)propyl]-carbamate

The sub-title compound was prepared by the method of Example 10 step (a) using 2-bromothiazole instead of isothiazole.
¹H NMR 300MHz (CDCl₃) 8.01 (1H, m), 7.69 (1H, m), 5.05 (1H, br s), 3.57 (2H, q), 3.39 (2H, t), 1.46 (9H, s).

### b) 1,1-Dimethylethyl [(3R)-3-hydroxy-3-(2-thiazolyl)propyl]-carbamate

The sub-title compound was prepared by the method of Example 5 step (c) using the product of step (a) above.
MS APCI +ve ^{m}/z 259 ([M+H]⁺).
¹H NMR 300MHz (CDCl₃) 7.72 (1H, d), 7.29 (1H, d), 5.06-5.02 (1H, m), 4.92 (1H, bd s), 4.71 (1H, s), 3.70-3.58 (1H, m), 3.25-3.16 (1H, m), 2.24-2.216 (1H, m), 1.93-1.87 (1H, m), 1.44 (9H, s).

### c) 1,1-Dimethylethyl [(3R)-[[3-cyano-6-(trifluoromethyl)-2-pyridinyl]oxy]-3-(2-thiazolyl)propyl]-carbamate

The sub-title compound was prepared by the method of Example 1 step (a) using the product of step (b) above.
¹H NMR 300MHz (CDCl₃) 8.11 (1H, d), 7.79 (1H, d), 7.41 (1H, d), 7.34 (1H, d), 6.62 (1H, dd), 4.86 (1H, bd s), 3.43-3.25 (2H, m), 2.55-2.35 (2H, m), 1.39 (9H, s).
MS APCI+ve ^{m}/z 429 ([M+H]⁺).

### d) 2-[[(1R)-3-Amino-1-(2-thiazolyl)propyl]oxy]-6-(trifluoromethyl)-3-pyridinecarbonitrile hydrochloride

4M Hydrogen chloride in dioxan (1 ml) was added to a solution of the product from step (c) (135 mg) in dioxan and the mixture was stirred for 18 h. The white precipitate was collected, washed with ethyl acetate and vacuum dried to give the title compound as a white hygroscopic solid (105 mg). M.p. 151-153 °C.
MS APCI +ve ^{m}/z 329 ([M+H]⁺).
¹H NMR 300MHz (d₆-DMSO) 8.67 (1H, d), 8.16 (3H, bd s), 7.85 (1H, d), 7.80 (1H, d), 7.77 (1H, d), 6.56-6.51 (1H, m), 3.11-3.00 (2H, m), 2.63-2.43 (2H, m).

### Example 13

### (R)-2-(3-Dimethylamino-1-isoxazol-5-yl-propoxy)-6-trifluoromethyl-nicotinonitrile ethanedioate

A solution of the product of Example 5 step (f) (50 mg) in 10% trifluoroacetic acid in dichloromethane (10 ml) was stirred for 20 minutes and then concentrated and the residue treated with dilute aqueous sodium hydrogen carbonate solution (8 ml). Extraction into ethyl acetate (3 x 10 ml) and drying (Na₂SO₄) afforded a pale-yellow oil upon concentration. This material was taken up in acetonitrile (2 ml) and to this was added an excess of 37% w/w aqueous formaldehyde (150 µl) with rapid stirring at ambient temperature. After 5 minutes, sodium cyanoborohydride (16 mg) was added, and after stirring for an additional 20 minutes the mixture was acidified to pH -5 by the dropwise addition of acetic acid. After stirring for 16 h, the solution was concentrated *in vacuo* and subsequently treated with 0.5M sodium hydroxide solution and extracted into dichloromethane (3 x 10 ml), then ethyl acetate (1 x 10 ml). The combined organic extracts were washed with saturated brine (1 x 15 ml), dried (Na₂SO₄), and concentrated. The colourless residue was taken up in dichloromethane (3 ml) and treated with macroporous polystyrene resin-bound toluenesulfonic acid (ca. 360 mg, 1.4 mmol/g loading) with vigorous nitrogen gas agitation at ambient temperature for 2 h. Washing with methanol (2 x 3 mL) and release with 7M ammonia in methanol (3 x 2 ml, 5 minute agitation), followed by concentration, afforded the free base of the title compound (31 mg).
MS APCI + ve^{m}/z 341 ([M+H]⁺).
¹H NMR 300MHz (CDCl₃), 8.21 (1H, d), 8.09 (1H, d), 7.39 1H, (d), 6.50 (1H, t), 6.37 (1H, d), 2.49-2.21 (4H, m), 2.21 (6H, s).

The free base (31 mg) in acetonitrile (1 ml) was added to a solution of ethanedioic acid (8.1 mg) in methanol (0.5 ml). Concentration and drying *in vacuo* overnight yielded the ethanedioate salt (38 mg) as a white gum.
MS APCI +ve ^{m}/z 341 ([M+1]⁺).
¹H NMR 300MHz (MeOH-*d*₄) 8.40 (2H, m), 7.61 (1H, d), 6.56 (2H, s), 3.31-3.42 (3H, m), 2.96 (6H, s), 2.68-2.96 (3H, m).

### Example 14

### 6-Amino-4-[[(1R)-3-amino-1-(5-isoxazolyl)propyl]thiol-3-pyridinecarbonitrile (E)-butenedioate

### a) 2-Chloro-5-(4,5-dihydro-4,4-dimetbyl-2-oxazolyl)-pyridine

A suspension of 2-chloropyridine-5-carboxylic acid (100 g) in thionyl chloride (370 ml) was heated at 80 °C for 4 h. The reaction mixture was evaporated *in vacuo*, the residue azeotroped with toluene and then taken up into dichloromethane (300 ml) The solution was added dropwise over 1 h at 0 °C to a solution of 2-amino-2-methylpropanol (118.8 g) in dichloromethane (300 ml) and then stirred at 20 °C for 16 h. Water (500 ml) was added and the mixture was extracted with dichloromethane (5 x 500 ml). A solid suspension, which formed during extraction, is the required intermediate amide and needs extensive extraction. The organic layer was dried (MgSO₄) and evaporated to leave the intermediate amide (125.5 g). This material was suspended in dichloromethane (200 ml) at 0 °C and thionyl chloride (100 ml) was added dropwise and stirred for 1 h. A thick precipitate formed and more dichloromethane (300 ml) is added and reaction stirred for a further hour. The solvent was removed *in vacuo* to give the product as the hydrochloride salt (120 g). ¹H NMR 300MHz (CD₃OD) 9.03 (1H, t), 8.42 (1H, dd), 7.80(1H, dd), 4.96 (2H, s), 1.68 (6H, s).

The solid was suspended in water (800 ml) and treated portionwise with solid sodium hydrogen carbonate (ca. 70 g) until gas evolution ceased. The mixture was extracted with dichloromethane (2 x 500 ml), dried (MgSO₄) and evaporated to give the sub-title compound (99.5 g).
¹H NMR 300MHz (CDCl₃) 8.90 (1H, dd), 8.17 (1H, dd), 7.37 (1H, dd), 4.14 (2H, s), 1.39 (6H, s).

### b) 5-(4,5-Dihydro-4,4-dimethyl-2-oxazolyl)-2-methoxy-pyridine

The product from step (a) (99.5 g) in methanol (500 ml) was treated with sodium methoxide (0.61 mol of a 25 wt% solution in methanol) and heated at reflux for 12 h. The solvent was removed under reduced pressure and the residue taken up in water (200 ml) and extracted with dichloromethane (2 x 300 ml). The extract was dried (MgSO₄) and evaporated to dryness to give the sub-title compound as an orange oil (85 g).
¹H NMR 300MHz (CDCl₃) 8.68 (1H, dd), 8.10 (1H, dd), 6.75 (1H, dd), 4.09 (2H, s), 3.98 (3H, s), 1.37 (6H, s).

### c) 5-(4,5-Dihydro-4,4-dimethyl-2-oxazolyl)-2-methoxy-4-(methylthio)-pyridine

To 2,2,6,6-tetramethylpiperidine (51.22 g) in THF, under nitrogen, at 0 °C, was added nBuLi (227 ml of a 1.6M solution in hexanes) dropwise and stirred for 15 minutes. The reaction mixture was cooled to -78 °C and the product from step (b) above (43.97 g) in THF (50 ml) was added dropwise. The cooling bath was removed and the reaction temperature was allowed to warm up to -20 °C and kept at this temperature for 30 minutes. It was then cooled to -78 °C and dimethyldisulfide (80 ml) was dripped in. The reaction mixture temperature rose to -30 °C during this addition. The cooling bath was then removed and the reaction was stirred to room temperature for 12 h. The resulting red solution was quenched with water and concentrated to ca. 600 ml on a rotary evaporator. Water was added (500 ml) and the mixture was extracted with ethyl acetate (2 x 600 ml).

The combined organics were washed with citric acid (500 ml of a 1M aqueous solution), dried (MgSO₄) and evaporated to give the sub-title compound as a pale yellow solid (58.5 g).
¹H NMR 300MHz (CDCl₃) 8.50 (1H, s), 6.52 (1H, s), 4.04 (2H, s), 3.97 (3H, s), 2.40 (3H, s), 1.40 (6H, s).

### d) 6-Methoxy-4-(methylthio)-3-pyridinecarbonitrile

A stirred solution of the oxazoline from step (c) above (45 g) in pyridine (350 ml) was treated with phosphorus oxychloride (68 ml) and the mixture stirred under reflux for 4.5 h. The dark brown solution was cooled to room temperature and then cautiously poured onto ice (1 kg). The resulting suspension was filtered and the solid washed with water (300 ml), 2M HCl (100 ml) and again with water (300 ml). The damp product was dissolved in dichloromethane (600 ml) and the solution dried (MgSO₄). Activated charcoal was added (15 g) and the suspension filtered. Concentration of the filtrate and trituration of the solid with 40-60 petroleum ether gave the sub-title compound as a very pale pink solid (26 g). ¹H NMR 300MHz (CDCl₃) 8.31 (1H, s), 6.51 (1H, s), 3.98 (3H, s), 2.52 (3H, s).

### e) 6-Methoxy-4-(methylsulfonyl)-3-pyridinecarbonitrile

A solution of the product from step (d) above (13 g) in dichloromethane (150 ml) was cooled to 0 °C and treated portionwise with MCPBA (21.74 g of ~57% purity) over 10 minutes. The mixture was allowed to slowly warm up to 20 °C. After 8 h, LC/MS indicated a mixture of sulfoxide / sulfone products (72:28). Additional MCPBA was added (7.2 g) and after a further 4 h LC/MS indicated a 50:50 mixture of products. More MCPBA was added (12 g) and stirring continued for a further 2 h before reaction was complete. The reaction was cooled to 0 °C and treated with excess aqueous sodium metabisulfite solution. The organic layer was washed with saturated sodium hydrogen carbonate solution (3 x 200 ml), dried (MgSO₄) and evaporated to give the sub-title compound as a white solid (11.2 g).

### f) 1,6-dihydro-4-(methylsulfonyl)-6-oxo-3-pyridinecarbonitrile

6-Methoxy-4-(methylsulfonyl)-3-pyridinecarbonitrile from step (e) (5.1 g) was dissolved in acetonitrile (200 ml) and sodium iodide (7.28 g) and trimethylsilylchloride (5.28 g) were added. The reaction was heated under reflux for 48 h and then cooled and the solvent evaporated *in vacuo*. The residue was partitioned between water (120 ml) and ethyl acetate (120 ml). After shaking, the layers were filtered and the solid collected and dried in a vacuum oven at 60 °C to give the sub-title compound as an off-white solid (3.6 g). ¹H NMR 400MHz (d₆-DMSO) 13.15 (1H, bs), 8.58 (1H, s), 6.89 (1H, s), 3.39 (3H, s).

### g) 5-Cyano-4-(methylsulfonyl)-2-pyridinyl trifluoromethanesulfonate

Triflic anhydride (3.1 ml) was added to a solution of the product from step (f) (1.83 g) and triethylamine (2.7 ml) in acetonitrile (50 ml) at -20 °C and stirred at 0 °C for 3 h. Water was added and the mixture was extracted with dichloromethane. The organic extracts were dried (Na₂SO₄), evaporated and purified by chromatography (silica, isohexane - dichloromethane as eluent) gave the sub-title compound (2.60 g).
¹H NMR 300MHz (CDCl₃) 8.94 (1H, s), 7.91 (1H, s), 3.37 (3H, s).

### h) 6-Amino-4-(methylsulfonyl)-3-pyridinecarbonitrile

0.5M Ammonia in dioxane (2 ml) was added to a solution of the product from step (g) above (164 mg) in THF (2 ml) and the mixture was stirred for 16 h. The solvent was removed *in vacuo* and the residue purified by chromatography (silica, *iso*hexane/ethyl acetate as eluent) to give the sub-title compound (33 mg).
¹H NMR 300MHz (d₆-DMSO) 8.57 (1H, s), 7.78 (2H, s), 7.05 (1H, s), 3.35 (3H, s).

### i) 1,1-Dimethylethyl [(3R)-3-[(2-amino-5-cyano-2-pyridinyl)thio]-3-(5-isoxazolyl)propyl]-carbamate

The product from Example 6 step (d) (286 mg) was dissolved in 7M ammonia in methanol (2 ml), stirred at room temperature under nitrogen for 4 h and then the solvent was evaporated. The residue was dissolved in acetonitrile (3 ml) and a mixture of caesium carbonate (410 mg) and the product from step (h) above (178 mg) added at 0 °C. After stirring at 0 °C for 1 h and then at 20°C for 1 h, aqueous ammonium chloride and water were added, and the mixture was extracted with ethyl acetate (three times). The organic layers were washed with water, dried (MgSO₄), evaporated and purified by chromatography (silica, diethyl ether as eluent) to give the sub-title compound as a white solid (132 mg).
MS APCI +ve ^{m}/z 376 ([M+H]⁺).

### j) 6-Amino-4-[[(1R)-3-amino-1-(5-isoxazolyl)propyl]thio]-3-pyridinecarbonitrile (E)-butenedioate

The product from step (i) above (132 mg) in methanol (0.5 ml) and 4M HCl in dioxane (1 ml) was stirred for 2 h. The solvent was removed *in vacuo*, the residue purified by reversed phase HPLC and the (E)-butenedioate salt prepared in methanol - ethyl acetate to give the title compound (75 mg). M.p. 160-163 °C.
MS APCI +ve ^{m}/z 276 ([M+H]⁺).
¹H NMR 400MHz (DMSO-d₆) 8.57 (1H, d), 8.23 (1H, s), 7.23 (2H, s), 6.62 (2H, s), 6.45 (2H, s), 5.19 (1H, d), 2.87 (2H, t), 2.43 - 2.25 (2H, m).

### Example 15

### γ-[(5-Chloro-2-methoxy-4-pyridinyl)thio]-(γ⁵R)-5-isoxazolepropanamine (E)-butenedioate

### a) 2,5-Dichloro-4-(methylthio)-pyridine

To DMF (3.1 ml) in THF (20 ml), under nitrogen, at 0 °C, was added nBuLi (8.9 ml of a 2.5M solution in hexanes) dropwise and the reaction mixture was stirred for 15 minutes. The reaction mixture was added dropwise to a solution of 2,5-dichloropyridine (3 g) in THF (20 ml) at -78 °C. After 2 h, dimethyldisulfide (2.4 ml) was added and the reaction temperature was allowed to warm up to 0 °C. Water was added and the mixture was extracted with ethyl acetate. The combined organics were dried (Na₂SO₄) and evaporated to give the sub-title compound as a yellow solid (3 g).
¹H NMR 400MHz (CDCl₃) 8.18 (1H, s), 7.02 (1H, s), 2.50 (3H, s).

### b) 5-Chloro-2-methoxy-4-(methylthio)-pyridine

The product of step (a) (1.4 g) in methanol (20 ml) was treated with sodium methoxide (8.2 ml of a 25 wt% solution in methanol) and heated at reflux for 48 h. The solvent was removed under reduced pressure and the residue was partitioned between water (50 ml) and dichloromethane (50 ml). The organic phase was dried (MgSO₄) and evaporated to dryness. Purification by chromatography (silica, dichloromethane as eluent) gave the sub-title compound (345 mg) as a white solid. -
MS APCI +ve ^{m}/z 189 ([M+H]⁺).

### c) 5-Chloro-2-methoxy-4-(methylsulfonyl)pyridine

The sub-title compound was prepared by the method of Example 4 step (b) using the product from step (b).
MS APCI +ve ^{m}/z 222/224 ([M+H]⁺).

### d) 1,1-Dimethylethyl [(3R)-3-[(5-chloro-2-methoxy-4-pyridinyl)thio]-3-(5-isoxazolyl)propyl]-carbamate

The sub-title compound was prepared from the product of step (c) and the product of Example 6 step (d) using the method of Example 6 step (e).
MS APCI +ve ^{m}/z 400/2 ([M+H]⁺).

### e) γ-[(5-Chloro-2-methoxy-4-pyridinyl)thio]-(γ⁵R)-5-isoxazolepropanamine (E)-butenedioate

The title compound was prepared by the method of Example 5 step (g) using the product from step (d) above and with preparation of the (*E*)-butenedioate salt in methanol/acetonitrile. M.p. 142-143 °C.
MS APCI +ve ^{m}/z 300/2 ([M+H]⁺).
¹H NMR 400MHz (DMSO-d₆) 8.56 (1H, d), 8.16 (1H, s), 6.92 (1H, s), 6.60 (1H, d), 6.45 (2H, s), 5.33 (1H, t), 3.87 (3H, s), 2.89 - 2.76 (2H, m), 2.35 - 2.21 (2H, m).

### Example 16

### 4-[[(1R)-3-Amino-1-(5-isoxazolyl)propyl]thio]-6-methoxy-3-pyridinecarbonitrile (E)-butenedioate

### a) 1,1-Dimethylethyl [(3R)-3-[(5-cyano-2-methoxy-4-pyridinyl)thio]-3-(5-isoxazolyl)propyl]carbamate

The sub-title compound was prepared from the product of Example 14 step (e) and the product of Example 6 step (d) using the method of Example 6 step (e).
MS APCI +ve ^{m}/z 391 ([M+H]⁺).

### b) 4-[[(1R)-3-Amino-1-(5-isoxazolyl)propyl]thio]-6-methoxy-3-pyridinecarbonitrile (E)-butenedioate

The title compound was prepared by the method of Example 5 step (g) using the product from step (a) above and with preparation of the (*E*)-butenedioate salt in methanol/acetonitrile. M.p. 152-153 °C.
MS APCI +ve ^{m}/z 291 ([M+H]⁺).
¹H NMR 400MHz (DMSO-d₆) 8.61 (1H, s), 8.57 (1H, d), 7.07 (1H, s), 6.62 (1H, d), 6.44 (2H, s), 5.49 (1H, t), 3.93 (3H, s), 2.90 - 2.81 (2H, m), 2.43 - 2.26 (2H, m).

### Example 17

### 4-[[(1R)-3-[(2-Hydroxyethyl)amino]-1-(5-isoxazolyl)propyl]thio]-6-methoxy-3-pyridinecarbonitrile (E)-butenedioate

A solution of the free base of the product of Example 16 (53 mg), triethylamine (20 µl) and iodoethanol (25 µl) in THF (2 ml) was refluxed for 8 h. The solvent was removed *in vacuo* and the residue was purified by chromatography (silica, dichloromethane/7M ammonia in methanol (14:1) as eluent) and the (*E*)-butenedioate salt prepared to give the title compound as a white solid (11 mg).
MS APCI +ve ^{m}/z 335 ([M+H]⁺).
¹H NMR 400MHz (DMSO-d₆) 8.60 (1H, d), 8.56 (1H, d), 7.05 (1H, d), 6.61 (1H, d), 6.50 (2H, s), 5.42 (1H, t), 3.95 (3H, s), 3.53 (2H, t), 2.86 - 2.75 (4H, m), 2.39 - 2.24 (2H, m).

### Example 18

### 2-[[(1R)-3-Amino-1-(3-chloro-5-isoxazolyl)propyl]oxy]-6-(trifluoromethyl)- 3-pyridinecarbonitrile (E)-butenedioate

### a) N-Methoxy-N-methyl-3-chloro-5-isoxazolecarboxamide

The sub-title compound was prepared from 3-chloro-5-isoxazolecarboxylic acid by the method of Example 5 step (a).
¹H NMR 300MHz (CDCl₃) 6.86 (1H, d), 3.82 (3H, s), 3.38 (3H, s).

### b) 3-Chloro-1-(3-chloro-5-isoxazolyl)-1-propanone

The sub-title compound was prepared from the product of step (a) by the method of Example 5 step (b).
¹H NMR 300MHz (CDCl₃) 6.98 (1H, d), 3.87 (2H, t), 3.33 (2H, t).

### c) (α⁵-R) α-(2-Chloroethyl)-3-chloro-5-isoxazolemethanol

The sub-title compound was prepared from the product of step (b) by the method of Example 5 step (c).
¹H NMR 300MHz (CDCl₃) 6.30 (1H, d), 5.14 (2H, t), 3.84-3.76 (1H, m), 3.72-3.63 (1H, m) 2.78 (2H, dq).

### d) 1,1-Dimethylethyl [(3R)-3-hydroxy-3-(3-chloro-5-isoxazolyl)propyl]carbamate

The sub-title compound was prepared from the product of step (c) by the method of Example 5 steps (d) and (e), without intermediate purification.
¹H NMR 300MHz (CDCl₃) 6.30 (1H, s), 4.89 - 4.78 (2H, m), 4.69 - 4.64 (1H, m), 3.63 - 3.48 (1H, m), 3.28 - 3.15 (1H, m), 2.08 - 1.82 (2H, m), 1.45 (9H, s).

### e) 1,1-Dimethylethyl [(3R)-3-[[3-cyano-6-(trifluoromethyl)-2-pyridinyl]oxy]-3-(3-chloro-5-isoxazolyl)propyl]carbamate

The sub-title compound was prepared from the product of step (d) by the method of Example 5 step (f).
¹H NMR 300MHz (CDCl₃) 8.13 (1H, d), 7.43 (1H, d), 6.42 (1H, s), 6.36 (1H, dd), 4.72 (1H, s), 3.41 - 3.27 (2H, m), 2.48 - 2.27 (2H, m), 1.39 (9H, s).

### f) 2-[[(1R)-3-Amino-1-(3-chloro-5-isoxazolyl)propyl]oxy]-6-(trifluoromethyl)-3-pyridinecarbonitrile, (E)-butenedioate

The title compound was prepared by the method of Example 5 step (g) using the product from step (e) with preparation of the (*E*)-butenedioate salt in methanol/acetonitrile.
M.p. 151-152 °C.
MS APCI +ve ^{m}/z 347/9 ([M+H]⁺).
¹H NMR 300MHz (DMSO-d₆) 8.65 (1H, d), 7.75 (1H, d), 6.97 (1H, s), 6.42 (2H, s), 6.38 - 6.32 (1H, m), 3.00 (2H, t), 2.47 - 2.29 (2H, m).

### Example 19

### 2-[[(1R)-3-Amino-1-(3-chloro-5-isoxazolyl)propyl]thio]-6-methyl-3-pyridinecarbonitrile (E)-butenedioate

### a) (α⁵-S) α-(2-Chloroethyl)-3-chloro-5-isoxazolemethanol

The sub-title compound was prepared from the product of Example 18 step (b) by the method of Example 6 step (a).
¹H NMR 300MHz (CDCl₃) 6.30 (1H, d), 5.14 (2H, t), 3.84-3.76 (1H, m), 3.72-3.63 (1H, m) 2.78 (2H, dq).

### b) 1,1-Dimethylethyl [(3S)-3-hydroxy-3-(3-chloro-5-isoxazolyl)propyl]carbamate

The sub-title compound was prepared from the product of step (a) by the method of Example 5 steps (d) and (e), without intermediate purification.
¹H NMR 300MHz (CDCl₃) 6.30 (1H, s), 4.89 - 4.78 (2H, m), 4.69 - 4.64 (1H, m), 3.63 - 3.48 (1H, m), 3.28 - 3.15 (1H, m), 2.08 - 1.82 (2H, m), 1.45 (9H, s).

### c) 1,1-Dimethylethyl [(3R)-3-(benzoylthio)-3-(3-chloro-5-isoxazolyl)propyl]carbamate

The sub-title compound was prepared from the product of step (b) by the method of Example 6 step (d).
MS APCI +ve ^{m}/z 397/9 ([M+H]⁺).

### d) 1,1-Dimethylethyl [(3R)-3-[(3-cyano-6-methyl-2-pyridinyl)thio]-3-(3-chloro-5-isoxazolyl)propyl]carbamate

The sub-title compound was prepared from the product of step (c) and 2-chloro-6-methyl-3-pyridinecarbonitrile by the method of Example 6 step (e).
MS APCI +ve ^{m}/z 408/10 ([M+H]⁺).

### e) 2-[[(1R)-3-Amino-1-(3-chloro-5-isoxazolyl)propyl]thio]-6-methyl-3-pyridinecarbonitrile (E)-butenedioate

The title compound was prepared by the method of Example 5 step (g) using the product from step (d) above with preparation of the (*E*)-butenedioate salt in methanol/acetonitrile. M.p. 186-187 °C.
MS APCI +ve ^{m}/z 309/11 ([M+H]⁺).
¹H NMR 400MHz (DMSO-d₆) 8.14 (1H, d), 7.25 (1H, d), 6.91 (1H, s), 6.39 (2H, s), 5.51 (1H, t), 2.90 - 2.81 (2H, m), 2.55 (3H, s), 2.42 - 2.25 (2H, m).

### Example 20

### 2-[3-Amino-1-(3-methyl-4-isoxazolyl)propoxy]-6-(trifluoromethyl)-3-pyridinecarbonitrile (E)-butenedioate

### a) N-Methoxy-N,3-dimethyl-4-isoxazolecarboxamide

The sub-title compound was prepared from 3-methyl-4-isoxazole carboxylic acid by the method of Example 5 step (a).
MS APCl +ve ^{m}/z 171 ([M+H]⁺).

### b) α-(2-Chloroethyl)-3-methyl-4-isoxazolemethanol

The sub-title compound was prepared from the product of step (a) by the method of Example 5 steps (b) and (c).
¹H NMR 300MHz (CDCl₃) 8.28 (1H, d), 4.95 (1H, dt), 3.79 (1H, ddd), 3.71 - 3.57 (1H, m), 2.39 (3H, s), 2.33 - 2.05 (2H, m), 2.02 (1H, d).

### c) 2-[[3-Chloro-1-(3-methyl-4-isoxazolyl)propyl]oxy]-6-(trifluoromethyl)- 3-pyridinecarbonitrile

The sub-title compound was prepared from the product of step (b) and 2-chloro-6-(trifluoromethyl)-3-pyridinecarbonitrile by the method of Example 5 step (f).
¹H NMR 300MHz (CDCl₃) 8.45 (1H, s), 8.08 (1H, d), 7.39 (1H, d), 6.39 (1H, dd), 3.80 - 3.71 (1H, m), 3.60 (1H, ddd), 2.78 - 2.65 (1H, m), 2.46 (1H, s), 2.42 - 2.29 (3H, m).

### d) 2-[[3-Iodo-1-(3-methyl-4-isoxazolyl)propyl]oxy]-6-(trifluoromethyl)-3-pyridinecarbonitrile

The sub-title compound was prepared from the product of step (c) by the method of Example 7 step (f).
¹H NMR 300MHz (CDCl₃) 8.45 (1H, s), 8.08 (1H, d), 7.39 (1H, d), 6.39 (1H, dd), 3.35 - 3.17 (2H, m), , 2.71 (1H, quintet), 2.46 (1H, s), 2.48 - 2.37 (3H, m).

### e) 2-[3-Amino-1-(3-methyl-4-isoxazolyl)propoxy]-6-(trifluoromethyl)- 3-pyridinecarbonitrile (E)-butenedioate

A solution of the product from step (d) (178 mg) and sodium azide (42 mg) in DMSO (2 ml) was stirred at 20 °C for 2 days. Triphenylphosphine (393 mg), water (1 ml) and THF (1 ml) were added and stirred for 2 days. Water was added and the mixture was extracted with ethyl acetate (three times). The organic layers were washed with water, dried (MgSO₄), evaporated and purified by passage through SCX ion exchange resin. The free base of the title product was taken up in acetonitrile/methanol, (*E*)-butenedioic acid (1 equivalent) added, the product collected and dried to yield the title product as a white solid (36 mg). -
MS APCI +ve ^{m}/z 327 ([M+H]⁺).
¹H NMR 400MHz (DMSO-d₆) 8.98 (1H, s), 8.59 (1H, d), 7.68 (1H, d), 6.36 (2H, s), 6.13 (1H, dd), 2.94 - 2.83 (2H, m), 2.45 - 2.17 (5H, m).

### Example 21

### 2-[[3-Amino-1-(5-isothiazolyl)propyl]thio]-6-methyl-3-pyridinecarbonitrile ethanedioate

### a) S-[3-[[(1,1-Dimethylethoxy)carbonyl]amino]-1-(5-isothiazolyl)propyl] benzenecarbothioate

The sub-title compound was prepared by the method of Example 6 step (d) using the product of Example 10 step (b).
¹H NMR 300MHz (CDCl₃) 8.38 (1H, d), 7.95 (1H, d), 7.93 (1H, d), 7.61 (1H, t), 7.47 (2H, t), 7.23 (1H, d), 5.25 (1H, d), 4.92 - 4.85 (1H, m), 3.45 - 3.32 (1H, m), 3.25 - 3.12 (1H, m), 2.46 - 2.18 (2H, m), 1.47 (9H, s).

### b) 1,1-Dimethylethyl [3-[(3-cyano-6-methyl-2-pyridinyl)thio]-3-(5-isothiazolyl)propyl]-carbamate

The sub-title compound was prepared from the product of step (a) and 2-chloro-6-methyl-3-pyridinecarbonitrile using the method of Example 6 step (e).
MS APCI +ve ^{m}/z 391 ([M+H]⁺).

### c) 2-[[3-Amino-1-(5-isothiazolyl)propyl]thio]-6-methyl-3-pyridinecarbonitrile, ethanedioate

The title compound was prepared by the method of Example 5 step (g) using the product from step (b) with preparation of the salt in methanol/acetonitrile. M.p. 103-104 °C.
MS APCI +ve ^{m}/z 291 ([M+H]⁺).
¹H NMR 300MHz (DMSO-d₆) 8.48 (1H, d), 8.14 (1 H, d), 7.49 (1H, d), 7.26 (1H, d), 5.63 (1H, t), 3.03 - 2.84 (2H, m), 2.62 (3H, s), 2.54 - 2.40 (2H, m).

### Example 22

### 2-[3-Amino-1-(5-isothiazolyl)propoxy]-6-methyl-3-pyridinecarbonitrile ethanedioate

### a) 1,1-Dimethylethyl [3-[(3-cyano-6-methyl-2-pyridinyl)oxy]-3-(5-isothiazolyl)propyl]-carbamate

The sub-title compound was prepared by the method of Example 1 step (a) using the product of Example 10 step (b) and 2-chloro-6-methyl-3-pyridinecarbonitrile.
¹H NMR 300MHz (CDCl₃) 8.40 (1H, d), 7.79 (1H, d), 7.27 (1H, d), 6.88 (1H, d), 6.65 (1H, dd), 5.01 (1H, s), 3.46 - 3.21 (2H, m), 2.43 - 2.25 (2H, m), 2.54 (3H, s), 1.43 (9H, s).

### b) 2-[3-Amino-1-(5-isothiazolyl)propoxy]-6-methyl-3-pyridinecarbonitrile ethanedioate

The title compound was prepared by the method of Example 5 step (g) using the product from step (a) above, with preparation of the salt in methanol/acetonitrile. M.p. 175-177 °C. MS APCI +ve ^{m}/z 275 ([M+H]⁺).
¹H NMR 300MHz (DMSO-d₆) 8.52 (1H, d), 8.19 (1H, d), 7.49 (1H, t), 7.12 (1H, d), 6.67 (1H, dd), 2.89 (2H, t), 2.46 - 2.24 (2H, m), 2.50 (3H, s).

### Example 23

### 2-[3-Amino-1-(3-isothiazolyl)propoxy]-6-methyl-3-pyridinecarbonitrile ethanedioate

### a) N-Methoxy-N-methyl-3-isothiazolecarboxamide

3-Isothiazolecarboxylic acid (1.21 g) was suspended in dichloromethane (150 ml) and 4-dimethylaminopyridine (1.14 g), *N,O*-dimethylhydroxylamine hydrochloride (912 mg), N-methylmorpholine (946 mg) and 1-(3-dimethylaminopropyl)-3-ethylcarbodiimide hydrochloride (1.79 g) added and the resultant solution stirred for 3 days at room temperature. The reaction was diluted with dichloromethane (100 ml) and washed with 2M aqueous hydrochloric acid (3 x 40 ml), saturated aqueous sodium hydrogen carbonate solution (3 x 40 ml) and water (20 ml). The organic phase was dried (MgSO₄) and evaporated *in vacuo* to give the sub-title compound (1.406 g) as a colourless oil.
¹H NMR 300MHz (CDCl₃) 8.68 (1H, d), 7.69 (1H, d), 3.81 (3H, s), 3.47 (3H, s).

### b) 1-(3-Isothiazolyl)-2-propen-1-one

The product from step (a) (1.4 g) was dissolved in anhydrous THF (50 ml) and the solution cooled to 0 °C. Vinyl magnesium chloride (1.6M in THF) (7.6 ml) was added dropwise and the reaction stirred overnight whilst slowly attaining room temperature. The reaction mixture was added dropwise to 2M aqueous hydrochloric acid (50 ml) and then extracted with ethyl acetate (3 x 60 ml). The combined organic extracts were washed with water (3 x 20 ml), dried (MgSO₄) and evaporated *in vacuo* to give the sub-title compound (950 mg) as a straw coloured oil.
¹H NMR 300MHz (CDCl₃) 8.68 (1H, d), 7.91 (1H, d), 7.63 (1H, q), 6.64 (1H, dd), 5.97 (1H, dd).

### c) a-(2-Chloroethyl)-3-isothiazolemethanol

The product from step (b) (945 mg) was dissolved in dichloromethane (80 ml) and 1M hydrochloric acid in diethyl ether (20 ml) added. The reaction was stirred at room temperature for 2 h. The solvent was removed *in vacuo* and the residue dissolved in ethanol (50 ml). Sodium borohydride (257 mg) was added in one portion and the reaction stirred at room temperature for 1 h. Water (50 ml) was added and the volume reduced on a rotary evaporator. The remaining aqueous was extracted with ethyl acetate (3 x 100 ml) and the combined extracts were washed with water (50 ml), dried (MgSO₄) and evaporated *in vacuo*. The residue was purified by chromatography (silica, hexane/ethyl acetate (1:1) as eluent) to give the sub-title compound (355 mg) as a pale coloured oil.
¹H NMR 300MHz (CDCl₃) 8.68 (1H, d), 7.23 (1H, d), 5.17-5.10 (1H, m), 3.86-3.77 (1H, m), 3.70-3.62 (1H, m), 3.03 (1H, d), 2.38-2.19 (2H, m).

### d) a-(2-Azidoethyl)-3-isothiazolemethanol

The product from step (c) (350 mg) was dissolved in DMSO (10 ml) and sodium azide (192 mg) added. The reaction was heated to 65 °C and stirred for 18 h. It was then cooled and water (50 ml) was added. The mixture was extracted with ethyl acetate (4 x 60 ml) and the combined extracts were washed with water (3 x 30 ml), dried (MgSO₄) and evaporated *in vacuo* to give the sub-title compound (325 mg) as an oil.
¹H NMR 300MHz (CDCl₃) 8.68 (1H, d), 7.23 (1H, d), 5.07-5.01 (1H, m), 3.62-3.43 (2H, m), 3.07 (1H, d), 2.22-1.98 (2H, m).

### e) 2-[3-Azido-1-(3-isothiazolyl)propoxy]-6-methyl-3-pyridinecarbonitrile

The product from step (d) (158 mg) and 2-chloro-6-methyl-3-pyridinecarbonitrile (131 mg) were dissolved in DMF (5 ml). Caesium carbonate (841 mg) was added and the reaction stirred overnight at room temperature. Water (60 ml) was added and the mixture extracted with ethyl acetate (3 x 60 ml). The combined organic extracts were washed with water (3 x 25 ml), dried (MgSO₄) and evaporated *in vacuo*. The residue was purified by chromatography (silica, hexane/ethyl acetate (4:1) as eluent) to give the sub-title compound (174 mg) as a pale yellow oil.
MS APCI +ve ^{m}/z 301 ([M+H]⁺).

### f) 2-[3-Amino-1-(3-isothiazolyl)propoxy]-6-methyl-3-pyridinecarbonitrile oxalte

The product from step (e) (172mg) was dissolved in THF (20 ml) and triphenylphosphine (240 mg) added. The mixture was stirred at room temperature for 15 minutes and then water (5 ml) was added and stirring continued for 24 h. The solvent was removed *in vacuo* and the residue purified by chromatography (silica, 5% 7N ammonia in methanol in dichloromethane as eluent). The product was dissolved in ethyl acetate and treated with one equivalent of ethanedioic acid. The precipitated ethanedioate salt was filtered off and dried under high vacuum to give the title compound (111 mg, 52%) as a white solid.
MS APCI +ve ^{m}/z 275 ([M+H]⁺).
¹H NMR 300MHz (DMSO-d₆) 9.07 (1H, d), 8.16 (1H, d), 7.38 (1H, d), 7.07 (1H, d), 6.42 (1H, t), 3.04-2.89 (2H, m), 2.40 (3H, s), 2.40-2.31 (2H, m).

### Example 24

### 2-[[(1R)-3-Amino-1-(5-methyl-3-isoxazolyl)propyl]oxy]-6-(trifluoromethyl)-3-pyridinecarbonitrile ethanedioate

### a) N-Methoxy-N,5-dimethyl-3-isoxazolecarboxamide

5-Methyl-3-isoxazolecarboxylic acid (4.3 g) was subjected to the procedure described in Example 5 step (a) to give the sub-title product as an oil (4.8 g).
¹H NMR 300MHz (CDCl₃) δ 6.33 (1H, s), 3.79 (3H, s), 3.40 (3H, bs), 2.48 (3H, s).

### b) 3-Chloro-1-(5-methyl-3-isoxazolyl)-1-propanone

The product of step (a) (4.25 g) was subjected to the procedure described in Example 5 step (b) to give the sub-title product as an oil (3.3 g).
¹H NMR 300MHz (CDCl₃) 6.40 (1H, s), 3.90 (2H, t), 3.52 (2H, t), 2.50 (3H, s).

### c) (α³-R) -(2-Chloroethyl)-5-methyl-3-isoxazolemethanol

The product of step (b) (3.3 g) was subjected to the procedure described in Example 5 step (c) to give the sub-title product as an oil (1.7 g).
¹H NMR 300MHz (CDCl₃) 6.01 (1H, s), 5.06 (1H, m) 3.84-3.64 (2H, m), 2.43 (3H, s), 2.3-2.2 (2H, m).

### d) (α³-R) α-(2-Azidoethyl)-5-methyl-3-isoxazolemethanol,

The product of step (c) (1.05 g) was subjected to the procedure described in Example 5 step (d) to give the sub-title product as an oil (1.0 g).
¹H NMR 300MHz (CDCl₃) 6.01 (1H, s), 5.0-4.94 (1H, m), 3.62-3.45 (2H, m), 2:52 (1H, d), 2.43 (3H, s), 2.09-2.02 (2H, m).

### e) 2-[[(1R)-3-Azido-1-(5-methyl-3-isoxazolyl)propyl]oxy]-6-(trifluoromethyl)-3-pyridinecarbonitrile

The product of step (d) (0.19 g) and 2-chloro-6-(trifluoromethyl)-3-pyridinecarbonitrile (0.25 g) were dissolved in DMF (5 ml) and treated with sodium hydride (0.05 g, 60% dispersion in oil) and stirred under a nitrogen atmosphere at ambient temperature for 1 h. The reaction was quenched with saturated ammonium chloride solution (25 ml) and extracted with ethyl acetate (3 x 25 ml). The combined organic extracts were washed with water, brine, dried (MgSO₄) and evaporated to dryness to give the sub-title product as an oil (0.4 g).
MS APCI +ve ^{m}/z 324 ([M-28]⁺).

### f) 2-[[(1R)-3-Amino-1-(5-methyl-3-isoxazolyl)propyl]oxy]-6-(trifluoromethyl)-3-pyridinecarbonitrile ethanedioate

The product of step (e) (400 mg) was subjected to the procedure described in Example 2 step (d) to give the sub-title product as white solid (19 mg). M.p. 163-165 °C.
MS APCI +ve ^{m}/z 327 ([M+H]⁺).
¹H NMR 400MHz (d₆-DMSO) 8.64 (1H, d), 7.73 (1H, d), 6.38 (1 H, s), 6.33-6.31 (1H, dd), 6.3 (2H, s), 2.97 (2H, m), 2.38 (3H, s), 2.35-2.27 (2H, m).

### Example 25

### 4-[[(1R)-3-Amino-1-(3-isoxazolyl)pronyl]oxy]-6-methoxy-3-pyridinecarbonitrile ethanedioate

### a) 4-[[(1R)-3-Azido-1-(3-isoxazolyl)propyl]oxy]-6-methoxy-3-pyridinecarbonitrile

The product from Example 11 step (d) (0.34 g) and the product of Example 14 step (e) (0.64 g) were dissolved in DMF (5 ml) and treated with caesium carbonate (1.0 g) and stirred under a nitrogen atmosphere at ambient temperature for 18 hr. The reaction was quenched with 2M hydrochloric acid (50 ml) and extracted with ethyl acetate (3 x 25 ml).

The combined organic extracts were washed with water, brine, dried (MgSO₄) and evaporated to dryness to give the sub-title product as an oil (0.9 g).
MS APCl +ve ^{m}/z 300 ([M]⁺).

### b) 4-[[(1R)-3-Amino-1-(3-isoxazolyl)propyl]oxy]-6-methoxy-3-pyridinecarbonitrile ethanedioate

The product from step (a) (0.9 g) was subjected to the procedure described in Example 2 step (d) to give the title product as a white solid (0.41 g). M.p. 173-174 °C.
MS APCI +ve ^{m}/z 275 ([M+H]⁺).
¹H NMR 400MHz (d₆-DMSO) 9.0 (1H, d), 8.55 (1H, s), 6.73 (1H, d), 6.58 (1H, s), 6.4 (2H, s), 6.11-6.08 (1H, dd), 3.88 (3H, s), 2.92 (2H, t), 2.41-2.22 (2H, m).

### Example 26

### 2-[[3-Amino-1-(4-methyl-3-isoxazolyl)propyl]thio]-6-methyl-3-pyridinecarbonitrile ethanedioate

### a) N-Methoxy-N,4-dimethyl-3-isoxazolecarboxamide

The sub-title compound was prepared by the method of Example 11 step (a) using 4-methyl-3-isoxazolecarboxylic acid. MS APCI +ve ^{m}/z 336 ([M-100+H]⁺).
¹H NMR 400MHz (CDCl₃) 8.22 (1H, s), 3.77 (3H, s), 3.39 (3H, s), 2.12 (3H, s).

### b) 3-Chloro-1-(4-methyl-3-isoxazolyl)-1-propanone

The sub-title compound was prepared by the method of Example 5 step (b) using the product from step (a).
¹H NMR 400MHz (CDCl₃) 8.27 (1H, s), 3.91 (2H, t), 3.55 (2H, t), 2.25 (3H, s).

### c) α-(2-Chloroethyl)-4-methyl-3-isoxazolemethanol

The sub-title compound was prepared by the method of Example 5 step (c) using the product of step (b) above and (3a*R*)-tetrahydro-1-methyl-3,3-diphenyl- 3*H*-pyrrolo[1,2-*c*][1,3,2]oxazaborole as catalyst.
¹H NMR 400MHz (CDCl₃) 8.14 (1H, s), 5.11 (1H, ddd), 3.83 (1H, ddd), 3.70 (1H, quintet), 2.42 (1H, d), 2.30 (2H, m), 2.10 (3H, s).

### d) α-(2-Azidoethyl)-4-methyl-3-isoxazolemethanol

The sub-title compound was prepared by the method of Example 2 step (a) using the product of step (c) above.
¹H NMR 400MHz (CDCl₃) 8.14 (1H, d), 5.00 (1H, dt), 3.61 (1H, dt), 3.52 (1H, dt), 2.39 (1H, d), 2.12 (1H, m), 2.10 (3H, d).

### e) 2-[[3-Amino-1-(4-methyl-3-isoxazolyl)propyl]thio]-6-methyl-3-pyridinecarbonitrile ethanedioate

The title compound was prepared by the methods of Example 2 step (b), Example 6 (e) and Example 2 step (d) using the product from step (d) above. The product was purified by reversed phase HPLC and the ethanedioate salt prepared to give the title compound (10 mg). M.p. 132-134 °C.
MS APCI +ve ^{m}/z 289 ([M+H]⁺).
¹H NMR 400MHz (CD₃OD) 8.43 (1H, s), 7.95 (1H, d), 7.20 (1H, d), 5.54 (1H, t), 3.23-3.12 (2H, m), 2.62 (4H, m), 2.51 (1H, m), 2.05 (3H, s).

### Example 27

### 2-[3-Amino-1-(2-oxazolyl)propoxy]-6-(trifluoromethyl)-3-pyridinecarbonitrile ethanedioate

### a) 3-Chloro-1-(2-oxazolyl)-1-propanone

To a solution of oxazole (2.93 g) in THF (150 ml) at -70 °C under a nitrogen atmosphere was added n-butyllithium (17 ml of a 2.5M solution in hexanes) dropwise and the solution stirred for 20 minutes. Zinc chloride (84.9 ml of a 1M solution in diethyl ether) was added and the solution warmed to 0 °C over 45 minutes. Solid cuprous iodide (8.09 g) was added and after 10 minutes, 3-chloropropionyl chloride (8.38 ml) was added. After 1 h, ethyl acetate and aqueous ammonium chloride solution were added. The organic layer was separated and washed sequentially with aqueous ammonium chloride solution, water and brine. The solution was dried (Na₂SO₄) and evaporated to yield 15.5 g of the crude product as a red oil. This mixture was used without further purification.
¹H NMR 300MHz (CDCl₃) 7.86 (1H, s), 7.36 (1H, s), 3.93 (2H, t), 3.57 (2H, m).

### b) R-α-(2-Azidoethyl)-2-oxazolemethanol

(*S*)-2-Methyl-CBS-oxazaborolidine (0.72 ml of a 1M solution in toluene) was added to THF (5 ml) under a nitrogen atmosphere and the solution cooled to -5 °C. Borane-THF complex (7.2 ml of a 1M solution in THF) was added dropwise and the solution stirred for 10 minutes. A solution of the crude product from step (a) (ca. 7.24 mmol) in THF (7 ml) was added dropwise and the reaction warmed slowly to 0 °C over 16 h. Methanol (20 ml) was cautiously added and the volatiles removed *in vacuo*. Two further methanol addition /solvent evaporation cycles were performed. The residue was purified by flash chromatography using 10-40% ethyl acetate / isohexane as eluent to give a colourless oil (724 mg). This was taken up into DMSO (5 ml), solid sodium azide (450 mg) added and the reaction heated at 65 °C for 16 h. After cooling to room temperature, water was added and the solution extracted three times with diethyl ether. The combined organic extracts were dried (Na₂SO₄) and the solvent removed *in vacuo* to yield the sub-title compound (490 mg) as an orange oil that was used without further purification.
¹H NMR 300MHz (CDCl₃) 7.65 (1H, s), 7.10 (1H, s), 4.97 (1H, dt), 3.63-3.47 (2H, m), 3.05 (1H, bs), 2.28-2.07 (2H, m).

### c) 2-[[(1R)-3-Amino-1-(2-oxazolyl)propyl]oxy]-6-(trifluoromethyl)-3-pyridinecarbonitrile ethanedioate

To a solution of the product from step (b) (160 mg) in DMF (2 ml) was added sodium hydride (76 mg of a 60% dispersion in mineral oil) and the reaction stirred for 1 h. Solid 2-chloro-6-(trifluoromethyl)-3-pyridinecarbonitrile (393 mg) was added and the reaction stirred for 2 h. Water was added and the solution extracted with diethyl ether. The organic extract was separated, dried (Na₂SO₄) and the solvent removed *in vacuo.* The residue was taken up in THF (4 ml) and triphenylphosphine (283 mg) added. After 5 minutes, water (1 ml) was added and the reaction stirred for 16 h. Further water (2 ml) was added and the reaction stirred at 55 °C for 3 h and then 48 h at room temperature. The reaction was poured into ethyl acetate / aqueous 1M sodium hydroxide. The organic extract was separated, dried (Na₂SO₄) and the solvent removed *in vacuo*. Purification by reversed phase-HPLC and ethanedioate salt formation in diethyl ether / dichloromethane (1:1) gave the title product as a hydroscopic white solid.
MS APCI +ve ^{m}/z 313 [(M+H)⁺].
¹H NMR 400MHz (d₄-MeOH) 8.42 (1H, d), 7.94 (1H, s), 7.60 (1H, d), 7.19 (1H, s), 6.37 (1H, t), 3.26 (2H, t), 2.56 (2H, m).

### Example 28

### 4 -[[3-Amino-1-(4-chloro-5-thiazolyl)propyl]thio]-6-methyl-3-pyridinecarbonitrile (E)-butenedioate

### a) 1,1-Dimethethyl [3-(2,4-dichloro-5-thiazolyl)-3-hydroxypropyl]carbamate

The sub-title compound was prepared by the method of Example 10 step (a) using 1,1-dimethylethyl 3-(oxopropyl)carbamate and 2,4-dichloro-5-thiazolyllithium instead of isothiazolelithium. Purification by chromatography (silica, 20% ethyl acetate/*iso*hexane as eluent) afforded the sub-title compound (2.05 g) as an orange oil.
MS (APCI+ve) ^{m}/z 227/229/231 [M+H(-Boc)]⁺.
¹H NMR 300MHz (CDCl₃) 4.97 (1H, d), 4.88 (1H, bd s), 4.69 (1H, bd s), 3.64-3.56 (1H, m), 3.17-3.10 (1H, m), 2.02-1.93 (1H, m), 1.76-1.70 (1H, m) and 1.46 (9H, s).

### b) 1,1-Dimethylethy [3-(4-chloro-5-thiazolyl)-3-hydroxypropyl]carbamate

To a stirred suspension of palladium on activated charcoal (75 mg) and sodium acetate trihydrate (380 mg, 1.5 eq.) in methanol (10 ml) was added a solution of [3-(2,4-dichloro-5-thiazolyl)-3-hydroxypropyl]carbamic acid 1,1-dimethethyl ester in methanol (15 ml). The mixture was subjected to an atmosphere of hydrogen (5 bar) for 72 h. The mixture was filtered and evaporated to dryness. The residue was dissolved in dichloromethane (25 ml), dried (Na₂SO₄), filtered and concentrated *in vacuo.* Purification by chromatography (silica, 20% ethyl acetate in isohexane as eluent) afforded the sub-title compound (1.15 g) as a colourless gum.
MS (APCI+ve) ^{m}/z 293/295 [M+H]⁺.
¹H NMR 300MHz (CDCl₃) 8.64 (1H, s), 5.08 (1H, d), 4.94 (1H, bd s), 4.58 (1H, bd s), 3.70-3.56 (1H, m), 3.22-3.10 (1H, m), 2.04-1.96 (1H, m), 1.86-1.74 (1H, m) and 1.46 (9H, s).

### c) S-[1-(4-Chloro-5-thiazolyl)-3-[[(1,1-dimethylethoxy)carbonyl]amino]propyl] ethanethioate

The sub-title compound was made by the method of Example 6 step (d) using 1,1-dimethylethyl [3-(4-chloro-5-thiazolyl)-3-hydroxypropyl]carbamate and thioacetic acid instead of thiobenzoic acid. Purification by chromatography (silica, 5%-10% ethyl acetate in *iso*hexane as eluent) afforded the sub-title compound (460 mg) as a colourless oil.
MS (APCI+ve) ^{m}/z 351/353 [M+H]⁺.
¹H NMR 300MHz (CDCl₃) 8.63 (1H, s), 4.88-4.80 (1H, m), 3.30-3.05 (2H, m), 2.36 (3H, s), 2.26-2.06 (2H, m) and 1.44 (9H, s).

### d) 1,1-Dimethylethyl [3-(4-chloro-5-thiazolyl)-3-[(5-cyano-2-methyl-4-pyridinyl)thio]-propyl]-carbamate

To a solution of 4-chloro-6-methyl-3-pyridinecarbonitrile (75 mg) and *S*-[1-(4-chloro-5-thiazolyl)-3-[[(1,1-dimethylethoxy)carbonyl]amino]propyl] ester ethanethioic acid (154 mg) in methanol (3 ml) was added 7*M* ammonia in methanol (3 ml). The mixture was stirred at room temperature for 18 h, concentrated *in vacuo*, dissolved in dichloromethane and pre-absorbed onto silica. Purification by chromatography (silica, 30% ethyl acetate in isohexane) afforded the sub-title compound (145 mg) as a clear gum.
MS (APCI+ve) ^{m}/z 425/427 [M+H]⁺.
¹H NMR 300MHz (CDCl₃) 8.61 (1H, s), 7.69 (1H, d), 6.96 (1H, d) 5.59 (1H, t), 5.04 (1H, bd s), 3.38-3.18 (2H, m), 2.26 (2H, q) and 1.45 (9H, s).

### e) 4-[[3-Amino-1-(4-chloro-5-thiazolyl)propyl]thio]-6-methyl-3-pyridinecarbonitrile (E)-butenedioate

The title compound was made by the method of Example 5(g) to yield the title compound (120 mg, 62%) as a pale yellow solid. M.p. 180-181 °C.
MS (APCI+ve) ^{m}/z 325/327 [M+H]⁺.
¹H NMR 300MHz (DMSO-*d*₆) 9.12 (1H, s), 8.77 (1H, s), 7.48 (1H, m), 6.45 (2H, s), 5.41 (1H, t), 2.88-2.72 (2H, m), 2.50 (3H, s) and 2.34-2.18 (2H, m).

### Example 29

### 2-[1-(4-Chloro-1,3-thiazol-5-yl)-3-(methylamino)propoxy]-6-methylnicotinonitrile ethanedioate

### a) N³-(tert-butoxycarbonyl)-N¹-methoxy-N¹,N³-dimethyl-β-alaninamide

*N*-[[(1,1-dimethylethyl)oxy]carbonyl]-β-alanine (63 g), EDCI (59.4 g), DMAP (37.89 g), NMM (34.1 g) and *N,O*-dimethylhydroxylamine hydrochloride (31 g) were stirred in dichloromethane (600 ml) for 72 h before being washed with 2M hydrochloric acid (250 ml). The aqueous was washed twice with dichloromethane (500 ml). The combined organics were washed with saturated aqueous sodium hydrogen carbonate, then brine, dried (MgSO₄), filtered and concentrated *in vacuo,* and purified by chromatography (silica, 33% ethyl acetate/*iso*hexanes as eluent) to give the sub-title compound (56 g).
¹H NMR 400MHz (d₆-DMSO) 3.66 (3H,s), 3.37(2H,t), 3.09(3H,s), 2.87 (3H, s), 2.58(2H,t), 1.39(9H,s).

### b) 1,1-Dimethylethyl 3-(2,4-dichloro-1,3-thiazol-5-yl)-3-oxopropyl(methyl)carbamate

2,4-Dichloro-thiazole (4 g) was dissolved in THF (80 ml) at -78 °C under a nitrogen atmosphere before a freshly prepared 1M solution of LDA in THF (26 ml) was added. The mixture was left for 20 minutes before the product from step (a) above (6 g) was added. The mixture was left for a further 20 minutes before water (50 ml) was added and the reaction allowed to warm to room temperature. The THF was removed by concentration *in vacuo* before the residues were extracted into ethyl acetate. The aqueous was then neutralised with concentrated hydrochloric acid and extracted again with ethyl acetate. The combined organic extracts were concentrated *in vacuo* and purified (silica, 20% diethyl ether/isohexane as eluent) to give the sub-title compund (4.65 g).

### c) 1,1-Dimethylethyl 3-(4-chloro-1,3-thiazol-5-yl)-3-hydroxypropyl(methyl)carbamate

To a suspension of Pd/C (1.6 g) in methanol (300 ml) was added the product from step (b) (5.96 g) and sodium acetate (2.55 g). The mixture was treated with hydrogen at 4 bar for 84 h. The mixture was then filtered through celite, washing with ethanol. The filtrate was then concentrated in vacuo and re-dissolved in ethanol (200 ml) before being treated with sodium borohydride (2.35 g) and stirred for 16 h. The mixture was then concentrated in *vacuo* and partitioned between ethyl acetate and water. The organics were collected, dried (MgSO₄), filtered and concentrated *in vacuo*. Purification (silica, 25% to 50% ethyl acetate/isohexane as eluent) gave the sub-title compound. (1.82 g).

### d) 1-(4-Chloro-1,3-thiazol-5-yl)-3-(methylamino)propan-1-ol

The product from step (c) was dissolved in TFA (10 ml) and dichloromethane (100 ml) and stirred for 21 h before being concentrated *in vacuo* and re-dissolved in methanol. The methanolic solution was then purified *via* SCX resin to give the sub-title compound (2.15 g).
¹H NMR 400MHz (d₆-DMSO) 9.03 (1H,s), 4.97 (2H,m), 2.58 (2H,m), 2.26 (3H, s), 1.75 (2H,m).

### e) 2-[1-(4-Chioro-1,3-thiazol-5-yl)-3-(methylamino)propoxy]-6-methylnicotinonitrile ethanedioate

The product of step (d) (200 mg) was dissolved in DMF (3 ml) and treated with sodium hydride (60% dispersion in oil, 77.5 mg) portionwise. The mixture was stirred for 20 minutes before 2-chloro-6-methylpyridine-3-carbonitrile (200 mg) was added as a solid. The mixture was stirred for 20 minutes before water (500 µl) was added and the reaction mixture loaded directly onto SCX resin. The resin was washed with methanol then the product was eluted from the resin by washing with 10% 0.88 ammonia in methanol. The methanolic ammonia was concentrated *in vacuo* and the residue purified by RPHPLC before being dissolved in diethyl ether and treated with ethanedioic acid (50% saturated in diethyl ether, 2 ml). The suspension was ultrasonicated for 2 h before the pure title compound was collected via filtration (11 mg).
MS APCI +ve ^{m}/z 323/325 ([M+H]⁺).
¹H NMR 400MHz (d₆-DMSO) 9.13 (1H ,s), 8.12 (1H ,d), 7.12 (1H ,d), 6.49 (1H, m), 3.18-3.02 (2H, m), 2.37-2.33 (2H, m), 2.62 (3H, s), 2.46 (3H, s).

### Example 30

### 2-[[1-(3-Fluorophenyl)-3-(methylamino)propyl]oxy]pyridine-3-carbonitrile ethanedioate

### a) 1,1-Dimethylethyl 3-(3-fluorophenyl)-3-oxopropyl(methyl)carbamate

1-Bromo-3-fluorobenzene (2.3 ml) was dissolved in THF (30 ml) at - 78 °C before being treated with BuLi (2.5M, 8.2 ml). The solution was left for 10 minutes before N³-(tert-butoxycarbonyl)-*N*¹-methoxy-*N*¹,*N*³-dimethyl-β-alaninamide (5 g) was added. After 10 minutes, water was added and the resultant ice slurry was allowed to warm to room temperature. The THF was removed *in vacuo* and the aqueous extracted twice with ethyl acetate. The organics were combined, dried (MgSO₄), filtered and concentrated in vacuo to give the sub-title compound (5.49 g).

### b) 1,1-Dimethylethyl 3-(3-fluorophenyl)-3-hydroxypropyl(methyl)carbamate

The product from step (a) was dissolved in ethanol (30 ml) and treated with sodium borohydride (1.52 g). The mixture was stirred for 1 h before being quenched with water. The ethanol was removed *in vacuo* and the remaining aqueous was extracted with ethyl acetate. The mixture was concentrated *in vacuo* and purified (silica, ethyl acetate as eluent) to yield the sub-title compound (2.59 g).

### c) 1-(3-Fluorophenyl)-3-(methylamino)propan-1-ol

The product from step (c) was dissolved in dichloromethane (10 ml) and treated with TFA (2 ml). The reaction was stirred for 16 h before being concentrated *in vacuo* and then under high vacuum. The residue was re-dissolved in dichloromethane (10 ml) and treated with triethylamine (3 ml). The solution was then concentrated *in vacuo* to yield the sub-title compound as a yellow oil (1.7 g).

### d) 2-[[1-(3-Fluorophenyl)-3-(methylamino)propyl]oxy]pyridine-3-carbonitrile ethanedioate

The product of step (c) (425 mg) was dissolved in DMF (4 ml) and treated with sodium hydride (60% in mineral oil, 115 mg). After 10 minutes, the solution was treated with 2-chloropyridine-3-carbonitrile (319 mg) and the reaction stirred for 1 h before being treated with water (1 ml). The mixture was stirred for 16 h before being treated with SCX resin. Impurities were removed by washing the resin with methanol before the product was collected by treating the resin with 10% 0.88 ammonia in methanol. The material collected from the resin was then purified via RPHPLC (NH₃ buffer) to give the pure product as the free base. This was then dissolved in ether before being treated with ethanedioic acid solution in ether (50% saturated). The resultant solid was washed with ether to give the title compound (114 mg).
MS APCI +ve ^{m}/z 286 ([M+H]⁺).
¹H NMR 400MHz (d₆-DMSO) 8.38 (1H, m), 8.31 (1H, d), 7.46 (1H; m), 7.25 (2H, m), 7.17 (2H, m), 6.28 (1H, m), 3.00 (2H, m), 2.57 (3H, s), 2.38-2.21 (2H, m).

### Example 31

### 6-Acetyl-2-[[3-(methylamino)-1-phenylpropyl]thio]-3-pyridinecarbonitrile (E)-butenedioate

A mixture of 6-acetyl-2-mercapto-3-pyridinecarbonitrile (242 mg), diisoproplyethylamine (2 ml) and γ-chloro-N-methylbenzenepropanamine hydrochloride (300 mg) in methanol (20 ml) was heated to 50°C for 20 h. The mixture was then concentrated to dryness and the residue dissolved in methanol (50 ml). The solution was stirred for 5 minutes with SCX resin (5 g) and the suspension filtered. The resin was washed well with methanol and the filtrate discarded. The resin was then stripped with 7M ammonia in methanol (50 ml), and the ammoniacal solution concentrated to dryness. The crude product was purified by reversed phase HPLC and the (*E*)-butenedioate salt prepared in methanol - ethyl acetate to give the title compound (25 mg).
¹H NMR 300MHz (DMSO-d₆) 8.40 (1H, d), 7.74 (1H, d), 7.55-7.28 (5H, m), 6.42 (2H, s), 5.28 (1H, t), 2.91-2.65 (5H, m), 2.5-2.3 (5H, m).
MS APCI +ve ^{m}/z 326 ([M+H]⁺).

### Example 32

### 5-Fluoro-6-methyl-2-[[(1R)-3-(methylamino)-1-phenylpropyl]thio]-3-pyridinecarbonitrile, hydrochloride

### a) S-[(1R)-3-[[(1,1-Dimethylethoxy)carbonyl]methylamino]-1-phenylpropyl] ethanethioate

The sub-title compound was prepared by the method of Example 6 step (d) using thioacetic acid and 1,1-dimethylethyl [(3*S*)-3-hydroxy-3-phenylpropyl]methylcarbamate.

### b) 1,1-Dimethylethyl [(3R)-3-mercapto-3-phenylpropyl]methylcarbamate

A solution of the product of step (a) (520 mg) and 1M sodium hydroxide solution (25 ml) in degassed ethanol (50 ml) was stirred under nitrogen for 1 h and then acidified with glacial acetic acid. The ethanol was removed *in vacuo* and the aqueous suspension was extracted with dichloromethane (2x50 ml). The organic layers were separated, dried (CaCl₂), concentrated and dried under high vacuum to give the sub-title compound as a colourless oil (455 mg).
MS APCI +ve ^{m}/z 282 ([M+H]⁺).
¹H NMR 300MHz (C₆D₆) 65 °C 7.25-7.09 (5H, m), 3.90-3.83 (1H, m), 3.27-3.17 (2H, m), 2.68 (3H, s), 2.17-2.03 (2H, m), 1.96 (1H, d), 1.54 (9H, s).

### c) 1,1-Dimethylethyl [(3R)-3-[(3-cyano-5-fluoro-6-methyl-2-pyridinyl)thio]-3-phenylpropyl]methylcarbamate

A solution of the product from step (b) (451 mg) in dry THF (10 ml) was added dropwise to a suspension of sodium hydride (70 mg, 60% dispersion in oil) at room temperature. After hydrogen evolution ceased, the solution was added to 2-chloro-5-fluoro-6-methyl-3-pyridinecarbonitrile (273 mg) and the mixture was then heated at 50 °C for 1 h. The reaction was quenched with glacial acetic acid (100 µl), evaporated and purified by chromatography (silica, 3% diethyl ether in dichloromethane as eluent) to give the sub-title compound as a colourless oil (220 mg).
MS APCI +ve ^{m}/z 316 ([M-BOC+2H]⁺).
¹H NMR 300MHz (d₆-DMSO) 65 °C 8.10 (1H, d), 7.44-7.23 (5H, m), 4.98 (1H, dd), 3.20 (2H, dd), 2.52 (3H, s), 2.24 (2H, ddd), 1.33 (9H, s).

### d) 5-Fluoro-6-methyl-2-[[(1R)-3-(methylamino)-1-phenylpropyl]thio]-3-pyridinecarbonitrile hydrochloride

The title compound was prepared by the method of Example 1 step (b), triturating with dichloromethane.
MS APCI +ve ^{m}/z 316 ([M+H]⁺).
¹H NMR 300MHz (d₆-DMSO) 8.98 (2H, br s), 8.26 (1H, d), 7.51-7.27 (5H, m), 5.11 (1H, dd), 3.05-2.62 (2H, br m), 2.57 (3H, br s), 2.51-2.37 (2H, br m).

### Example 33

### 6-Ethyl-5-fluoro-2-[[(1R)-3-(methylamino)-1-phenylpropyl]thio]-3-pyridinecarbonitrile, dihydrochloride

The title compound was prepared by the methods of Example 5 step (f) and Example 1 step (b) using the product of Example 32 step (a) and 2-chloro-5-fluoro-6-ethyl-3-pyridinecarbonitrile.
MS (APCI+) ^{m}/z 330 (M+H⁺).
¹H NMR 300MHz (D₂O) 7.83 (1H, d), 7.47 (5H, m), 5.03 (1H, t), 3.27 (1H, m), 3.08 (1H, m), 2.95 (2H, m), 2.76 (3H, s), 2.54 (2H, m), 1.32 (3H, t).

### Example 34

### 2-[[(1R)-3-[(2-Hydroxyethyl)methylamino]-1-phenylpropyl]thio]-6-(trifluoromethyl)- 3-pyridinecarbonitrile (E)-butanedioate

### a) 2-[[(1R)-3-Chloro-1-phenylpropyl]oxy]-6-(trifluoromethyl)-3-pyridinecarbonitrile

2-Chloro-6-(trifluoromethyl)-3-pyridinecarbonitrile (12.1 g) and caesium carbonate (52.1 g, 0.176 mol) were added to a solution of (*R*)-3-chloro-1-phenylpropan-1-ol (10.0 g) in DMF (600 ml) under a nitrogen atmosphere and the mixture was stirred at room temperature for 20h. The mixture was poured into water (300 ml) and extracted with diethyl ether (3 x 500 ml). The combined organic extracts were washed with brine (300 ml), dried (MgSO₄) and evaporated. Purification by chromatography (silica, isohexane:ethyl acetate 20:1 to 10:1 as eluent) gave the sub-title compound as a brown oil (17.2 g).
¹H NMR 300MHz (CDCl₃) 8.00 (d, 1H), 7.47 (d, 2H), 7.25-7.38 (m, 4H), 6.39 (dd, 1H), 3.68-3.76 (m, 1H), 3.52-3.60 (m, 1H), 2.62-2.73 (m, 1H), 2.30-2.41 (m, 1H).

### b) 2-[[(1R)-3-Iodo-1-phenylpropyl]oxy]-6-(trifluoromethyl)-3-pyridinecarbonitrile

A solution of the product from step (a) (17.2 g) in acetone (1600 ml) saturated with sodium iodide was heated under reflux for 48 h. After cooling, the mixture was poured into water (500 ml) and extracted with diethyl ether (3 x 500 ml). The combined organic extracts were washed with brine (500 ml), dried over (MgSO₄), and concentrated. Purification by chromatography (silica, isohexane:ethyl acetate 20:1 to 10:1 as eluent) gave the sub-title compound as a brown oil (17.1 g).
¹H NMR 300MHz (CDCl₃) 8.00 (d, 1H), 7.47 (d, 2H), 7.26-7.37 (m, 4H), 6.29 (dd, 1H), 3.13-3.34 (m, 2H), 2.65-2.76 (m, 1H), 2.35-2.50 (m, 1H).

### c) 2-[[(1R)-3-[(2-Hydroxyethyl)methylamino]-1-phenylpropyl]thio]-6-(trifluoromethyl)-3-pyridinecarbonitrile (E)-butanedioate

2-Methylaminoethanol (54.3 µl) was added to a solution of the product from step (b) (150 mg) in THF (2 ml). The reaction was subjected to microwave irradiation for six minutes at 140 °C and was then concentrated. After purification by chromatography (silica, dichloromethane/7M ammonia in methanol (10:1) as eluent), the (*E*)-butenedioate salt was prepared to give the title compound as a white solid (49.0 mg).
¹H NMR 300MHz (d₆-DMSO) 8.55 (d, 1H), 7.62 (d, 1H), 7.46 (d, 2H), 7.28-7.40 (m, 3H), 6.59 (s, 2H), 6.19 (t, 1H), 3.45 (t, 2H), 2.53-2.59 (m, 4H), 2.32 (m, 4H), 2.06-2.15 (m, 1H).

### Example 35

### 2-[[(1R)-3-[(2-Hydroxyethyl)propylamino]-1-phenylpropyl]oxy]-6-(trifluoromethyl) 3-pyridinecarbonitrile (E)-butanedioate

The title compound was prepared according to the method of Example 34 step (c) using 2-propylaminoethanol (119 µl) to give a white solid (56.0 mg).
¹H NMR 300MHz (d₆-DMSO) 8.55 (d, 1H), 7.62 (d, 1H), 7.45 (d, 2H), 7.27-7.40 (m, 3H), 6.60 (s, 2H), 6.23 (dd, 1H), 3.40 (t, 2H), 2.45-2.74 (m, 6H), 2.24-2.30 (m, 1H), 2.03-2.10 (m, 1H), 1.32-1.39 (m, 2H), 0.78 (t, 3H).

### Example 36

### 2-[[(1R)-3-[(3-Hydroxypropyl)amino]-1-phenylpropyl]oxy]-6-(trifluoromethyl)- 3-pyridinecarbonitrile

3-Aminopropan-1-ol (53.6 µl) was added to a solution of the product from Example 34 step (b) (150 mg) in THF (2 ml). The reaction was subjected to microwave irradiation for six minutes at 140 °C and was then concentrated. Purification by chromatography (silica, dichloromethane/7M ammonia in methanol (10:1) as eluent) gave the title compound as a white solid (21 mg).
¹H NMR 300MHz (CDCl₃) 8.00 (d, 1H), 7.46 (d, 2H), 7.24-7.37 (m, 4H), 6.25 (dd, 1H), 3.78 (t, 2H), 2.84 (m, 2H), 2.74 (t, 2H), 2.30-2.42 (m, 1H), 2.08-2.20 (m, I H), 1.67 (m, 2H).

### Example 37

### 2-[[(1R)-3-(Methylpropylamino)-1-phenylpropyl]oxy]-6-(trifluoromethyl)- 3-pyridinecarbonitrile (E)-butanedioate

The title compound was prepared according to the method of Example 34 step (c) using methylpropylamine 1 (720 µl) to give a white solid (20.0 mg).
¹H NMR 300MHz (d₆-DMSO) 8.4 (d, 1H), 7.50 (d, 1H), 7.42 (d, 2H), 7.27-7.37 (m, 3H), 6.62 (s, 2H), 6.25 (t, 1H), 2.27 (t, 4H), 2.18 (s, 3H), 2.04-2.14 (m, 2H), 1.35 (q, 2H), 0.79 (t, 3H).

### Example 38

### 2-[[(1R)-3-[[(2S)-2-Hydroxypropyl]amino]-1-phenylpropyl]oxy]-6-(trifluoromethyl)-3-pyridinecarbonitrile (E)-butanedioate

The title compound was prepared according to the method of Example 34 step (c) using (*S*)-1-aminopropan-2-ol (82.2 µl) to give a white solid (63.1 mg).
¹H NMR 300MHz (d₆-DMSO) 8.56 (d, 1H), 7.62 (d, 1H), 7.30-7.46 (m, 5H), 6.48 (s, 2H), 6.22 (dd, 1H), 3.81 (m, 1H), 2.88 (t, 2H), 2.75-2.80 (m, 1H), 2.57-2.64 (m, 1H), 2.40-2.50 (m, 1H), 2.18-2.24 (m, 1H), 1.06 (d, 3H).

### Example 39

### 2-[[(1R)-1-Phenyl-3-[(phenylmethyl)amino]propyl]oxy]-6-(trifluoromethyl)- 3-pyridinecarbonitrile (E)-butanedioate

The title compound was prepared according to the method of Example 34 step (c) using benzylamine (114 µl) as the amine, to give a white solid (55.3 mg).
¹H NMR 300MHz (d₆-DMSO) 8.54 (d, 1H), 7.63 (d, 1H), 7.22-7.44 (m, 10H), 6.53 (s, 2H), 6.27 (dd, 1H), 3.84 (s, 2H), 2.76 (t, 2H), 2.31-2.40 (m, 1H), 2.09-2.21 (m, 1H).

### Example 40

### 2-[[(1R)-3-[(1,1-Dimethylethyl)amino]-1-phenylpropyl]oxy]-6-(trifluoromethyl)-3-pyridinecarbonitrile

The title compound was prepared according to the method of Example 36 using *tert*-butylamine (110 µl) as the amine, to give a white solid (22.1 mg).
¹H NMR 300MHz (d₆-DMSO) 8.59 (d, 1H), 7.66 (d, 1H), 7.30-7.48 (m, 5H), 6.19 (dd, 1H), 3.07 (m, 2H), 2.35-2.45 (m, 1H), 2.19-2.26 (m, 1H), 1.26 (s, 9H).

### Example 41

### 2-[[(1R)-3-[[2-(4-Hydroxyphenyl)ethyl]amino]-1-phenylproyl]oxy]-6-(trifluoromethyl)-3-pyridinecarbonitrile

The title compound was prepared according to the method of Example 36 using 4-(2-aminoethyl)phenol (143 mg) as the amine, to give a white solid (35.6 mg).
¹H NMR 300MHz (d₆-DMSO) 9.18 (s, 1H), 8.57 (d, 1H), 7.64 (d, 1H), 7.30-7.45 (m, 5H), 7.00 (d, 2H), 6.67 (d, 2H), 6.20 (dd, 1H), 2.92 (m, 4H), 2.68 (m, 2H), 2.36 (m, 1H), 2.19 (m, 1H).

### Example 42

### 2-[4-Amino-1-(5-isoxazolyl)butoxy]-6-(trifluoromethyl)-3-pyridinecarbonitrile (E)-butenedioate

### a) 1-(5-Isoxazolyl)-1,4-butanediol

isoPropyl magnesium chloride (25.3 ml, 1.7M in THF) was slowly added to a solution of chloropropanol (3.6 ml) in THF (20 ml) at 0 °C. Magnesium (1.48 g) and 1,2-dibromoethane (0.1 ml) were added and the mixture was heated under reflux for 3 h. This solution was added to a solution of isoxazole-5-carboxaldehyde (2.66 g) in THF (920 ml) at 0 °C and stirred for 1 h. 2M hydrochloric acid (30 ml) was added and the mixture was extracted with ethyl acetate (6 times). The organic extracts were dried (Na₂SO₄), evaporated and purified by chromatography (silica, petrol/acetone (3:2) as eluent) to give the sub-title compound as a colourless oil (1.40 g).
MS APCI +ve ^{m}/z 158 ([M+H]⁺).

### b) 1-(5-Isoxazolyl)-1,4-butanediolyl 4-(4-methylbenzenesulfonate)

Tosyl chloride (1.95 g) was added to a solution of the product from step (a) (1.39 g) in pyridine (6 ml) and dichloromethane (5 ml) and stirred for 16 h. 2M hydrochloric acid (20 ml) was added and the mixture was extracted with dichloromethane (three times). The organic extracts were dried (MgSO₄), evaporated and purified by chromatography (silica, *iso*hexane/ether (3:2) as eluent) to give the sub-title compound as an orange gum (667 mg). MS APCI +ve ^{m}/z 312 ([M+H]⁺).

### c) 1,1-Dimethylethyl [4-hydroxy-4-(5-isoxazolyl)butyl]-carbamate

The sub-title compound was prepared from the product of step (b) by the methods of Example 5 steps (d) and (e), without intermediate purification. MS APCI +ve ^{m}/z 257 ([M+H]⁺).

### d) 1,1-Dimethylethyl [4-[[3-cyano-6-(trifluoromethyl)-2-pyridinyl]oxy]-4-(5-isoxazolyl)butyl]-carbamate

The sub-title compound was prepared from the product of step (c) by the method of Example 5 step (f).
MS APCI +ve ^{m}/z 427 ([M+H]⁺).

### e) 2-[4-Amino-1-(5-isoxazolyl)butoxy]-6-(trifluoromethyl)- 3-pyridinecarbonitrile (E)-butenedioate

The title compound was prepared by the method of Example 5 step (g) using the product from step (d) above with preparation of the (*E*)-butenedioate salt in methanol/acetonitrile. M.p. 155-157 °C.
MS APCI +ve ^{m}/z 327 ([M+H]⁺).
¹H NMR 300MHz (DMSO-d₆) 8.65 (1H, d), 8.59 (1H, d), 7.76 (1H, d), 6.60 (1H, dd), 6.42 - 6.35 (3H, m), 2.84 (2H, t), 2.29 - 2.15 (2H, m), 1.76 - 1.58 (2H, m).

### Example 43

### 2-[[4-Amino-1-(5-isoxazolyl)butyl]thio]-6-methyl-3-pyridinecarbonitrile (E)-butenedioate

### a) S-[4-[[(1,1-Dimethylethoxy)carbonyl]amino]-1-(5-isoxazolyl)butyl] benzenecarbothioate

The sub-title compound was prepared from the product of Example 42 step (c) by the method of Example 6 step (d).
MS APCI +ve ^{m}/z 377 ([M+H]⁺).

### b) 1,1-Dimethylethyl [4-[(3-cyano-6-methyl-2-pyridinyl)thio]-4-(5-isoxazolyl)butyl]-carbamate

The sub-title compound was prepared from the product of Example 14 step (e) and the product of step (a) above using the method of Example 6 step (e).
MS APCI +ve ^{m}/z 405 ([M+H]⁺).

### c) 2-[[4-Amino-1-(5-isoxazolyl)butyl]thio]-6-methyl-3-pyridinecarbonitrile (E)-butenedioate

The title compound was prepared by the method of Example 5 step (g) using the product from step (b) above with preparation of the (*E*)-butenedioate salt in methanol/acetonitrile. M.p. 155-157 °C.
MS APCI +ve ^{m}/z 305 ([M+H]⁺).
¹H NMR 300MHz (DMSO-d₆) 8.60 (1H, d), 8.54 (1H, d), 7.06 (1H, s), 6.58 (1H, d), 6.41 (2H, s), 5.37 (1H, t), 3.92 (3H, s), 2.83 (2H, t), 2.18 - 2.06 (2H, m), 1.76- 1.51 (2H, m).

### Example 44

### 4-[[4-Amino-1-(5-isoxazolyl)butyl]thio]-6-methoxy-3-pyridinecarbonitrile hydrochloride

### a) 1,1-Dimethylethyl [4-[(5-cyano-2-methoxy-4-pyridinyl)thio]-4-(5-isoxazolyl)butyl]-carbamate

The sub-title compound was prepared from the product of Example 43 step (a) and 2-chloro-6-methyl-3-pyridinecarbonitrile by the method of Example 6 step (e).
MS APCI +ve ^{m}/z 389 ([M+H]⁺).

### b) 4-[[4-Amino-1-(5-isoxazolyl)butyl]thiol-6-methoxy-3-pyridinecarbonitrile hydrochloride

The title compound was prepared from the product of step (a) by the method of Example 12 step (d) to give a white solid. M.p.136-137 °C.
MS APCl +ve ^{m}/z 289 ([M+H]⁺).
¹H NMR 300MHz (DMSO-d₆) 8.58 (1H, s), 8.15 (1H, d), 8.09 (2H, s), 7.46 (1H, s), 7.26 (1H, d), 6.57 (1H, d), 5.47 (1 H, t), 2.91 - 2.77 (2H, m), 2.58 (3H, s), 2.29 - 2.07 (2H, m), 1.83 - 1.58 (2H, m).

### Screens

The pharmacological activity of compounds according to the invention was tested in the following screens.

### Screen 1

The activity of compounds of formula (I), or a pharmaceutically acceptable salt thereof, may be screened for nitric oxide synthase inhibiting activity by a procedure based on that of Förstermann *et al*., Eur. J. Pharm., 1992, **225**, 161-165. Nitric oxide synthase converts ³H-L-arginine into ³H-L-citrulline which can be separated by cation exchange chromatography and quantified by liquid scintillation counting.

Enzyme is prepared, after induction, from the cultured murine macrophage cell line J774A-1 (obtained from the laboratories of the Imperial Cancer Research Fund). J774A-1 cells are cultured in Dulbeccos Modified Eagles Medium (DMEM) supplemented with 10% foetal bovine serum, 4 mM L-glutamine and antibiotics (100 units/ml penicillin G, 100 mg/ml streptomycin & 0.25 mg/ml amphotericin B). Cells are routinely grown in 225 cm³ flasks containing 35 ml medium kept at 37 °C and in a humidified atmosphere containing 5% CO₂.

Nitric oxide synthase is produced by cells in response to interferon-g (IFNg) and lipopolysaccharide (LPS). The medium from confluent culture flasks is removed and replaced with 25 ml (per flask) of fresh medium containing 1 mg/ml LPS and 10 units/ml IFNg. After a period of 17-20 hours in culture, harvesting of cells is accomplished by scraping the cell sheet from the flask surface into the culture medium. Cells are collected by centrifugation (1000 g for 10 minutes) and lysate prepared by adding to the cell pellet a solution containing 50 mM Tris-HCl (pH 7.5 at 20 °C), 10% (v/v) glycerol, 0.1% (v/v) Triton-X-100, 0.1 mM dithiothreitol and a cocktail of protease inhibitors comprising leupeptin (2 mg/ml), soya bean trypsin inhibitor (10 mg/ml), aprotinin (5 mg/ml) and phenylmethylsulphonyl fluoride (50 mg/ml).

For the assay, 25 µl of substrate cocktail (50 mM Tris-HCl (pH 7.5 at 20 °C), 400 µM NADPH, 20 µM flavin adenine dinucleotide, 20 µM flavin mononucleotide, 4 µM tetrahydrobiopterin, 12 µM L-arginine and 0.025 mCi L-[³H] arginine) is added to wells of a 96 well filter plate (0.45µM pore size) containing 25 µl of a solution of test compound in 50 mM Tris-HCl. The reaction is started by adding 50 µl of cell lysate (prepared as above) and after incubation for 1 hour at room temperature is terminated by addition of 50 µl of an aqueous solution of 3 mM nitroarginine and 21 mM EDTA.

Labelled L-citrulline is separated from labelled L-arginine using Dowex AG-50W. 150 µl of a 25% aqueous slurry of Dowex 50W (Na⁺ form) is added to the assay after which the whole is filtered into 96 well plates. 75 µl of filtrate is sampled and added to wells of 96 well plates containing solid scintillant. After allowing the samples to dry the L-citrulline is quantified by scintillation counting.

In a typical experiment basal activity is 300 dpm per 75 µl sample which is increased to 1900 dpm in the reagent controls. Compound activity is expressed as IC₅₀ (the concentration of drug substance which gives 50% enzyme inhibition in the assay) and aminoguanidine, which gives an IC₅₀ (50% inhibitory concentration) of 10 µM, is tested as a standard to verify the procedure. Compounds are tested at a range of concentrations and from the inhibitions obtained IC₅₀ values are calculated. Compounds that inhibit the enzyme by at least 25% at 100 µM are classed as being active and are subjected to at least one retest.

In the above screen, the compounds of Examples 1 to 44 were tested and gave IC₅₀ values of less than 10 µM indicating that they are expected to show useful therapeutic activity.

### Screen 2

Recombinant human NO synthases (iNOS, eNOS & nNOS) were expressed in *E. coli* and lysates were prepared in Hepes buffer (pH 7.4) containing co-factors (FAD, FMN, H₄B), protease inhibitors, lysozyme and the detergent, CHAPS. These preparations were used, at suitable dilution, to assess inhibition of the various isoforms. Inhibition of NOS was determined by measuring the formation of L-[³H]citrulline from L-[³H]arginine using an adaptation of the method of Förstermann *et al*.⁹ Enzyme assays were performed in the presence of 3 µM [³H]arginine, 1 mM NADPH and other co-factors required to support NOS activity (FAD, FMN, H₄B, calmodulin, Ca²⁺). Since various NOS inhibitors have been reported to exhibit slow binding kinetics, or to inactivate the enzyme in a time dependent manner, enzyme and inhibitor were pre-incubated for 60 min in the presence of NADPH before addition of arginine to initiate the reaction. Incubations continued for a further 60 min before the assays were quenched and [³H]citrulline separated from unreacted substrate by chromatography on Dowex-50W resin in a 96-well format.

In the above screen, the compounds of Examples 1 to 44 were tested and gave IC₅₀ values of less than 10 µM against the iNOS enzyme indicating that they are expected to show useful therapeutic activity.

### Screen 3

Compounds also show activity against the human form of induced nitric oxide synthase as can be demonstrated in the following assay.

The human colorectal carcinoma cell line, DLD-1 (obtained from the European Collection of Animal Cell Culture - cell line number 90102540) was routinely grown in RPMI 1640 supplemented with 10%(v/v) foetal bovine serum, and 2mM L-glutamine, at 37 °C in 5% CO₂.

Nitric oxide synthase was induced in cells by addition of medium containing human recombinant gamma-IFN (1000 units/ml), TNF-alpha (200 U/ml), IL-6 (200 U/ml) and IL-1-beta (250 U/ml). After incubation for 18 hours at 37 °C, the medium was removed and the cells washed with warm phosphate buffered saline. Cells were incubated for a further 5 hours at 37 °C / 5% CO₂ in RPMI 1640 containing 100µM L-arginine and 100µM verapamil-HCl in the presence and absence of test compounds.

Nitrite accumulation was determined by mixing an equal volume of culture media with Griess reagent (10 mg/ml sulphanilamide, 1 mg *N*-(1-naphthyl)ethylenediamine in I ml 2.5% (v/v) phosphoric acid). Inhibition in the presence of compounds was calculated relative to the nitrite levels produced by untreated cells. IC₅₀ values were estimated from a semi-log plot of % inhibition versus concentration of compound.

In this screen the compounds of Examples 1 to 44 gave IC₅₀ values of less than 100 µM, indicating that they are predicted to show useful therapeutic activity.

## Claims

1. A compound of formula (I) 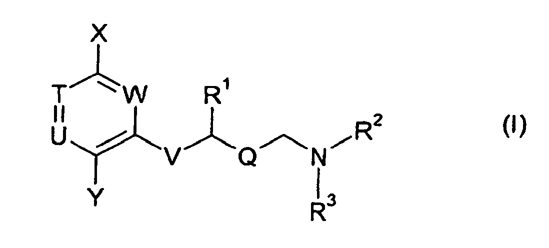 wherein:
X represents H, C1 to 4 alkyl, C1 to 4 alkoxy, halogen, OH, NHR⁹, CN, C ≡CH, NO₂, CHO, COCH₃ or NHCHO; said alkyl or alkoxy group being optionally further substituted by one or more fluorine atoms or by an OH group;
Y represents C1 to 4 alkyl, C1 to 4 alkoxy, halogen, OH, CN, C≡CH, NO₂, CHO, COCH₃ or NHCHO; said alkyl or alkoxy group being optionally further substituted by one or more fluorine atoms;
One of T, U and W represents N and the other two independently represent CR⁴; and each R⁴ group independently represents H, F or CH₃;
V represents O or S(O)ₙ;
n represents an integer 0, 1 or 2;
Q represents CH₂ or (CH₂)₂;
R¹ represents phenyl or a five or six membered aromatic heterocyclic ring containing 1 to 3 heteroatoms independently selected from O, S and N; said phenyl or aromatic heterocyclic ring being optionally substituted by one or more substituents selected independently from halogen, C1 to 4 alkyl, C1 to 4 alkoxy, OH, CN, NO₂ or NR⁵R⁶; said alkyl or alkoxy group being optionally further substituted by one or more fluorine atoms;
R² and R³ independently represent H, C1 to 4 alkyl or C3 to 6 cycloalkyl; said alkyl group being optionally substituted by C1 to 4 alkoxy, halogen, hydroxy, -Z-NR⁷R⁸, phenyl or a five or six membered aromatic or saturated heterocyclic ring containing 1 to 3 heteroatoms independently selected from O, S and N; said phenyl or aromatic heterocyclic ring being optionally further substituted by halogen, C1 to 4 alkyl, C1 to 4 alkoxy, CF₃, OCF₃, OH, CN or NO₂;
Z represents -CO- or a bond;
R⁵, R⁶, R⁷ and R⁸ independently represent H or C1 to 4 alkyl;
R⁹ represents H or C1 to 4 alkyl; said alkyl being optionally further substituted by one or more fluorine atoms;
or a pharmaceutically acceptable salt thereof.

2. A compound of formula (I), according to Claim 1, wherein V represents O.

3. A compound of formula (I), according to Claim 1, wherein V represents S(O)ₙ and n represents 0.

4. A compound of formula (I), according to any one of Claims I to 3, wherein X and Y independently represent Br, Cl, CH₃, CH₂F, CHF₂, CF₃, CH₃CH₂, NH₂, OCH₃, COCH₃ or CN.

5. A compound according to Claim 4 wherein Y represents CN.

6. A compound of formula (I), according to Claim 1, which is:
2-[[(1*R*)-3-(methylamino)-1-phenylpropyl]oxy]-6-(trifluoromethyl)-3-pyridinecarbonitrile;
2-[[(1*R*)-3-amino-1-phenylpropyl]thio]-6-methyl-3-pyridinecarbonitrile;
2-[[(1*R*)-3-amino-1-phenylpropyl]thio]-6-(fluoromethyl)-3-pyridinecarbonitrile;
2-[[(1*R*)-3-amino-1-phenylpropyl]thio]-6-(difluoromethyl)-3-pyridinecarbonitrile;
2-[[(1*R*)-3-amino-1-(5-isoxazolyl)propyl]oxy]-6-(trifluoromethyl)- 3-pyridinecarbonitrile;
2-[[(1*R*)-3-amino-1-(5-isoxazolyl)propyl]thio]-6-methyl-3-pyridinecarbonitrile;
2-[[(1R)-3-amino-1-(3-thienyl)propyl]oxy]-6-(trifluoromethyl)-3-pyridinecarbonitrile;
2-[[(1R)-3-[(3-hydroxypropyl)amino]-1-(3-thienyl)propyl]oxy]-6-(trifluoromethyl)-3-pyridinecarbonitrile;
3-[[(3R)-3-[[3-cyano-6-(trifluoromethyl)-2-pyridinyl]oxy]-3-(3-thienyl)propyl]amino]-*N-*methyl propanamide;
2-[[3-amino-1-(5-isothiazolyl)propyl]thio]-6-(trifluoromethyl)-3-pyridinecarbonitrile;
2-[[(1*R*)-3-amino-1-(3-isoxazolyl)propyl]oxy]-6-(trifluoromethyl)-3-pyridinecarbonitrile;
2-[[(1*R*)-3-amino-1-(2-thiazolyl)propyl]oxy]-6-(trifluoromethyl)-3-pyridinecarbonitrile;
(R)-2-(3-dimethylamino-1-isoxazol-5-yl-propoxy)-6-trifluoromethyl-nicotinonitrile;
6-amino-4-[[(1*R*)-3-amino-1-(5-isoxazolyl)propyl]thio]-3-pyridinecarbonitrile;
γ-[(5-chloro-2-methoxy-4-pyridinyl)thio]-(γ⁵*R*)-5-isoxazolepropanamine, 4-[[(1*R*)-3-amino-1-(5-isoxazolyl)propyl]thio]-6-methoxy-3-pyridinecarbonitrile;
4-[[(1*R*)-3-[(2-hydroxyethyl)amino]-1-(5-isoxazolyl)propyl]thio]-6-methoxy-3-pyridinecarbonitrile;
2-[[(1*R*)-3-amino-1-(3-chloro-5-isoxazolyl)propyl]oxy]-6-(trifluoromethyl)- 3-pyridinecarbonitrile;
2-[[(1*R*)-3-amino-1-(3-chloro-5-isoxazolyl)propyl]thio]-6-methyl-3-pyridinecarbonitrile;
2-[3-amino-1-(3-methyl-4-isoxazolyl)propoxy]-6-(trifluoromethyl)- 3-pyridinecarbonitrile;
2-[[3-amino-1-(5-isothiazolyl)propyl]thio]-6-methyl-3-pyridinecarbonitrile;
2-[3-amino-1-(5-isothiazolyl)propoxy]-6-methyl-3-pyridinecarbonitrile;
2-[3-amino-1-(3-isothiazolyl)propoxy]-6-methyl-3-pyridinecarbonitrile;
2-[[(1R)-3-amino-1-(5-methyl-3-isoxazolyl)propyl]oxy]-6-(trifluoromethyl)-3-pyridinecarbonitrile;
4-[[(1*R*)-3-amino-1-(3-isoxazolyl)propyl]oxy]-6-methoxy-3-pyridinecarbonitrile;
2-[[3-amino-1-(4-methyl-3-isoxazolyl)propyl]thio]-6-methyl-3-pyridinecarbonitrile;
2-[3-amino-1-(2-oxazolyl)propoxy]-6-(trifluoromethyl)-3-pyridinecarbonitrile;
4 -[[3-amino-1-(4-chloro-5-thiazolyl)propyl]thlo]-6-methyl-3-pyridinecarbonitrile;
2-[1-(4-chloro-1,3-thiazol-5-yl)-3-(methylamino)propoxy]-6-methylnicotinonitrile;
2-[[1-(3-fluorophenyl)-3-(methylamino)propyl]oxy]pyridine-3-carbonitrile;
6-acetyl-2-[[3-(methylamino)-1-phenylpropyl]thio]-3-pyridinecarbonitrile;
5-fluoro-6-methyl-2-[[(1*R*)-3-(methylamino)-1-phenylpropyl]thio]-3-pyridinecarbonitrile;
6-ethyl-5-fluoro-2-[[(1*R*)-3-(methylamino)-1-phenylpropyl]thio]-3-pyridinecarbonitrile;
2-[[(1*R*)-3-[(2-hydroxyethyl)methylamino]-1-phenylpropyl]thio]-6-(trifluoromethyl)- 3-pyridinecarbonitrile;
2-[[(1*R*)-3-[(2-hydroxyethyl)propylamino]-1-phenylpropyl]oxy]-6-(trifluoromethyl) 3-pyridinecarbonitrile;
2-[[(1*R*)-3-[(3-hydroxypropyl)amino]-1-phenylpropyl]oxy]-6-(trifluoromethyl)- 3-pyridinecarbonitrile;
2-[[(1*R*)-3-(methylpropylamino)-1-phenylpropyl]oxy]-6-(trifluoromethyl)- 3-pyridinecarbonitrile;
2-[[(1*R*)-3-[[(2*S*)-2-hydroxypropyl]amino]-1-phenylpropyl]oxy]-6-(trifluoromethyl)-3-pyridinecarbonitrile;
2-[[(1*R*)-1-phenyl-3-[(phenylmethyl)amino]propyl]oxy]-6-(trifluoromethyl)- 3-pyridinecarbonitrile;
2-[[(1*R*)-3-[(1,1-dimethylethyl)amino]-1-phenylpropyl]oxy]-6-(trifluoromethyl)- 3-pyridinecarbonitrile;
2-[[(1*R*)-3-[[2-(4-hydroxyphenyl)ethyl]amino]-1-phenylpropyl]oxy]-6-(trifluommethyl)-3-pyridinecarbonitrile;
2-[4-amino-1-(5-isoxazolyl)butoxy]-6-(trifluoromethyl)- 3-pyridinecarbonitrile;
2-[[4-amino-1-(5-isoxazolyl)butyl]thio]-6-methyl-3-pyridinecarbonitrile;
4-[[4-amino-1-(5-isoxazolyl)butyl]thio]-6-methoxy-3-pyridinecarbonitrile;
or a pharmaceutically acceptable salt, enantiomer or racemate thereof.

7. A compound of formula (I), according to any one of Claims I to 6, or a pharmaceutically acceptable salt thereof, for use as a medicament.

8. A pharmaceutical composition comprising a compound of formula (I) according to any one of Claims 1 to 6, or a pharmaceutically acceptable salt thereof, in admixture with a pharmaceutically acceptable adjuvant, diluent or carrier.

9. The use of a compound of formula (I) according to any one of Claims 1 to 6, or a pharmaceutically acceptable salt thereof, in the manufacture of a medicament for the treatment or prophylaxis of inflammatory disease.

10. The use as claimed in Claim 9 wherein the disease is inflammatory bowel disease.

11. The use as claimed in Claim 9 wherein the disease is rheumatoid arthritis.

12. The use as claimed in Claim 9 wherein the disease is osteoarthritis.

13. The use of a compound of formula (I) as defined in any one of Claims 1 to 6, or a pharmaceutically acceptable salt thereof, in the manufacture of a medicament, for the treatment or prophylaxis of pain.

14. The use of a compound of formula (I) as defined in any one of Claims 1 to 6, or a pharmaceutically acceptable salt thereof, in combination with a COX-2 inhibitor, in the manufacture of a medicament, for the treatment or prophylaxis of inflammatory diseases.

15. A process for the preparation of a compound of formula (I), as defined in any one of Claims 1 to 6, or a pharmaceutically acceptable salt, enantiomer or racemate thereof, or a tautomer thereof, wherein the process comprises:
(a) reaction of a compound of formula (II) 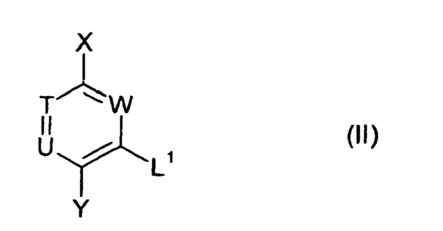 wherein T, U, X, Y and W are as defined in Claim 1 and L¹ represents a leaving group, with a compound of formula (III) 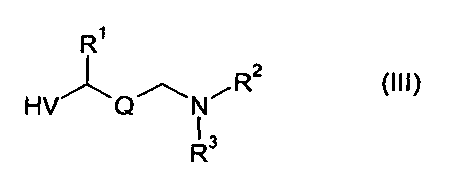 wherein R¹, R², R³, Q and V are as defined in Claim 1; or
(b) reaction of a compound of formula (IV) 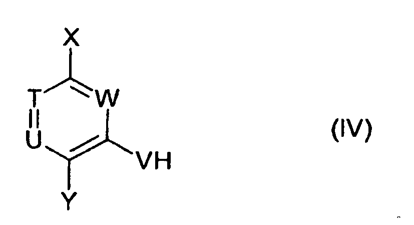 wherein T, U, W, X, Y and V are as defined in Claim 1, with a compound of formula (V) 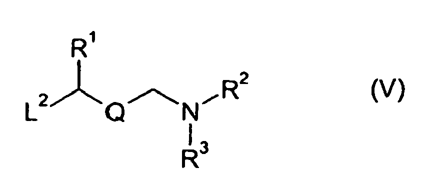 wherein R¹, R², R³ and Q are as defined in Claim I and L² is a leaving group; or
(c) reaction of a compound of formula (VI) 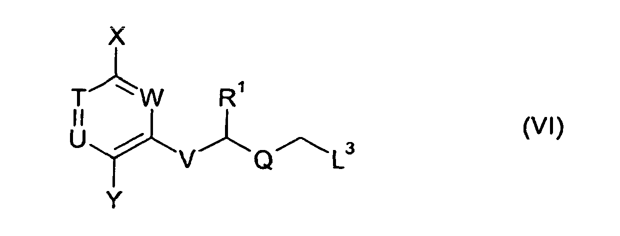 wherein R¹, Q, T, U, W, X, Y and V are as defined in Claim 1 and L³ is a leaving group, with a compound of formula (VII)
R²R³NH (VII)
wherein R² and R³ are as defined in Claim 1; or
(d) reduction of a compound of formula (VIII) 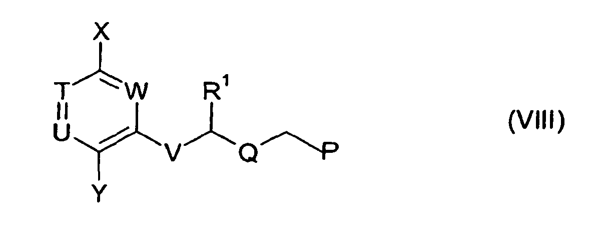 wherein R¹, Q, T, U, W, X, Y and V are as defined in Claim 1 and P represents azide (N₃); or
(e) hydrolysis of a compound of formula (VIII) 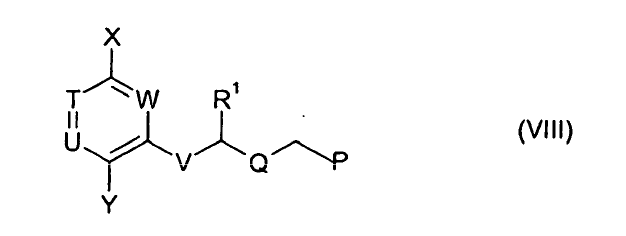 wherein R¹, Q, T, U, W, X, Y and V are as defined in Claim 1 and P represents an imide group;
and where desired or necessary converting the resultant compound of formula (I), or another salt thereof, into a pharmaceutically acceptable salt thereof; or converting one compound of formula (I) into another compound of formula (I); and where desired converting the resultant compound of formula (I) into an optical isomer thereof.

## Patentansprüche

1. Verbindungen der Formel (I), 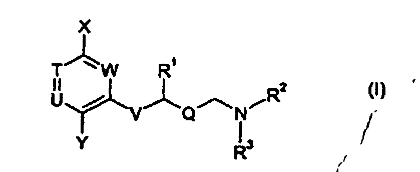 in der
X H, C1- bis C4-Alkyl, C1- bis C4-Alkoxy, Halogen, OH, NHR⁹, CN, C ≡ CH, NO₂, CHO, COCH₃ oder NHCHO bedeutet, wobei die Alkyl- oder Alkoxygruppe gegebenenfalls weiter durch ein oder mehrere Fluoratome oder durch eine OH-Gruppe substituiert ist;
Y C1- bis C4-Alkyl, C1- bis C4-Alkoxy, Halogen, OH, CN, C ≡ CH, NO₂, CHO, COCH₃ oder NHCHO bedeutet, wobei die Alkyl- oder Alkoxygruppe gegebenenfalls weiter durch ein oder mehrere Fluoratome substituiert ist;
einer der Substituenten T, U und W N bedeutet und die beiden anderen unabhängig CR⁴ bedeuten; und jede R⁴-Gruppe unabhängig H, F oder CH₃ bedeutet;
V O oder S(O)ₙ bedeutet;
n eine ganze Zahl 0, 1 oder 2 bedeutet;
Q CH2 oder (CH₂)₂ bedeutet;
R¹ einen fünf- oder sechsgliedrigen aromatischen heterocyclischen Ring bedeutet, der 1 bis 3 Heteroatome, die unabhängig aus der Reihe O, S und N gewählt werden, enthält; wobei der Phenylring oder aromatische heterocyclische Ring gegebenenfalls durch einen oder mehrere Substituenten, die unabhängig aus der Reihe Halogen, C1- bis C4-Alkyl, C1- bis C4-Alkoxy, OH, CN, NO₂ oder NR⁵R⁶ gewählt sind, substituiert ist; wobei die Alkyl- oder Alkoxygruppe gegebenenfalls weiter durch ein oder mehrere Fluoratome substituiert ist;
R² und R³ bedeuten unabhängig H, C1- bis C4-Alkyl oder C3- bis C6-Cycloalkyl; wobei die Alkylgruppe gegebenenfalls durch C1- bis C4-Alkoxy, Halogen, Hydroxy, -Z-NR⁷R⁸, Phenyl oder einen fünf- oder sechsgliedrigen aromatischen oder gesättigten heterocyclischen Ring, der 1 bis 3 Heteroatome, die unabhängig aus der Reihe O, S und N gewählt sind, enthält, substituiert ist; wobei der Phenylring oder aromatische heterocyclische Ring gegebenenfalls weiter durch Halogen, C1- bis C4-Alkyl, C1- bis C4-Alkoxy, CF₃, OCF₃, OH, CN oder NO₂ substituiert ist;
Z -CO- oder eine Bindung bedeutet;
R⁵ R⁶, R⁷ und R⁸ unabhängig H oder C1- bis C4-Alkyl bedeuten;
R⁹ H oder C1- bis C4-Alkyl bedeutet; wobei das Alkyl gegebenenfalls weiter durch ein oder mehrere Fluoratome substituiert ist;
oder eines ihrer pharmazeutisch annehmbaren Salze.

2. Verbindungen der Formel (I) nach Anspruch 1, in der V O bedeutet.

3. Verbindungen der Formel (I) nach Anspruch 1, in der V S(O)ₙ bedeutet und n 0 bedeutet.

4. Verbindung der Formel (I) nach einem der Ansprüche 1 bis 3, in der X und Y unabhängig Br, Cl, CH₃, CH₂F, CHF₂, CF₃, CH₃CH₂, NH₂, OCH₃, COCH₃ oder CN bedeuten.

5. Verbindung nach Anspruch 4, in der Y CN bedeutet.

6. Verbindung der Formel (I) nach Anspruch 1, bei der es sich um
2-[[(1*R*)-3-(Methylamino)-1-phenylpropyl]oxy]-6-(trifluormethyl)-3-pyridincarbonsäurenitril;
2-[[(1*R*)-3-Amino-1-phenylpropyl]thio]-6-methyl-3-pyridincarbonsäurenitril;
2-[[(1*R*)-3-Amino-1-phenylpropyl]thio]-6-(fluormethyl)-3-pyridincarbonsäurenitril;
2-[[(1*R*)-3-Amino-1-phenylpropyl]thio]-6-(difluormethyl)-3-pyridincarbonsäurenitril;
2-[[(1*R*)-3-Amino-1-(5-isoxazolyl)propyl]oxy]-6-(trifluormethyl)-3-pyridincarbonsäurenitril;
2-[[(1*R*)-3-Amino-1-(5-isoxazolyl)propyl]thio]-6-methyl-3-pyridincarbonsäurenitril;
2-[[(1*R*)-3-Amino-1-(3-thienyl)propyl]oxy]-6-(trifluormethyl)-3-pyridincarbonsäurenitril;
2-[[(1*R*)-3-[(3-Hydroxypropyl)amino-1-(3-thienyl) propyl]oxy]-6-(trifluormethyl)-3-pyridincarbonsäurenitril;
3-[[(3*R*)-3-[[3-Cyan-6-(trifluormethyl)-2-pyridinyl]oxy]-3-(3-thienyl)propyl]amino]-*N*-methylpropanamid;
2-[[3-Amino-1-(5-isoxazolyl)propyl]thio]-6-(trifluormethyl)-3-pyridincarbonsäurenitril;
2-[[(1*R*)-3-Amino-1-(3-isoxazolyl)propyl]oxy]-6-(trifluormethyl)-3-pyridincarbonsäurenitril;
2-[[(1*R*)-3-Amino-1-(2-thiazolyl)propyl]oxy]-6-(trifluormethyl)-3-pyridincarbonsäurenitril;
(R)-2-(3-Dimethylamino-1-isoxazol-5-yl-propoxy)-6-trifluormethyl-3-nicotinsäurenitril;
6-Amino-4-[[(1*R*)-3-amino-1-(5-isoxazolyl)propyl] thio]-3-pyridincarbonsäurenitril;
γ-[(5-Chlor-2-metoxy-4-pyridinyl)thio]-(γ⁵*R*)-5-isoxazolpropanamin,
4-[[(1*R*)-3-Amino-1-(5-isoxazolyl)propyl]thio]-6-methoxy-3-pyridincarbonsäurenitril;
4-[[(1*R*)-3-[(2-Hydroxyethyl)amino]-1-(5-isoxazolyl)propyl]thio]-6-methoxy-3-pyridincarbonsäurenitril;
2-[[(1*R*)-3-Amino-1-(3-chlor-5-isoxazolyl)propyl]-oxy]-6-(trifluormethyl)-3-pyridincarbonsäurenitril;
2-[[(1*R*)-3-Amino-1-(3-chlor-5-isoxazolyl)propyl] thio]-6-methyl-3-pyridincarbonsäurenitril;
2-[3-Amino-1-(3-methyl-4-isoxazolyl)propyl]-6-(trifluormethyl)-3-pyridincarbonsäurenitril;
2-[[3-Amino-1-(5-isothiazolyl)propyl]thio]-6-methyl-3-pyridincarbonsäurenitril;
2-[3-Amino-1-(5-isothiazolyl)propoxy]-6-methyl-3-pyridincarbonsäurenitril;
2-[3-Amino-1-(3-isothiazolyl)propoxy]-6-methyl-3-pyridincarbonsäurenitril;
2-[[(1*R*)-3-Amino-1-(5-methyl-3-isoxazolyl)propyl]-oxy]-6-(trifluormethyl)-3-pyridincarbonsäurenitril;
4-[[(1R)-3-Amino-1-(3-isoxazolyl)propyl]oxy]-6-methoxy-3-pyridincarbonsäurenitril;
2-[[3-Amino-1-(4-methyl-3-isoxazolyl)propyl] thio]-6-methyl-3-pyridincarbonsäurenitril;
2-[3-Amino-1-(2-oxazolyl)propoxy]-6-(trifluormethyl)-3-pyridincarbonsäurenitril;
4-[[3-Amino-1-(4-chlor-5-thiazolyl)propyl]thio]-6-methyl-3-pyridincarbonsäurenitril;
2-[1-(4-Chlor-1,3-thiazol-5-yl)-3-(methylamino) propoxy]-6-methylnicotinsäurenitril;
2-[[1-(3-Fluorphenyl)-3-(methylamino)propyl]oxy] pyridin-3-carbonsäurenitril;
6-Acetyl-2-[[3-(methylamino)-1-phenylpropyl]thio]-3-pyridincarbonsäurenitril;
5-Fluor-6-methyl-2-[[(1*R*)-3-(methylamino)-1-phenylpropyl]thio]-3-pyridincarbonsäurenitril;
6-Ethyl-5-fluor-2-[[(1*R*)-3-(methylamino)-1-phenylpropyl]thio]-3-pyridincarbonsäurenitril;
2-[[(1*R*)-3-[(2-Hydroxyethyl)methylamino]-1-phenylpropyl]thio]-6-(trifluormethyl)-3-pyridincarbonsäurenitril;
2-[[(1R)-3-[(2-Hydroxyethyl)propylamino]-1-phenylpropyl]oxy]-6-(trifluormethyl)-3-pyridincarbonsäurenitril;
2-[[(1*R*)-3-[(3-Hydroxypropyl)amino]-1-phenylpropyl]oxy]-6-(trifluormethyl)-3-pyridincarbonsäurenitril;
2-[[(1*R*)-3-(Methylpropylamino]-1-phenylpropyl]oxy]-6-(trifluormethyl)-3-pyridincarbonsäurenitril;
2-[[(1*R*)-3-[[(2*S*)-2-Hydroxypropyl]amino]-1-phenylpropyl]oxy]-6-(trifluormethyl)-3-pyridincarbonsäurenitril;
2-[[(1R)-1-Phenyl-3-[(phenylmethyl)amino]propyl] oxy]-6-(trifluormethyl)-3-pyridincarbonsäurenitril;
2-[[(1*R*)-3-[(1,1-Dimethyl)amino]-1-phenylpropyl]-oxy]-6-(trifluormethyl)-3-pyridincarbonsäurenitril;
2-[[(1*R*)-3-[[2-(4-Hydroxyphenyl)ethyl]amino]-1-phenylpropyl]oxy]-6-(trifluormethyl)-3-pyridincarbonsäurenitril;
2-[4-Amino-1-(5-isoxazolyl)butoxy]-6-(trifluormethyl)-3-pyridincarbonsäurenitril;
2-[[4-Amino-1-(5-isoxazolyl)butyl]thio]-6-methyl-3-pyridincarbonsäurenitril;
4-[[4-Amino-1-(5-isoxazolyl)butyl]thio]-6-methoxy-3-pyridincarbonsäurenitril;
oder eines ihrer pharmazeutisch annehmbaren Salze, Enantiomere oder Racemate handelt.

7. Verbindung der Formel (I) nach einem der Ansprüche 1 bis 6 oder eines ihrer pharmazeutisch annehmbaren Salze zur Verwendung als Arzneimittel.

8. Pharmazeutische Zusammensetzung, umfassend eine Verbindung der Formel (I) nach einem der Ansprüche 1 bis 6 oder eines ihrer pharmazeutisch annehmbaren Salze in Abmischung mit einem pharmazeutisch annehmbaren Hilfsstoff, Verdünnungsmittel oder Träger.

9. Verwendung einer Verbindung der Formel (I) nach einem der Ansprüche 1 bis 6 oder eines ihrer pharmazeutisch annehmbaren Salze bei der Herstellung eines Arzneimittels für die Behandlung oder Prophylaxe von entzündlichen Erkrankungen.

10. Verwendung nach Anspruch 9, wobei es sich bei der Krankheit um Reizdarm handelt.

11. Verwendung nach Anspruch 9, wobei es sich bei der Krankheit um rheumatoide Arthritis handelt.

12. Verwendung nach Anspruch 9, wobei es sich bei der Krankheit um Osteoarthritis handelt.

13. Verwendung einer Verbindung der Formel (I) nach einem der Ansprüche 1 bis 6 oder eines ihrer pharmazeutisch annehmbaren Salze bei der Herstellung eines Arzneimittels für die Behandlung oder Prophylaxe von Schmerzen.

14. Verwendung einer Verbindung der Formel (I) nach einem der Ansprüche 1 bis 6 oder eines ihrer pharmazeutisch annehmbaren Salze in Kombination mit einem COX-2-Hemmer bei der Herstellung eines Arzneimittels zur Behandlung oder Prophylaxe von entzündlichen Erkrankungen.

15. Verfahren zur Herstellung einer Verbindung der Formel (I) nach einem der Ansprüche 1 bis 6 oder eines ihrer pharmazeutisch annehmbaren Salze, Enantiomere oder Racemate, oder eines ihrer Tautomere, **dadurch gekennzeichnet, daß** man
(a) eine Verbindung der Formel (II), 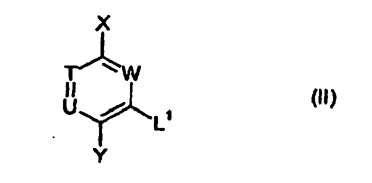 in der T, U, X, Y und W wie in Anspruch 1 definiert sind und L¹ eine Abgangsgruppe bedeutet, mit einer Verbindung der Formel (III), 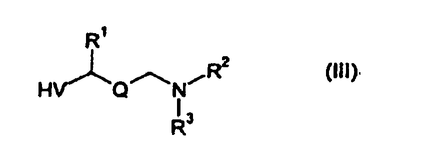 in der R¹, R², R³, Q und V wie in Anspruch 1 definiert sind, umsetzt; oder
(b) eine Verbindung der Formel (IV), 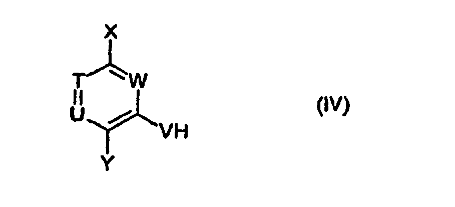 in der T, U, W, X, Y und V wie in Anspruch 1 definiert sind,
mit einer Verbindung der Formel (V), 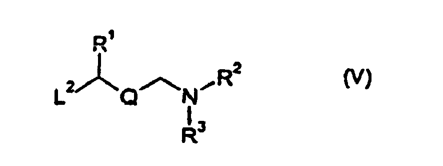 in der R¹, R², R³und Q wie in Anspruch 1 definiert sind und L² eine Abgangsgruppe bedeutet, umsetzt; oder
(c) eine Verbindung der Formel (VI), 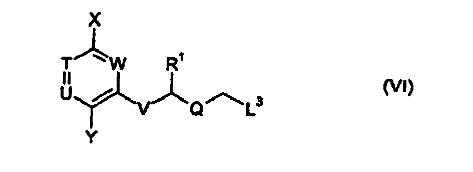 in der R¹, Q, T, U, W, X, Y und V wie in Anspruch 1 definiert sind und L³ eine Abgangsgruppe bedeutet, mit einer Verbindung der Formel (VII),
R²R³NH (VII)
in der R² und R³, wie in Anspruch 1 definiert sind, umsetzt; oder
(d) eine Verbindung der Formel (VIII), 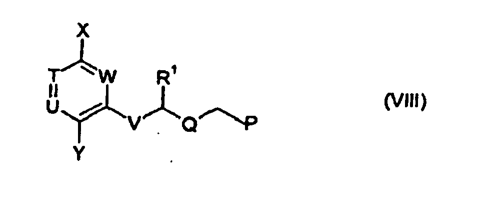 in der R¹, Q, T, U, W, X, Y und V wie in Anspruch 1 definiert sind und P Azid (N₃) bedeutet, reduziert; oder
(e) eine Verbindung der Formel (VIII), 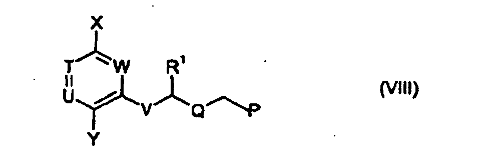 in der R¹, Q, T, U, W, X, Y und V wie in Anspruch 1 definiert sind und P eine Imidgruppe bedeutet, hydrolysiert;
und erforderlichenfalls die erhaltene Verbindung der Formel (I) oder ein anderes ihrer Salze in eines ihrer pharmazeutisch annehmbaren Salze umwandelt; oder eine Verbindung der Formel (I) in eine andere Verbindung der Formel (I) umwandelt; und, falls erwünscht, die erhaltene Verbindung der Formel (I) in eines ihrer optischen Isomere umwandelt.

## Revendications

1. Composé de formule (I) 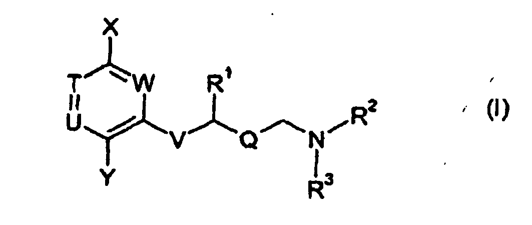 dans laquelle :
X représente H, alkyle en C₁₋₄, alcoxy en C₁₋₄, halogène, OH, NHR⁹, CN, C≡CH, NO₂, CHO, COCH₃ ou NHCHO ; ledit groupement alkyle ou alcoxy étant éventuellement substitué en outre par un ou plusieurs atomes de fluor ou par un groupement OH ;
Y représente alkyle en C₁₋₄, alcoxy en C₁₋₄, halogène, OH, CN, C≡CH, NO₂, CHO, COCH₃ ou NHCHO ; ledit groupement alkyle ou alcoxy étant éventuellement substitué en outre par un ou plusieurs atomes de fluor ;
l'un parmi T, U et W représente N et les deux autres représentent indépendamment CR⁴; et chaque groupement R⁴ représente indépendamment H, F ou CH₃ ;
V représente O ou S(O)ₙ ;
n représente un nombre entier 0, 1 ou 2 ;
Q représente CH₂ ou (CH₂)₂ ;
R¹ représente phényle ou un cycle hétérocyclique aromatique de cinq ou six chaînons contenant de 1 à 3 hétéroatomes choisis indépendamment parmi O, S et N ; ledit phényle ou cycle hétérocyclique aromatique étant éventuellement substitué par un ou plusieurs substituants choisis indépendamment parmi halogène, alkyle en C₁₋₄, alcoxy en C₁₋₄, OH, CN, NO₂ ou NR⁵R⁶ ; ledit groupement alkyle ou alcoxy étant éventuellement substitué en outre par un ou plusieurs atomes de fluor ;
R² et R³ représentent indépendamment H, alkyle en C₁₋₄ ou cycloalkyle en C₃₋₆ ; ledit groupement alkyle étant éventuellement substitué par alcoxy en C₁₋₄, halogène, hydroxy, -Z-NR⁷R⁸, phényle ou un cycle hétérocyclique saturé ou aromatique de cinq ou six chaînons contenant de 1 à 3 hétéroatomes choisis indépendamment parmi O, S et N ; ledit phényle ou cycle hétérocyclique aromatique étant éventuellement substitué en outre par halogène, alkyle en C₁₋₄, alcoxy en C₁₋₄, CF₃, OCF₃, OH, CN ou NO₂ ;
Z représente -CO- ou une liaison ;
R⁵, R⁶, R⁷ et R⁸ représentent indépendamment H ou alkyle en C₁₋₄ ;
R⁹ représente H ou alkyle en C₁₋₄ ; ledit alkyle étant éventuellement substitué en outre par un ou plusieurs atomes de fluor ;
ou un sel pharmaceutiquement acceptable de celui-ci.

2. Composé de formule (I) selon la revendication 1, dans laquelle V représente O.

3. Composé de formule (I) selon la revendication 1, dans laquelle V représente S(O)ₙ et n représente 0.

4. Composé de formule (I) selon l'une quelconque des revendications 1 à 3, dans laquelle X et Y représentent indépendamment Br, Cl, CH₃, CH₂F, CHF₂, CF₃, CH₃CH₂, NH₂, OCH₃, COCH₃ ou CN.

5. Composé selon la revendication 4, dans lequel Y représente CN.

6. Composé de formule 5I) selon la revendication 1, qui est :
le 2-[[(1*R*)-3-(méthylamino)-1-phénylpropyl]oxy]-6-(trifluorométhyl)-3-pyridinecarbonitrile ;
le 2-[[(1*R*)-3-amino-1-phénylpropyl]thio]-6-méthyl-3-pyridinecarbonitrile ;
le 2-[[(1*R*)-3-amino-1-phénylpropyl]thio]-6-(fluorométhyl)-3-pyridinecarbonitrile ;
le 2-[[(1*R*)-3-amino-1-phénylpropyl]thio]-6-(difluorométhyl)-3-pyridinecarbonitrile ;
le 2-[[(1*R*)-3-amino-1-(5-isoxazolyl)propyl]oxy]-6-(trifluorométhyl)-3-pyridinecarbonitrile ;
le 2-[[(1*R*)-3-amino-1-(5-isoxazolyl)propyl]thio]-6-méthyl-3-pyridinecarbonitrile ;
le 2-[[(1*R*)-3-amino-1-(3-thiényl)propyl]oxy]-6-(trifluorométhyl)-3-pyridinecarbonitrile ;
le 2-[[(1*R*)-3-[(3-hydroxypropyl)amino]-1-(3-thiényl)-propyl]oxy]-6-(trifluorométhyl)-3-pyridinecarbonitrile ;
le 3-[[(3*R*)-3-[[3-cyano-6-(trifluorométhyl)-2-pyridinyl]oxy]-3-(3-thiényl)propyl]amino]-*N*-méthylpropanamide ;
le 2-[[3-amino-1-(5-isothiazolyl)propyl]thio]-6-(trifluorométhyl)-3-pyridinecarbonitrile ;
le 2-[[(1*R*)-3-amino-1-(3-isoxazolyl)propyl]oxy]-6-(trifluorométhyl)-3-pyridinecarbonitrile ;
le 2-[[(1*R*)-3-amino-1-(2-thiazolyl)propyl]oxy]-6-(trifluorométhyl)-3-pyridinecarbonitrile ;
le (*R*)-2-(3-diméthylamino-1-isoxazol-5-ylpropoxy)-6-trifluorométhylnicotinonitrile ;
le 6-amino-4-[[(1*R*)-3-amino-1-(5-isoxazolyl)propyl]-thio]-3-pyridinecarbonitrile ;
la γ-[(5-chloro-2-méthoxy-4-pyridinyl)thio]-(γ⁵*R*)-5-isoxazolepropanamine,
le 4-[[(1*R*)-3-amino-1-(5-isoxazolyl)propyl]thio]-6-méthoxy-3-pyridinecarbonitrile ;
le 4-[[(1*R*)-3-[(2-hydroxyéthyl)amino]-1-(5-isoxazolyl)propyl]thio]-6-méthoxy-3-pyridinecarbonitrile ;
le 2-[[(1*R*)-3-amino-1-(3-chloro-5-isoxazolyl)propyl]-oxy]-6-(trifluorométhyl)-3-pyridinecarbonitrile ;
le 2-[[(1*R*)-3-amino-1-(3-chloro-5-isoxazolyl)propyl]-thio]-6-méthyl-3-pyridinecarbonitrile ;
le 2-[3-amino-1-(3-méthyl-4-isoxazolyl)propoxy]-6-(trifluorométhyl)-3-pyridinecarbohitrile ;
le 2-[[3-amino-1-(5-isothiazolyl)propyl]thio]-6-méthyl-3-pyridinecarbonitrile ;
le 2-[3-amino-1-(5-isothiazolyl)propoxy]-6-méthyl-3-pyridinecarbonitrile ;
le 2-[3-amino-1-(3-isothiazolyl)propoxy]-6-méthyl-3-pyridinecarbonitrile ;
le 2-[[(1*R*)-3-amino-1-(5-méthyl-3-isoxazolyl)propyl]-oxy]-6-(trifluorométhyl)-3-pyridinecarbonitrile ;
le 4-[[(1*R*)-3-amino-1-(3-isoxazolyl)propyl]oxy]-6-méthoxy-3-pyridinecarbonitrile ;
le 2-[[3-amino-1-(4-méthyl-3-isoxazolyl)propyl]thio]-6-méthyl-3-pyridinecarbonitrile ;
le 2-[3-amino-1-(2-oxazolyl)propoxy]-6-(trifluorométhyl)-3-pyridinecarbonitrile ;
le 4-[[3-amino-1-(4-chloro-5-thiazolyl)propyl]thio]-6-méthyl-3-pyridinecarbonitrile ;
le 2-[1-(4-chloro-1,3-thiazol-5-yl)-3-(méthylamino)-propoxy]-6-méthylnicotinonitrile ;
le 2-[[1-(3-fluorophényl)-3-(méthylamino)propyl]oxy]-pyridine-3-carbonitrile ;
le 6-acétyl-2-[[3-(méthylamino)-1-phénylpropyl]thio]-3-pyridinecarbonitrile ;
le 5-fluoro-6-méthyl-2-[[(1*R*)-3-(méthylamino)-1-phénylpropyl]thio]-3-pyridinecarbonitrile ;
le 6-éthyl-5-fluoro-2-[[(1*R*)-3-(méthylamino)-1-phénylpropyl]thio]-3-pyridinecarbonitrile ;
le 2-[[(1*R*)-3-[(2-hydroxyéthyl)méthylamino]-1-phénylpropyl]thio]-6-(trifluorométhyl)-3-pyridinecarbonitrile ;
le 2-[[(1*R*)-3-[(2-hydroxyéthyl)propylamino]-1-phénylpropyl]oxy]-6-(trifluorométhyl)-3-pyridinecarbonitrile ;
le 2-[[(1*R*)-3-[(3-hydroxypropyl)amino]-1-phénylpropyl]-oxy]-6-(trifluorométhyl)-3-pyridinecarbonitrile ;
le 2-[[(1*R*)-3-(méthylpropylamino)-1-phénylpropyl]oxy]-6-(trifluorométhyl)-3-pyridinecarbonitrile ;
le 2-[[(1*R*)-3-[[(2*S*)-2-hydroxypropyl]amino]-1-phénylpropyl]oxy]-6-(trifluorométhyl)-3-pyridinecarbonitrile ;
le 2-[[(1*R*)-1-phényl)-3-[(phénylméthyl)amino]propyl]-oxy]-6-(trifluorométhyl)-3-pyridinecarbonitrile ;
le 2-[[(1*R*)-3-[(1,1-diméthyléthyl)amino]-1-phénylpropyl]oxy]-6-(trifluorométhyl)-3-pyridinecarbonitrile ;
le 2-[[(1*R*)-3-[[2-(4-hydroxyphényl)éthyl]amino]-1-phénylpropyl]oxy]-6-(trifluorométhyl)-3-pyridinecarbonitrile ;
le 2-[4-amino-1-(5-isoxazolyl)butoxy]-6-(trifluorométhyl)-3-pyridinecarbonitrile ;
le 2-[[4-amino-1-(5-isoxazolyl)butyl]thio]-6-méthyl-3-pyridinecarbonitrile ;
le 4-[[4-amino-1-(5-isoxazolyl)butyl]thio]-6-méthoxy-3-pyridinecarbonitrile ;
ou un sel pharmaceutiquement acceptable, un énantiomère
ou un racémate de celui-ci.

7. Composé de formule (I) selon l'une quelconque des revendications 1 à 6, ou un sel pharmaceutiquement acceptable de celui-ci, destiné à une utilisation comme médicament.

8. Composition pharmaceutique comprenant un composé de formule (I) selon l'une quelconque des revendications 1 à 6, ou un sel pharmaceutiquement acceptable de celui-ci, en co-mélange avec un adjuvant, diluant ou véhicule pharmaceutiquement acceptable.

9. Utilisation d'un composé de formule (I) selon l'une quelconque des revendications 1 à 6, ou d'un sel pharmaceutiquement acceptable de celui-ci, dans la fabrication d'un médicament destiné au traitement ou à la prophylaxie de maladies inflammatoires.

10. Utilisation selon la revendication 9, dans laquelle la maladie est l'affection abdominale inflammatoire.

11. Utilisation selon la revendication 9, dans laquelle la maladie est la polyarthrite rhumatoïde.

12. Utilisation selon la revendication 9, dans laquelle la maladie est l'ostéoarthrite.

13. Utilisation d'un composé de formule (I) telle que définie selon l'une quelconque des revendications 1 à 6, ou d'un sel pharmaceutiquement acceptable de celui-ci, dans la fabrication d'un médicament destiné au traitement ou à la prophylaxie de la douleur.

14. Utilisation d'un composé de formule (I) telle que définie selon l'une quelconque des revendications 1 à 6, ou d'un sel pharmaceutiquement acceptable de celui-ci, en association avec un inhibiteur de COX-2, dans la fabrication d'un médicament destiné au traitement ou à la prophylaxie de maladies inflammatoires.

15. Procédé de préparation d'un composé de formule (I) telle que définie selon l'une quelconque des revendications 1 à 6, ou d'un sel pharmaceutiquement acceptable, d'un énantiomère ou d'un racémate de celui-ci, ou d'un tautomère de celui-ci, dans lequel le procédé comprend :
(a) la réaction d'un composé de formule (II) 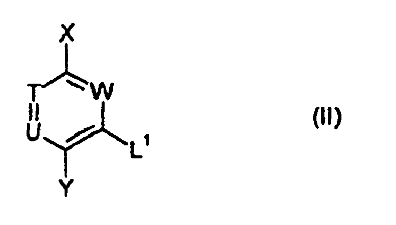 dans laquelle T, U, X, Y et W sont tels que définis selon la revendication 1 et L¹ représente un groupement partant, avec un composé de formule (III) 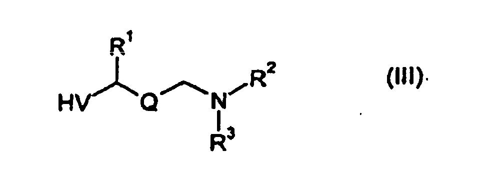 dans laquelle R¹, R², R³, Q et V sont tels que définis selon la revendication 1 ; ou
(b) la réaction d'un composé de formule (IV) 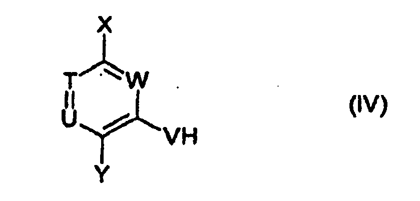 dans laquelle T, U, W, X, Y et V sont tels que définis selon la revendication 1,
avec un composé de formule (V) 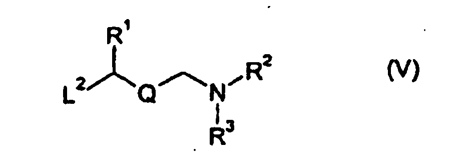 dans laquelle R¹, R², R³ et Q sont tels que définis selon la revendication 1 et L² est un groupement partant ; ou
(c) la réaction d'un composé de formule (VI) 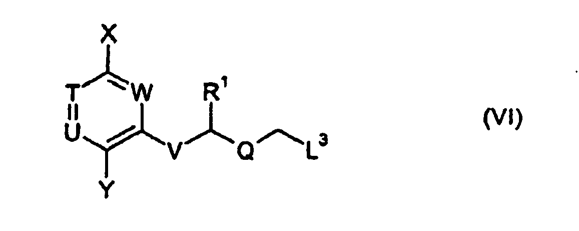 dans laquelle R¹, Q, T, U, W, X, Y et V sont tels que définis selon la revendication 1 et L³ est un groupement partant, avec un composé de formule (VII)
R²R³NH (VII)
dans laquelle R² et R³ sont tels que définis selon la revendication 1 ; ou
(d) la réduction d'un composé de formule (VIII) 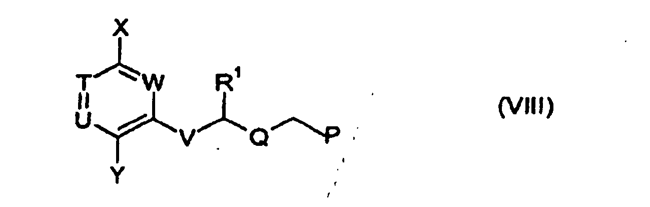 dans laquelle R¹, Q, T, U, W, X, Y et V sont tels que définis selon la revendication 1 et P représente azoture (N₃) ;
ou
(e) l'hydrolyse d'un composé de formule (VIII) 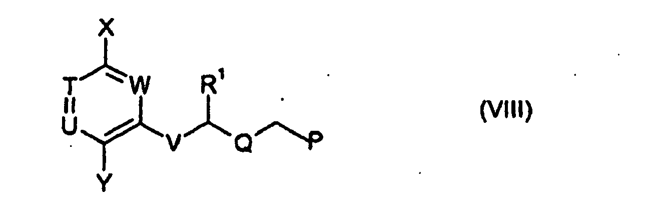 dans laquelle R¹, Q, T, U, W, X, Y et V sont tels que définis selon la revendication 1 et P représente un groupement imide ;
et si on le désire ou si nécessaire, la conversion du composé de formule (I) résultant, ou d'un autre sel de celui-ci, en un sel pharmaceutiquement acceptable de celui-ci ; ou la conversion d'un composé de formule (I) en un autre composé de formule (I) ; et si on le désire, la conversion du composé de formule (I) résultant en un isomère optique de celui-ci.
